(19) Europäisches Patentamt
European Patent Office
Office européen des brevets

(11) EP 2 612 866 A2

(12) # EUROPEAN PATENT APPLICATION
published in accordance with Art. 153(4) EPC

(43) Date of publication:
**10.07.2013 Bulletin 2013/28**

(21) Application number: **11822043.3**

(22) Date of filing: **21.07.2011**

(51) Int Cl.:
***C07K 14/32*** (2006.01) ***C12N 15/31*** (2006.01)
***C12N 15/63*** (2006.01) ***C12P 21/04*** (2006.01)

(86) International application number:
**PCT/KR2011/005380**

(87) International publication number:
**WO 2012/030068 (08.03.2012 Gazette 2012/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.09.2010 KR 20100086687**

(71) Applicant: **Biotopia Co., Ltd.**
**Chuncheon-si, Gangwon-do 200-883 (KR)**

(72) Inventors:
- **SEONG, Su-il**
  **Seoul 135-958 (KR)**
- **HWANG, Kyo Yeol**
  **Chuncheon-si**
  **Gangwon-do 200-760 (KR)**
- **LEE, Jae Yeon**
  **Suwon-si**
  **Gyeonggi-do 440-842 (KR)**
- **KANG, Kyung-don**
  **Yongin-si**
  **Gyeonggi-do 448-747 (KR)**
- **PARK, Young Shik**
  **Suwon-si**
  **Gyeonggi-do 440-737 (KR)**
- **CHO, Yong Seok**
  **Hwaseong-si**
  **Gyeonggi-do 445-737 (KR)**
- **YUCK, Won Jeong**
  **Chuncheon-si**
  **Gangwon-do 200-114 (KR)**

(74) Representative: **von Kreisler Selting Werner**
**Deichmannhaus am Dom**
**Bahnhofsvorplatz 1**
**50667 Köln (DE)**

(54) ## POLYPEPTIDE ASSOCIATED WITH THE SYNTHESIS OF 1-DEOXYNOJIRIMYCIN, AND A USE THEREFOR

(57) The present invention relates to a polypeptide associated with the biosynthesis of 1-deoxynojirimycin which is a substance that inhibits $\alpha$-glucosidase activity, to a polynucleotide coding therefor, to a vector comprising the polynucleotide, to a transformant comprising the vector, and to a method in which the transformant is used in order to produce either a polypeptide associated with the synthesis of 1-deoxynojirimycin or 1-deoxynojirimycin. The polynucleotide of the present invention can be used in the volume production of 1-deoxynojirimycin, or can be used in studies searching for novel 1-deoxynojirimycin derivatives using recombinant technology, and hence is useful in developing pathogenic virus inhibitors and hypoglycemic agents for diabetic patients.

[Fig. 11]

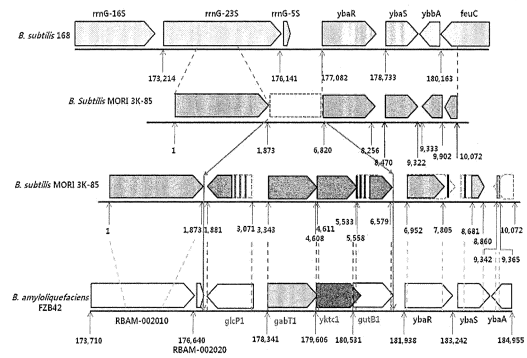


EP 2 612 866 A2

## Description

### FIELD OF THE INVENTION

The present invention relates to a 1-deoxynojirimycin synthesis-related polypeptide and the use thereof. More particularly, the present invention relates to a polypeptide involved in the biosynthesis of 1-deoxynojirimycin, which is an α-glucosidase inhibitor, a polynucleotide coding for the same, a vector carrying the polynucleotide, a transformant containing the vector, and a method for producing a 1-deoxynojirimycin synthesis-related polypeptide or 1-deoxynojirimycin using the transformant.

### BACKGROUND OF THE INVENTION

With the westernization of life environment and dietary habits and the increase of aged population, the onset of diabetes mellitus, representative of adult diseases associated with dietary life, has become increasingly prevalent in Korea. Diabetes mellitus, or simply diabetes, is a metabolic disease in which a person has high blood sugar, either because the pancreas does not produce enough insulin, or because cells in each organ respond to the insulin at very low efficiency. Diabetes without proper treatments can cause many serious complication including renal failure, cataracts, and retinopathy, thereby arousing personal and social attention.

Research and development has been conducted on hypoglycemic agents for diabetes on the basis of various mechanisms. A representative example among them is an inhibition mechanism against α-glucosidase, an enzyme catalyzing the degradation of glycogen to glucose. In the context of the development of such hypoglycemic agents, 1-deoxynojirimycin (DNJ), often isolated from mulberry leaves, is known to have potent inhibitory activity against α-glucosidase (Asano, N. et al., Carbohydr. Res. vol. 293, pp. 195-382, 1996; Asano, N. et al., Phytochemistry, vol. 53, pp. 397-382, 2000; Asano, N. et al, J. Agric. Food Chem., vol. 49, pp. 4208-4213,2001).

1-Deoxynojirimycin, a polyhydroxylated alkaloid, has a characteristic structure where the oxygen atom on the sugar moiety is substituted by a nitrogen atom. 1-Deoxynojirimycin functions to inhibit α-glucosidase and mannose dehydrogenase involved in breaking down disaccharides, such as maltose, sucrose, fructose, etc. into monosaccharise glucose, thereby suppressing blood sugar.

Many sources of 1-deoxynojirimycin are known. For example, 1-deoxynojirimycin can be obtained by separation from mulberry trees (*Morus alba* L.) or silkworms (*Bombyx mori* L.) (Asano, N. et al., J. Agric. Food Chem., vol. 49, pp. 4208-4213, 2001), extraction from plants (Evans, S. V. et al., Phytochemistry, vol. 24, pp. 1953-1955, 1985; Murao, S. and Miyata, S., Agric. Biol. Chem., vol. 44, pp. 219-221, 1980; Yamada, H., Oya, I. and Nagai, T., Shoyakugaku Zasshi, vol. 47, pp. 47-55, 1993), or production from microbes, such as Streptomyces and Bacillus (Ezure, Y. et al., Agric. Biol., Chem., vol. 49, pp. 1119-1125, 1985; Hardick, D. J. and Hutchinson, D. W., Biochemistry, vol. 49, pp. 6707-6716, 1993; Shibano, M. et al., Phytochemistry, vol. 65, pp. 2661-2665, 2004). However, extraction from plants to obtain 1-deoxynojirimycin is disadvantageous in that various components with similar structures make purification difficult, and reduce the production yield. On the other hand, the production of 1-deoxynojirimycin from microbes can be completed within a relatively short time, without destroying the environment, in contrast to the plant extraction, and guarantees a high yield. Nevertheless, neither polypeptides associated with the biosynthesis of 1-deoxynojirimycin have been known, nor genes coding for the polypeptides have been isolated thus far. For these reasons, no attempts have been made to improve the efficiency of the production from microbes or to develop new α-glucosidase, in spite of such advantages.

Many of the proteins constituting viral outer membranes are comprised of glycoproteins. In order to replicate within animal cells, viruses synthesize glycoproteins using synthesis tools of endoplasmic reticulum or golgi bodies. In animal cells, the synthesis of glycoproteins requires the addition of N-linked or O-linked sugar chains. Acting as an inhibitor against α-glucosidase I and II, and mannose dehydrogenase, which are involved in glycoprotein synthesis, 1-deoxynojirimycin blocks the synthesis of viral outer membrane proteins and allows the formation of incomplete outer membranes, thereby interfering with the production of virus particles. Accordingly, 1-deoxynojirimycin has been reported to have prophylactic effects on viral diseases (Hardick, D. J. et al., Tetrahedron, vol. 48, pp. 6285-6296, 1992), anticancer activity and preventive effects on cancer metastasis (Olden, K. et al., Pharmacol. Ther., vol. 50, pp. 285-290, 1991; Tayer, D. L. et al., AIDS, vol. 5, pp. 693-698, 1991).

As described above, 1-deoxynojirimycin is anticipated as a potential drug for treating various diseases, and therefore, there is therefore a need for developing a method for effectively producing 1-deoxynojirimycin.

The inventors of the present invention have endeavored to develop a method for producing 1-deoxynojirimycin and identified a polypeptide involved in the biosynthesis of 1-deoxynojirimycin, which can be used to produce 1-deoxynojirimycin.

## SUMMARY OF THE INVENTION

It is therefore an object of the present invention to provide a polypeptide involved in the biosynthesis of 1-deoxynojirimycin.

It is another object of the present invention to provide a polynucleotide encoding the polypeptide.

It is a further object of the present invention to provide a recombinant vector including the polynucleotide, and a transformant transformed with the vector.

It is a further object of the present invention to provide a method for producing a 1-deoxynojirimycin-related polypeptide, or 1-deoxynojirimycin by culturing the transformant.

In order to achieve the aforementioned objects, the present invention provides a polypeptide having the amino acid sequence of SEQ ID NO: 16, which includes coding regions of *gabT1, yktc1,* and *gutB1* genes; a polypeptide having the amino acid sequence of SEQ ID NO: 17, which includes coding regions of *gabT1 , yktc1, gutB1,* and *ybaR* genes; and a polypeptide having the amino acid sequence of SEQ ID NO: 18, which includes coding regions of *glcP1, gabT1, yktc1, gutB1, ybaR, ybaS,* and *ybbA* genes.

In order to achieve the aforementioned another object, the present invention provides a polynucleotide having the nucleotide sequence of SEQ ID NO: 19; a polynucleotide having the nucleotide sequence of SEQ ID NO: 20; and a polynucleotide having the nucleotide sequence of SEQ ID NO: 1, said polynucleotides encoding the polypeptides, respectively.

In order to achieve the aforementioned further object, the present invention provides a recombinant vector including one of the polynucleotide sequences; and a transformant transformed with the vector.

In order to achieve the aforementioned still another object, the present invention provides a method for producing a 1-deoxynojirimycin synthesis-related polypeptide or 1-deoxynojirimycin using the transformant.

The polynucleotide of the present invention, which encodes a polypeptide involved in the biosynthesis of 1-deoxynojirimycin, is useful for mass production of 1-deoxynojirimycin. In addition, the polynucleotide can be used in combination with a recombinant technology to search for novel 1-deoxynojirimycin derivatives which are effectively applied to the development of a hypoglycemic agent for diabetes patients, and an inhibitor against pathogenic viruses.

## BRIEF DESCRIPTION OF DRAWINGS

FIG. 1 is a photograph of an agarose gel electrophoresis where the genome extracted from *Bacillus subtilis* MORI 3K-85 of Example 1 were treated with *BamHI* and *Hin*dIII (lane 1; 1 kb DNA ladder, lane 2; genomic DNA, lane 3; *Bam*HI digest, lane 4; *Hin*dIII digest).

FIG. 2 is a schematic view showing a cleavage map of the CopyControl pCC1BAC™ vector according to the present invention.

FIG. 3 shows a TLC chromatogram analysis result of alkaloids extracted from a culture of the selected clone according to Example 1 (lane 1: reference 1-deoxynojirimycin, lane 2: C5-26, lane 3: C26-40, lane 4: C36-4, lane 5: C88-29, lane 6: C91-31).

FIG. 4A is an HPLC chromatogram analysis result of culture extracts of the selected clone C36-4 according to the present invention.

FIG. 4B is an HPLC chromatogram analysis result of 1-deoxynojirimycin used as a reference material for the compound produced in the present invention.

FIG. 5 shows LC-MS/MS profiles of 1-deoxynojirimycin used as a reference material (A), and of the culture extract of the selected clone C36-4 in the present invention (B).

FIG. 6 is a photograph of an agarose gel electrophoresis on which a plasmid prepared from the selected clone C36-4 was run (lane 1: DNA ladder; lane 2: plasmid).

FIG. 7 is a photograph of an agarose gel electrophoresis on which restriction enzyme digests of a plasmid prepared from the selected clone C36-4 according to the present invention were run (lane 1: wide-range DNA ladder, lane 2; empty vector, lane 3; C36-4-derived plasmid, undigested; lane 4: *Bam*HI digest; lane 5: *Eco*RI digest; lane 6: *Hin*dIII digest; lane 7: *Bam*HI/*Eco*RI digest; lane 8: *Bam*HI/*Hin*dIII digest; lane 9: empty vector; lane 10: 1kb DNA ladder).

FIG. 8 is a schematic view showing a restriction enzyme map of the gene insert of the selected clone C36-4 according to the present invention.

FIG. 9 is a schematic view showing the comparison of nucleotide sequences between the gene insert of the selected clone C36-4 according to the present invention and the genome of *Bacillus subtilis* 168.

FIG. 10 is a schematic view showing the comparison of nucleotide sequences between the gene insert of the selected clone C36-4 according to the present invention and the genome of *Bacillus amyloliquefaciens* FZB42.

FIG. 11 is a schematic view showing the comparison of nucleotide sequences between the gene insert of the selected clone C36-4 according to the present invention, and the genomes of *Bacillus subtilis* 168 and *Bacillus amyloliquefaciens* FZB42.

FIG. 12 shows photographs of agarose gel electrophoresis on which restriction enzyme digests of a plasmid prepared from the selected clone C36-4 according to the present invention were run (A. lane 1: 1kb DNA ladder, lane 2; *Hin*dIII digest, B. lane 1: 1kb DNA ladder, lane 2; *Hin*dIII/*Nru*I digest).

FIG. 13 is a schematic view showing the positions of *Hin*dIII restriction site on the plasmid of the selected clone C36-4, and the size of the DNA fragment after digestion of the plasmid with *Hin*dIII.

FIG. 14 is a schematic view showing the positions of *Hin*dIII and *Nru*I restriction sites on the plasmid of the selected clone C36-4, and the size of the DNA fragment after digestion of the plasmid with *Hin*dIII and *Nru*I.

## DETAILED DESCRIPTION OF THE INVENTION

Unless otherwise defined, all of technical and scientific terms used herein have the same meanings as commonly understood by one of ordinary skill in the art. Generally, the nomenclature used herein is well known in the art and commonly used.

Unless stated otherwise, the following definitions apply.

The term "nucleic acid molecule," as used herein, is intended to comprehensively comprise DNA (gDNA and cDNA) and RNA molecules. In the present invention, nucleotides which form building blocks of the nucleic acid molecules comprise sugar- or base-modified analogues as well as native nucleotides.

It should be understood by those skilled in the art from the following description of the present invention that the nucleic acid molecules of the present invention are not limited only to those encoding the specific amino acid sequences mentioned above, but are also construed as comprising those which encode either amino acid sequences with substantial identity to the specific amino acid sequences, or polypeptides having a corresponding function. In this context, the term "substantial identity" means that the amino acid sequence of the present invention and any sequence, when optimally aligned by a typical algorithm program in the art, have a sequence homology of at least 60 %, more preferably at least 80 %, and most preferably at least 90 %.

The term "polypeptide having a corresponding function" means a polypeptide having the same amino acid sequence as that of a reference polypeptide, but with a modification, such as a deletion, a substitution, an insertion, and/or an addition, on at least one amino acid residue of the amino acid sequence, while retaining the same function as that of the reference polypeptide. In the context of the 1-deoxynojirimycin synthesis-related polypeptide, the polypeptide having a corresponding function preferably has a lesser number of modifications such as a deletion, a substitution, an insertion and/or an addition. Furthermore, any polypeptide that has a sequence homology of about 60 % with the specific amino acid sequence stated above falls within the scope of the 1-deoxynojirimycin synthesis-related polypeptide according to the present invention, with higher preference for higher sequence homology.

As used herein, the term "complementary" or "complementarity" refers to the ability of polynucleotides to form base pairs with one another through hydrogen bonds between purine and pyrimidine nucleotide residues and thus to form a doublestranded nucleic acid molecule, and is also intended to partial complementarity. Base pairs relevant to the complementarity are as follows: guanine to cytosine; adenine to thymine; and adenine to uracil.

Hereinafter, the present invention will be described in detail.

In accordance with an aspect of the present invention, there is provided a polypeptide involved in the synthesis of 1-deoxynojirimycin. The polypeptide according to the present invention is derived from *Bacillus subtilis* MORI 3k-85, and encoded by a gene group responsible for the synthesis of 1-deoxynojirimycin.

In one embodiment of the present invention, there is provided a polypeptide having the amino acid sequence of SEQ ID NO: 16, which comprises the coding regions of *gabT1, yktc1,* and *gutB1* genes.

In another embodiment of the present invention, there is provided a polypeptide having the amino acid sequence of SEQ ID NO: 17, which comprises the coding regions of *gabT1, yktc1, gutB1,* and *ybaR* genes.

In a further embodiment of the present invention, there is provided a polypeptide having the amino acid sequence of SEQ ID NO: 18, which comprises the coding regions of *glcP1, gabT1 , yktc1 , gutB1, ybaR, ybaS,* and *ybbA* genes.

The polypeptide of the present invention has an amino acid sequence selected from the group consisting of: 1) the amino acid sequence of SEQ ID NO: 16; 2) the amino acid sequence of SEQ ID NO: 17, composed of the amino acid sequence of SEQ ID NO: 16 plus the coding region of *ybaR* gene; and 3) the amino acid sequence of SEQ ID NO: 18, composed of the amino acid sequence of SEQ ID NO: 17 plus the coding regions of *glcP1, ybaS,* and *ybbA* genes.

Specifically, in the polypeptides, *glcP1* codes for the amino acid sequence of SEQ ID NO: 9, *gabT1* for the amino acid sequence of SEQ ID NO: 10, *yktc1* for the amino acid sequence of SEQ ID NO: 11, *gutB1* for the amino acid sequence of SEQ ID NO: 12, *ybaR* for the amino acid sequence of SEQ ID NO: 13, *ybaS* for the amino acid sequence of SEQ ID NO: 14, and *ybbA* for the amino acid sequence of SEQ ID NO: 15.

In accordance with another aspect of the present invention, there is provided a polynucleotide having a nucleotide sequence coding for the polypeptide, or a complementary nucleotide sequence thereto.

In one embodiment of the present invention, there is provided a polynucleotide having the nucleotide sequence of SEQ ID NO: 19, which codes for a polypeptide having the amino acid sequence of SEQ ID NO: 16, or a complementary nucleotide sequence thereto.

In another embodiment of the present invention, there is provided a polynucleotide having the nucleotide sequence of SEQ ID NO: 20, which codes for a polypeptide having the amino acid sequence of SEQ ID NO: 17, or a complementary nucleotide sequence thereto.

In a further embodiment of the present invention, there is provided a polynucleotide having the nucleotide sequence of SEQ ID NO: 1, which codes for a polypeptide having the amino acid sequence of SEQ ID NO: 18, or a complementary nucleotide sequence thereto.

The polynucleotide of the present invention has a nucleotide sequence selected from the group consisting of: 1) the nucleotide sequence of SEQ ID NO: 19; 2) the nucleotide sequence of SEQ ID NO: 20, composed of the nucleotide sequence of SEQ ID NO: 19 plus the nucleotide sequence of the *ybaR* gene; and 3) the nucleotide sequence of SEQ ID NO: 1, composed of the nucleotide sequence of SEQ ID NO: 20 plus the nucleotide sequences of *glcP1, ybaS,* and *ybbA* genes.

Specifically, in the polynucleotides, *glcP1* has the nucleotide sequence of SEQ ID NO: 2; *gabT1* has the nucleotide sequence of SEQ ID NO: 3; *yktc1* has the nucleotide sequence of SEQ ID NO: 4; *gutB1* has the nucleotide sequence of SEQ ID NO: 5; *ybaR* has the nucleotide sequence of SEQ ID NO: 6; *ybaS* has the nucleotide sequence of SEQ ID NO: 7; and *ybbA* has the nucleotide sequence of SEQ ID NO: 8. More specifically, *glcP1* corresponds to nucleotides 3,071 to 1,881 of SEQ ID NO: 1, *gabT1* to nucleotides 3,343 to 4,611 of SEQ ID NO: 1, *yktc1* to nucleotides 4,608 to 5,558 of SEQ ID NO: 1, *gutB1* to nucleotides 5,533 to 6,579 of SEQ ID NO: 1, *ybaR* to nucleotides 6,820 to 8,256 of SEQ ID NO: 1, *ybaS* to nucleotides 8,470 to 9,322 of SEQ ID NO: 1, and *ybbA* to nucleotides 9,902 to 9,333 of SEQ ID NO: 1.

The functions of the polypeptides encoded by the above seven genes are inferred as follows. *glcP1* functions as a transmembrane transporter. *gabT1* has both 4-aminobutyrate transaminase activity and pyridoxal phosphate binding activity. *yktc1* has inositol or phosphatidylinositol phosphatase activity and *gutB1* has both oxidoreductase activity and zinc ion binding activity. *ybaR* has secondary active sulfate transmembrane transporter activity and *ybaS* has bile acid: sodium symporter activity. *ybbA* has not yet been known for its function (Tables 3 and 7).

According to one embodiment of the present invention, the polynucleotides are preferably isolated from strains, more preferably, *Bacillus subtilis* MORI 3K-85.

In accordance with a further aspect of the present invention, there are provided a recombinant vector comprising the polynucleotide, and a host cell transformed with the recombinant vector. The group of genes encoding the polypeptides involved in the biosynthesis of 1-deoxynojirimycin can be cloned into proper vectors such as BAC, plasmids, fosmids, cosmids, etc., and then the vectors may be introduced into suitable host cells. Preferable examples of the host cells are yeasts, *Bacillus sp.,* and *E. coli.* The introduction of the recombinant vector into host cells may be carried out using various typical techniques including heat shock, electroporation, etc. It is obvious to those skilled in the art that various host cells other than the aforementioned host cells can be employed depending on the purpose of expression.

In accordance with still another aspect of the present invention, there is provided a method for producing a 1-deoxynojirimycin synthesis-related polypeptide, comprising (1) culturing a transformant transformed with a recombinant vector comprising the polynucleotide of the present invention; and (2) isolating the 1-deoxynojirimycin synthesis-related polypeptide from the culture.

Also, the present invention provides a method for producing 1-deoxynojirimycin, comprising (1) culturing a transformant transformed with a recombinant vector comprising the polynucleotide of the present invention; and (2) isolating 1-deoxynojirimycin from the culture.

## EXAMPLES

Hereinafter, the present invention is described in more detail. The following Examples are given for the purpose of illustration only, and are not intended to limit the scope of the invention.

The following Examples are provided to illustrate preferred embodiments of the present invention, and are not intended to limit the scope of the present invention.

### EXAMPLE 1: Library Construction Using Genome of *Bacillus subtilis* MORI 3K-85 producing 1-Deoxynojirimycin

<1-1> Strain Culturing

*Bacillus subtilis* MORI (Accession No. KCCM-10450), isolated and identified from Korean traditional foods, was irradiated by gamma rays to obtain a modified *Bacillus subtilis* MORI 3K-85 strain with improved inhibitory activity against $\alpha$-glucosidase. Specifically, the MORI strain was cultured in Difco™ YM [Becton Dickinson and Company (BD), USA] broth at 37°C for one day with shaking at 180 rpm, and irradiated with gamma rays at a dose of 3 kGy or 5 kGy (Soya GreenTec, Korea). The cultures irradiated with gamma rays were spread on YM agar plates and incubated at 37°C for 18 hrs. Subsequently, the colonies thus formed were transferred onto fresh YM agar plates and incubated. Again, the colonies grown on the plates were inoculated into YM broths and cultured at 37°C for 5 days with shaking at 180 rpm. The cells were analyzed for inhibitory activity against α-glucosidase to obtain a modified sub-strain, *Bacillus subtilis* MORI 3K-85.

After incubation for one day, *Bacillus subtilis* MORI 3K-85 was inoculated in an amount of 1 % by volume into 500 mL of each of four YM broths, and cultured at 37°C for one day with shaking at 180 rpm. The culture was centrifuged at 8,000 rpm for 10 min, and supernatant was removed. The cell pellet thus formed was resuspended in 20 mL of 1X phosphate buffered saline (PBS) and stored at -20°C until use for gene extraction.

<1-2> Genome Extraction

Genomic DNA was extracted from *Bacillus subtilis* MORI 3K-85 using a genome extraction kit (Promega USA). 1 mL of the *Bacillus subtilis* MORI 3K-85 strain suspension stored at -20°C was thawed at 4°C, and centrifuged at 16,000 × g for 2 min. After removal of the supernatant, the cell pellet was resuspended in 480 μL of 50 mM EDTA (ethylene diamine tetra-acetic acid), and incubated with 120 μL of a cell lysis buffer at 37°C for 30 min. Centrifugation was performed in the same manner (16,000× g for 2 min), and the supernatant was transferred into a new 1.5 mL Eppendorf tube. To this supernatant was added 600 μL of a nucleus lysis buffer (included within the genome extraction kit, Promega, USA), followed by incubation at 80°C for 5 min and cooling the reaction solution to room temperature. Then, the solution was gently mixed five times with 3 μL of an RNase solution, incubated at 37°C for 30 min, and cooled to room temperature.

Thereafter, this solution was mixed and reacted for 5 min on ice with 200 μL of a protein precipitation solution to remove proteins therefrom, followed by centrifugation at 16,000× g for 2 min to obtain the supernatant. To 600 μL of the supernatant was added 600 μL of isopropanol, and the mixture was centrifuged at 16,000× g for 2 min. After removal of the supernatant, the pellet was washed three times with 600 μL of 70 % ethyl alcohol, followed by centrifugation at 16,000× g for 2 min. The pellet was dried by evaporating residual ethyl alcohol, and dissolved in 30 μL of the DNA rehydration solution (included within the genome extraction kit, Promega, USA) in a water bath at 65°C for 60 min.

<1-3> Digestion with Restriction Enzymes

DNA fragments were prepared by digestion with restriction enzymes *Bam*HI, *Hin*dIII, and *Eco*RI (Promega, USA). A mixture of 1 μL of the genomic DNA (1 μg/μL) obtained above, 16.3 μL of sterile water, 2 μL of a 10x buffer, and 0.2 μL of bovine serum albumin (10 μg/μL) was incubated with 0.5 μL of the restriction enzymes for 1 hr in a water bath at 37°C, and then incubated for 15 min in a water bath at 65°C to terminate the restriction digestion.

Subsequently, 120 mL of 0.5 % agarose gel containing 200 μL of ethidium bromide (2 μg/mL) was prepared and molded. Of a mixture of 5 μL of the restriction enzyme digest and 1 μL of a dye buffer, 5 μL was loaded to the 0.5% agarose gel. And then, 5 μL taken from a mixture of 5 μL of a 1 kb DNA marker (Promega, USA) and 1 μL of a dye buffer was loaded to the 0.5 % agarose gel. After the loads were run at 50 V for 30 min on the gel, they were irradiated with UV from a UV transilluminator (UVP, USA) to visualize the bands.

Upon *Bam*HI digestion, the DNA fragments were, for a large part, as large as or larger than 6 kb in size. On the other hand, *Hin*dIII digests predominantly have a size ranging from 2 to 7 kb (FIG. 1). *BamHI* digests of the DNA fragments were used to construct transformants later.

<1-4> Ligation of DNA fragments to pCC1BAC Vector

To clone the DNA fragments, 100 ng of the DNA fragment resulting from digestion with *Bam*HI in Example <1-3> was mixed with 1 μL of a CopyControl pCC1BAC Cloning-Ready *Bam*HI vector (Epicentre, USA; FIG. 2), and sterile water was added to the DNA mixture to form a total volume of 87 μL. The resulting DNA solution was incubated at 55°C for 10 min, cooled at room temperature for 15 min, and mixed with 10 μL of a 10x Fast-Link ligation buffer (included within CopyControl pCC1BAC Cloning-Ready *Bam*HI kit, Epicentre, USA), 1 μL of 100 mM ATP, and 2 μL of Fast-Link DNA ligase (CopyControl pCC1BAC Cloning-Ready *Bam*HI kit, Epicentre, USA) to form a total volume of 100 μL. The resulting mixture was incubated at 16°C for 4 hrs, followed by heating at 65°C for 15 min to inactivate the ligase. For use in desalting the resulting ligate solution, 0.9 g of glucose and 0.5 g of agarose were completely dissolved in 50 mL of primary distilled water by boiling, cooled to 50°C, and aliquoted in an amount of 800 μL in 1.5 mL tubes into which 0.5 mL tubes were then inserted before solidification to form a conical shape. The ligate solution was added thereto and incubated for 1 hr on ice. The recombinant vectors thus obtained were used to transform E. coli.

<1-5> Construction of Transformant

For use in the transformation of TransforMax™ EPI300™ electrocompetent *E. coli* (Epicentre, USA), an electroporation cuvette and a 1.5 mL tube were placed on ice. In the tube, 50 μL of the *E. coli* solution thawed slowly on ice, and 2 μL of the desalted ligate solution were mixed. The mixture was transferred into the cold electroporation cuvette, and electroporation was carried out at an electric field of 12 kV/cm using a Gene Pulser (Bio-Rad, USA). The transformed cells in the cuvette were mixed with 950 μL of an SOC medium (2 % bacto-trytone, 0.5 % bacto-yeast extract, 10 mM NaCl, 2.5 mM KCl, 20 mM $Mg_2^+$ buffer ($MgCl_26H_2O$, $MgSO_47H_2O$), 20 mM glucose), transferred into a 50 mL tube, and incubated at 37°C for 1 hr with shaking at 220~230 rpm. This cell solution was spread in an amount of 100 μL over a Luria-Bertani (LB) plate containing 12.5 μg/mL of chloramphenicol, 40 μg/mL of 5-bromo-4-chloro-3-indolyl-beta-D-galactopyranoside (X-Gal), and 50 μg/mL of 40 mM isopropyl beta-D-1-thiogalactopyranoside (IPTG), and cultured at 37°C for 12 hrs in an incubator. White colonies were selected from the plate, and cultured in a LB broth. The resulting cultures were used to select clones productive of 1-deoxynojirimycin.

**EXAMPLE 2: Screening by Measuring Inhibitor Activity against α-Glucosidase**

In order to select colonies capable of producing 1-deoxynojirimycin among the white colonies, a method of examining inhibitory activity against α-glucosidase was used. The cultures prepared in Example <1-5> were thermally treated at 100°C for 10 min, and centrifuged at 8,000 rpm for 10 min. To 5 μL of the supernatant, 75 μL of 0.1 M potassium phosphate buffer (pH 6.8), 50 μL of 12 mM *p*-nitrophenyl-α-glucopyranoside as a substrate, and 20 μL of a crude enzyme solution were added. In order to prepare the crude enzyme solution, 0.8 g of rat intestinal acetone powder (Sigma, USA) was weighed and subjected to extraction in 100 mL of 0.1 M potassium phosphate buffer for 1 hr. The resulting solution was centrifuged at 12,000 rpm for 10 min to obtain a supernatant used as the crude enzyme solution.

The mixture was incubated at 37°C for 35 min, and the enzymatic reaction was terminated with 50 μL of 200 mM sodium carbonate. Absorbance was read at 405 nm on a microplate reader (Molecular device, USA). Selection was made of the clones which were found to have 20 % or higher inhibitory activity against α-glucosidase as calculated by the following Formula 1 (Scofiled, A. M. et al., Life Sci., vol. 39, pp. 29-32, 1986).

<Formula 1>

$$\text{Inhibitory Activity (\%)} = \left\{1 - \frac{A_{405}\text{(Test)} - A_{405}\text{(Control)}}{A_{405}\text{(Enzyme Test)} - A_{405}\text{(Blank Test)}}\right\} x\ 100$$

For the selection of desired clones, a total of 40,000 clones were cultured. Among them, five were found to have inhibitory activity against α-glucosidase. The five clones are given in Table 1, below. Clone C5-26 was measured to inhibit α-glucosidase by 20 %; clones C26-40, C88-29, and C91-31 by 32 % ~ 38 %; and clone C36-4 by 48 % (Table 1). In Table 1, the colony numbers were assigned to each of total 40,000 colonies. For example, C5-26 represents a colony taken $26^{th}$ from the plate No. 5.

TABLE 1

| Inhibitory Activity of Selected Clones against α-Glucosidase | |
|---|---|
| Colony | No. Inhibitory Activity against α-Glucosidase (%) |
| Control | 0.0 |
| C5-26 | 20.0 |
| C26-40 | 32.0 |
| C36-4 | 48.0 |
| C88-29 | 36.0 |
| C91-31 | 38.0 |

**EXAMPLE 3: Detection of 1-Deoxynojirimycin**

The selected clones were examined whether they produce 1-deoxynojirimycin through TLC, HPLC, and LC-MS/MS analysis.

<3-1> TLC Analysis for Color Development of Alkaloids

Each of the cultures and the following samples for the fractions of ion exchange resin were dropwise loaded in an amount of 10 μL onto a TLC plate (Merck, silica gel 60 $F_{254}$), and run with a developing solvent of propanol : acetic acid : primary distilled water (4 : 1 : 1). Color development was carried out with a chlorine-O-tolidine reagent to detect alkaloids.

The Rf value of 1-deoxynojirimycin run on control lane 1 was 0.375. Around 0.375, a spot was also developed on lane 4 for clone C36-4. Therefore, C36-4 was selected as a clone that is likely to produce 1-deoxynojirimycin because it was found to have a 1-deoxynojirimycin spot on TLC while showing as high as 48 % α-glucosidase inhibition (FIG. 3).

<3-2> HPLC Analysis for 1-Deoxynojirimycin Detection

The presence of 1-deoxynojirimycin in a culture of clone C36-4 was examined. Clone C36-4 was cultured at 37°C for one day in 2 L of LB broth with shaking at 230 rpm, and the culture was heated at 100°C for 10 min and centrifuged at 10,000 rpm. The supernatant thus obtained was concentrated to 50 mL using a reduced pressure concentrator (Buchi, Swiss). The concentrate was absorbed onto a column (100×10 mm, $H^+$ type) filled with Amberlyst 15 (Sigma-5647, USA), washed with 10 mL of secondary distilled water, and eluted with 80 mL of 0.5 N ammonia water. The eluate was concentrated to 4 mL, loaded to a column (100×10 mm, $OH^-$ type) filled with Dowex 1×2-100 (Sigma-Aldrich 21,739-7, USA), and eluted with 40 mL of secondary distilled water to obtain a fraction.

To examine whether the obtained fraction contains 1-deoxynojirimycin, a method disclosed in an article of Kim *et al.* method (Kim, J. W. et al., Chromatography, vol. 1002, pp. 93-99, 2003) was used. In an Eppendorf tube, 10 μL of the sample was incubated at 20°C for 20 min with 10 μL of 0.4 M potassium borate buffer (pH 8.5) and 20 μL of 10 mM 9-fluorenylmethyl chloroformate (FMOC-Cl), and the reaction was stopped with the addition of 10 μL of 0.1 M glycine. The volume of the reaction mixture was adjusted to 1 mL by adding 950 μL of 0.1 % acetic acid thereto, followed by filtration through a 0.2 μm syringe filter (Nalgene, USA). 10 μL of the filtrate was subjected to HPLC analysis (Shiseido, Japan) (column: Capcell Pak $C_{18}$ MG, φ : 4.60×250 mm mobile phase: acetonitrile-0.1 % acetic acid (1:1, v/v); flow rate: 1 $min^{-1}$ FL3000 fluorescence detector: excitation 254 nm, extinction 322 nm).

On the HPLC chromatograms, the RT of the standard material 1-deoxynojirimycin was detected at 3.8 min. For the purified sample fraction obtained from a culture of clone C36-4 through the two ion exchange chromatography steps, a single peak was detected at an RT of 3.8 min, which was the same as in 1-deoxynojirimycin (FIGS. 4a and 4b). Accordingly, clone C36-4 was identified to produce 1-deoxynojirimycin, and inferred to have a group of genes responsible for the production of 1-deoxynojirimycin.

<3-2> LC-MS/MS Analysis for 1-Deoxynojirimycin Detection

The production of 1-deoxynojirimycin was confirmed by LC-MS/MS (liquid chromatography-tandem mass spectrometry). For LC-MS/MS, Agilent 1200 series HPLC and Agilent 6410B (Agilent Technologies, Santa Clara CA, USA) were employed. Chromatographic separation was performed at 40°C in an Agilent Epic HILIC-HC column (4.6 × 150 mm, 3 μm) loaded with 2 μL of the sample. A mobile phase of solution A (0.1 % formic acid in water) and solution B (0.1 % formic acid in acetonitrile) was applied at a flow rate of 0.8 mL/min in a gradient elution manner (0-0.1 min, 5 % A and 95 % B; 0.1-15 min, 80 % A and 20 % B; 15-20 min, 20 % A and 80 % B). Mass spectra were obtained in the positive mode and the scan mode for selected ion monitoring (SIM). Conditions for mass spectrometry were set forth as follows: Gas temperature: 320°C, Flow rate: 35 L/min (nitrogen), Capillary voltage: 4 kV, Fragmentor voltage: 150 V.

On the LC-MS/MS spectra, a peak for a culture of the transformant having a HN BAC gene was detected at an RT of 6.16 min, which was the same as in the standard material 200 mg/L 1-deoxynojirimycin, and the culture was analyzed to contain 1-deoxynojirimycin at a concentration of 5.99 mg/L (FIG. 5).

**EXAMPLE 4: Base Sequencing and Homology Analysis of Clone C36-4 Gene Inserted into Plasmid**

<4-1> Plasmid Extraction from Selected Clone

A plasmid was extracted in a large amount from the selected clone. First, the plasmid was amplified within the selected clone using a CopyControl BAC cloning kit (Epicentre, USA). One colony picked out from the selected clone C36-4 was inoculated into 5 mL of an LB broth containing 12.5 μg/mL of chloramphenicol in a 50 mL conical tube, and cultured at 37°C for 12 hrs in an incubator with shaking at 220 rpm. Together with 800 μL of an LB broth containing chloramphenical (12.5 μg/mL), 200 μL of an inoculate and 1 μL of a CopyControl induction solution (included within the CopyControl pCC1BAC Cloning-Ready *Bam*HI kit, Epicentre, USA) were placed in a 1.5 mL tube, and incubated at 37°C for 5 hrs with shaking. The culture was used for plasmid extraction.

Next, a plasmid was extracted from the clone using a GeneAll XPREP™ plasmid SV mini kit (GeneAll, Korea), which takes advantage of alkaline lysis. The extracted plasmid was run on a 1 % agarose gel and identified to have a size of 10 kb or greater including a vector (FIG. 6).

<4-2> Measurement of Size of Inserted Gene

To measure the size of the inserted gene present in the selected clone, the plasmid was digested with *Bam*HI, *Hin*dIII, *Eco*RI, a combination of *Bam*HI and *Eco*RI, or a combination of *BamHI* and *Hin*dIII according to the protocol of Promega. Subsequently, 5 μL of each of the restriction enzyme digests was mixed with 1 μL of a dye buffer, and 5 μL of this mixture was loaded to a 0.5 % agarose gel containing 200 μL of 0.5 M EDTA. On the other hand, 5 μL of a wide-range DNA marker and 5 μL of a 1 kb DNA marker (Promega USA) were separately mixed with 1 μL of a dye buffer, and 5 μL was taken from each of the resulting marker mixtures and loaded to the 0.5 % agarose gel. An electric field of 50 V was applied for 1.5 hrs to the gel to run the DNAs, followed by visualizing DNA bands under a UV transilluminator (USA).

The results are shown in FIG. 7. An empty vector with a size of 8.128 kb was run on lanes 2 and 9 while a circular plasmid extracted from the clone C36-4, not treated with any restriction enzyme, was run on lane 3, the *Bam*HI digest on lane 4 to separate into two DNA fragments (10.1kb, 8.1kb), the *Eco*RI digest on lane 5 to separate into three DNA fragments (13.8kb, 3.8kb, 0.56kb), the *Hin*dIII digest on lane 6 to separate into four DNA fragments (11.2kb, 5.6kb, 1.1kb, 0.26kb), the double *Bam*HI/*Eco*RI digest on lane 7 to separate into five DNA fragments (8.1kb, 5.7kb, 3.8kb, 0.54kb, 0.021kb), and the double *Bam*HI/*Hin*dIII digest on lane 8 to separate into six DNA fragments (8.1kb, 5.6kb, 3.2kb, 1.1kb, 0.26kb, 0.03kb) (FIG. 7, Table 2).

TABLE 2

| Sizes of Restriction Enzyme Digests of the Gene Inserted into Clone C36-4 | | |
|---|---|---|
| Enzyme | No. of Fragment | Size of Fragment (bp) |
| *Bam*HI | 2 | 10080,8128 |
| *Eco*RI | 3 | 13824, 3827, 557 |
| *Hin*dIII | 4 | 11243, 5562, 1141, 262 |
| *Bam*HI + *Eco*RI | 5 | 8107, 5717, 3827, 536, 21 |
| *BamHI* + *Eco*RI | 6 | 8098, 5562, 3145, 1111, 262, 30 |

To put the results of restriction enzyme digestion together, the plasmid present in clone C36-4 was inferred to have a total size of 18.208 kb, and the inserted gene was calculated to be 10.080 kb in length by subtracting the vector size of 8.128 kb. Restriction enzyme mapping indicated that the inserted gene has two *Eco*RI sites and three *Hin*dIII sites (FIG. 8).

<4-3> Base Sequencing of Plasmid

The nucleotide sequence of the cloned gene was analyzed by SolGent Co. Ltd. using an automatic base sequencer. For homology analysis, the cloned gene was compared with known genes using the database of the National Center of Biotechnology Information (NCBI) (http://www.ncbi.nlm.nih.bov/BLAST/) and http://www.uniprot.org, and http://gib.genes.nig.ac.jp/, thereby searching for genes associated with the biosynthesis of 1-deoxynojirimycin.

The nucleotide sequence of the gene insert in the clone C36-4 was identified (SEQ ID NO: 1), and compared in the database of the GenBank with the genome information of the standard strain *B. subtilis* 168 (Barbe, V et al., Microbiology, vol. 155, pp. 1758-1775, 2009; Kunst, F. et al., Nature, vol. 390, pp. 249-256, 1997; Liu, H. et al., Microbiology, vol. 143, pp. 2763-2767, 1997), and further with the genome information of *B. amyloliquefaciens* FZB42, known to produce 1-deoxynojirimycin (Chen, X. H. et al., Nat. Biotechnol., vol. 25, pp. 1007-1013, 2007), so as to infer the functions of the genes inserted into C36-4.

Comparison analysis between the nucleotide sequence of the gene inserted into clone C36-4 and the genome information of B. *subtilis* 168 showed that clone C36-4 has nucleotide sequences homologous to three open reading frames (ORF) of *ybaR, ybaS,* and *ybbA* on the genome of B. *subtilis* 168. Inference of the biological processes of the proteins encoded by the three open reading frames was made that *ybaR* might have sulfate transmembrane transport activity and *ybaS* have sodium ion transport activity, but no functions were found for *ybbA.*

Considering the molecular functions of the three open reading frames, *ybaR* and *ybaS* can have secondary active sulfate transmembrane transporter activity and bile acid:sodium symporter activity, respectively. However, the function of *ybbA* still remained undeducible (FIG. 9, Table 3).

Also, a nucleotide sequence present at the terminus of the inserted gene was found to have sequence homology with a part of the open reading frame *feuC.* The biological process in which the protein encoded by *feuC* is involved was inferred to have iron ion transport activity, with the deduction of transporter activity for its molecular function.

TABLE 3

| Results of Analysis of Genes Inserted into Clone C36-4 | | | | | | |
|---|---|---|---|---|---|---|
| **ORF** | **Size (aa)** | **Initiation** | **Termination** | **Protein name** | **Molecular function** | **Biological process** |
| *ybaR* | 478 | 6,820 | 8,256 | Putative sulfate transporter *ybaR* | Secondary active sulfate transmembrane transporter activity | Sulfate transport and transmembrane transport |
| *ybaS* | 283 | 8,470 | 9,322 | Uncharacterized protein *ybaS* | Bile acid:sodium symporter activity | Sodium ion transport |
| *ybbA* | 189 | 9,902 | 9,333 | Uncharacterized protein *ybbA* | Unknown | Unknown |

In addition, a characteristic 4.948 kb-long nucleotide sequence, not found in the gene of *Bacillus subtilis* 168, was found to exist between *rrnG-23S* and *ybaR* on the gene insert of the transformant capable of producing 1-deoxynojirimycin (*see* FIGS 9 and 11). A search of the NCBI database revealed that the characteristic nucleotide sequence (4.948 kb) has a sequence homology of 86 %, 78 %, and 75 % with the nucleotide sequences of *gabT1, yktc1,* and *gutB1,* respectively, existing on the genome of *B. amyloliquefaciens* FZB42, known to produce 1-deoxynojirimycin (Tables 4 to 6)

TABLE 4

| Comparison of Nucleotide Sequence between *gabT1* of C36-4 and *gabT1* of *B. amyloliquefaciens* FZB42 | | | | | | |
|---|---|---|---|---|---|---|
| Accession | Description | Max score | Total score | Query coverage | E value | Max identity |
| CP000560.1 | *Bacillus amyloliquefaciens* FZB42, complement genome | 1180 | 1266 | 100% | 0.0 | 86 % |
| FP929038.1 | *Coprococcus catus GD/7* draft genome | 105 | 105 | 10% | 5e-19 | 76% |
| FP929050.1 | *Roseburia intestinalis XB6B4* draft genome | 78.8 | 78.8 | 10% | 7e-11 | 74 % |
| FP929049.1 | *Roseburia intestinalis M50/1* draft genome | 78.8 | 78.8 | 10% | 7e-11 | 74 % |
| CP001348.1 | *Clostridium cellulolyticum H10,* complement genome | 78.8 | 78.8 | 11 % | 7e-11 | 71 % |

TABLE 5

| Comparison of Nucleotide Sequence *between yktc1* of C36-4 *and yktc1* of *B. amyloliquefaciens* FZB42 | | | | | | |
|---|---|---|---|---|---|---|
| Accession | Description | Max score | Total score | Query coverage | E value | Max identity |
| CP000560.1 | *Bacillus amyloliquefaciens* FZB42, complement genome | 809 | 809 | 100 % | 0.0 | 78 % |
| CP001510.1 | *Methylobacterium extorquens* AM I, complete genome | 44.6 | 44.6 | 3 % | 1.1 | 90 % |
| CP000908.1 | *Methylobacterium extorquens* PA1, complete genome | 44.6 | 44.6 | 3 % | 1.1 | 90 % |
| AB048345.1 | *Equus caballus* DNA chromosome Xp21, microsatellite TKY39 | 44.6 | 44.6 | 2 % | 1.1 | 96 % |

TABLE 6

| Comparison of Nucleotide Sequence between *gutB1* of C36-4 and *gutB1* of *B. amyloliquefaciens* FZB42 | | | | | | |
|---|---|---|---|---|---|---|
| Accession | Description | Max score | Total score | Query coverage | E value | Max identity |
| CP000560.1 | *Bacillus amyloliquefaciens* FZB42, complement genome | 720 | 720 | 98 % | 0.0 | 75 % |
| AL009126.3 | *Bacillus subtilis* subsp. *subtilis* str. 168 complement genome | 78.8 | 78.8 | 6 % | 6e-11 | 85 % |
| D84213.1 | *Bacillus subtilis* genome, trn1-feuABC region | 73.4 | 73.4 | 6 % | 3e-09 | 84 % |

Based on the results of the comparison between the nucleotide sequence of the gene inserted into clone C36-4 and the genome of *B. amyloliquefaciens* FZB42, the clone C36-4 was analyzed to have nucleotide sequences having sequence homologies with the four ORFs *glcP1, gabT1, yktc1,* and *gutB1* on the genome of *B. amyloliquefaciens* FZB42. With regard to the biological process in which the protein encoded by the four ORFs is involved, the *gutB1* was inferred to have oxidation reduction activity, but the biological processes of *glcP1, gabT1,* and *yktc1* could not be inferred.

Inference of the molecular functions of the four open reading frames was made that: *glcP1* might have transmembrane transporter activity; *gabT1* might have 4-aminobutyrate transaminase activity and pyridoxal phosphate binding activity; *yktc1* might have inositol or phosphatetidylinositol phosphatase activity; and *gutB1* might have oxidoreductase activity and zinc ion binding activity (FIG. 10, Table 7).

TABLE 7

| Analysis Results of Genes Inserted into Clone 36-4 | | | | | | |
|---|---|---|---|---|---|---|
| **ORF** | **Size (aa)** | **Initiation** | **Termination** | **Protein name** | **Molecular function** | **Biological process** |
| *glcP1* | 396 | 3,071 | 1,881 | *glcP1* | Transmembrane transport | Unknown |

(continued)

| Analysis Results of Genes Inserted into Clone 36-4 | | | | | | |
|---|---|---|---|---|---|---|
| **ORF** | **Size (aa)** | **Initiation** | **Termination** | **Protein name** | **Molecular function** | **Biological process** |
| *gabT1* | 422 | 3,343 | 4,611 | *gabT1* | 4-aminobutyrate transaminase activity and pyridoxal phosphate binding | Unknown |
| *yktc1* | 316 | 4,608 | 5,558 | *yktc1* | Inositol or phosphatidylinositol phosphatase activity | Unknown |
| *gutB1* | 348 | 5,533 | 6,579 | *gutB1* | Oxidoreductase activity and zinc ion binding | Oxidation reduction |

To sum up, the gene insert of the clone C36-4 were found to have a total of seven open reading frames containing these four open reading frames and the previously described three open reading frames which have sequence homology with genes of *B. amyloliquefaciens* FZB42.

For convenience, the seven genes (*glcP1, gabT1 , yktc1, gutB1, ybaR, ybaS,* and *ybbA*) are collectively named B BAC (Table 3 and FIG. 7).

**EXAMPLE 5: Assay for Measuring Inhibitory Activity of Gene Fragments against α-Glucosidase**

<5-1> Plasmid Extraction from Selected Clone

The plasmid was prepared on a large scale from clone C36-4 in the same manner as in Example <4-1> and used for digestion with restriction enzymes.

<5-2> Restriction Enzyme Digestion

The plasmid was digested with *Hin*dIII alone or a combination of *Hin*dIII and *Nru*I (Promega, USA) in the same manner as in Example <1-3> to prepare two groups of DNA fragments. Their sizes were determined by separation on a 0.5 % agarose gel containing 200 μL of 0.5 M EDTA in the same manner as in Example <1-3>.

As a result, the digestion of the plasmid prepared from clone C36-4 with *Hin*dIII produced a 5,562 bp-long DNA fragment containing the four genes *gabT1, yktcl, gutB1,* and *ybaR,* as shown in FIGS. 12A and 13, and the DNA fragment was named H BAC for convenience (FIGS. 12 and 13).

In addition, the *Hin*dIII/*Nru*I digest from the plasmid of C36-4 was, as shown in FIGS. 12B and 14, a 4,203 bp-long DNA fragment containing the three genes *gabT1, yktc1,* and *gutB1,* and this DNA fragment was named HN BAC for convenience (FIGS. 12 and 14). These two DNA fragments were ligated to pCC1BAC.

<5-3> Ligation of DNA Fragment to pCC1BAC and Construction of Transformant

The two DNA fragments H BAC and HN BAC obtained in Example <5-2>, were ligated into a pCC1BAC Cloning-Ready *Hin*dIII vector in the same manner as in Example 1-4 to construct recombinant plasmids. They were transformed into E. *coli* in the same manner as in Example <1-5> to prepare transformants.

<5-4> Culturing of Transformants and Assay for Measuring 1-Deoxynojirimycin Synthesis

The transformants prepared in Example <5-3> were cultured in the same manner as in Example <1-5>, and examined whether 1-deoxynojirimycin was produced in the culture by assaying α-glucosidase inhibitory activity.

The activity of α-glucosidase was inhibited by 51.0 % in the culture of the transformant containing the DNA fragment B BAC, by 71.7 % in the culture of the transformant containing the DNA fragment H BAC, and by 74.5 % in the culture of the transformant containing the DNA fragment HN BAC. Thus, the transformant containing the HN BAC gene group comprising *gabT1, yktc1,* and *gutB1* exhibited the highest inhibitory activity against α-glucosidase (Table 8).

TABLE 8

| Inhibitory Activity of B BAC, H BAC, and HN BAC Fragments against α-Glucosidase | |
|---|---|
| **Type of DNA fragments** | **α-Glucosidase inhibitory activity (%)** |
| B BAC | 51.0 |
| H BAC | 71.7 |
| HN BAC | 74.5 |

In conclusion, the clone C36-4 with excellent inhibitory activity against α-glucosidase contained a gene insert as long as about 10 kb (FIG. 7), and was found to produce 1-deoxynojirimycin as measured by HPLC (FIGS. 4a and 4b). A search of the GenBank database revealed that the gene insert contained a group of seven genes (*glcP1, gabT1, yktc1, gutB1, ybaR, ybaS,* and *ybbA*) relevant to the synthesis of 1-deoxynojirimycin.

A restriction enzyme assay showed that a group of three genes (*gabT1, yktc1, gutB1*) or four genes (*gabT1, yktc1, gutB1,* and *ybaR*) are particularly associated with the synthesis of 1-deoxynojirimycin, and that the three genes *gabT1, yktc1,* and *gutB1* in the HN BAC DNA fragment are more highly apt to be involved in the synthesis of 1-deoxynojirimycin.

## Claims

1. A polypeptide having the amino acid sequence of SEQ ID NO: 16, which includes coding regions of *gabT1, yktc1,* and *gutB1* genes.

2. A polypeptide having the amino acid sequence of SEQ ID NO: 17, which includes coding regions of *gabT1, yktc1, gutB1,* and *ybaR.*

3. A polypeptide having the amino acid sequence of SEQ ID NO: 18, which includes coding regions of *glcP1, gabT1, yktc1, gutB1, ybaR, ybaS,* and *ybbA.*

4. A polynucleotide having the nucleotide sequence of SEQ ID NO: 19, which codes for the polypeptide of claim 1, or a complementary nucleotide sequence thereto.

5. A polynucleotide having the nucleotide sequence of SEQ ID NO: 20, which codes for the polypeptide of claim 2, or a complementary nucleotide sequence thereto.

6. A polynucleotide having the nucleotide sequence of SEQ ID NO: 1, which codes for the polypeptide of claim 3, or a complementary nucleotide sequence thereto.

7. The polypeptide of any one of claims 1 to 3, wherein the polypeptide is involved in biosynthesis of 1-deoxynojirimycin.

**8.** The polypeptide of any one of claim 1 to 3, wherein the polypeptide has inhibitory activity against α-glucosidase.

**9.** A recombinant vector comprising the polynucleotide of any one of claims 4 to 6.

**10.** A transformant transformed with the recombinant vector of claim 9.

**11.** The transformant of claim 10, wherein the tranformant is selected from the group consisting of a yeast, *Bacillus sp.,* and *E. coli.*

**12.** A method for producing a 1-deoxynojirimycin synthesis-related polypeptide, comprising:

(1) culturing the transformant of claim 10; and
(2) isolating the 1-deoxynojirimycin synthesis-related polypeptide from the culture.

**13.** A method for producing 1-deoxynojirimycin, comprising

(1) culturing the transformant of claim 10; and
(2) isolating 1-deoxynojirimycin from the culture.

[Fig. 1]

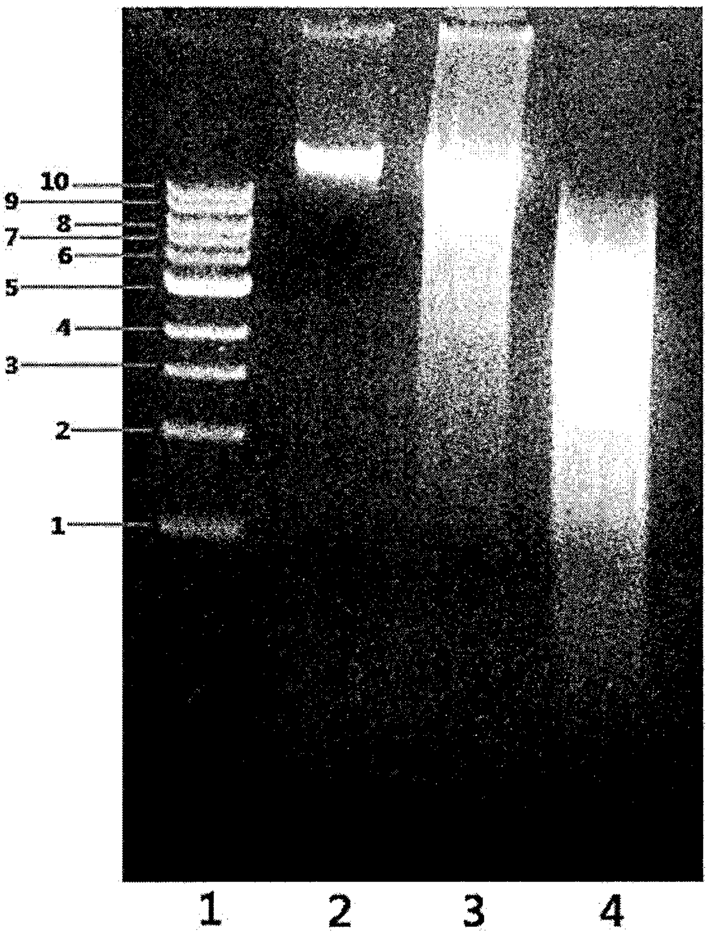

[Fig. 2]

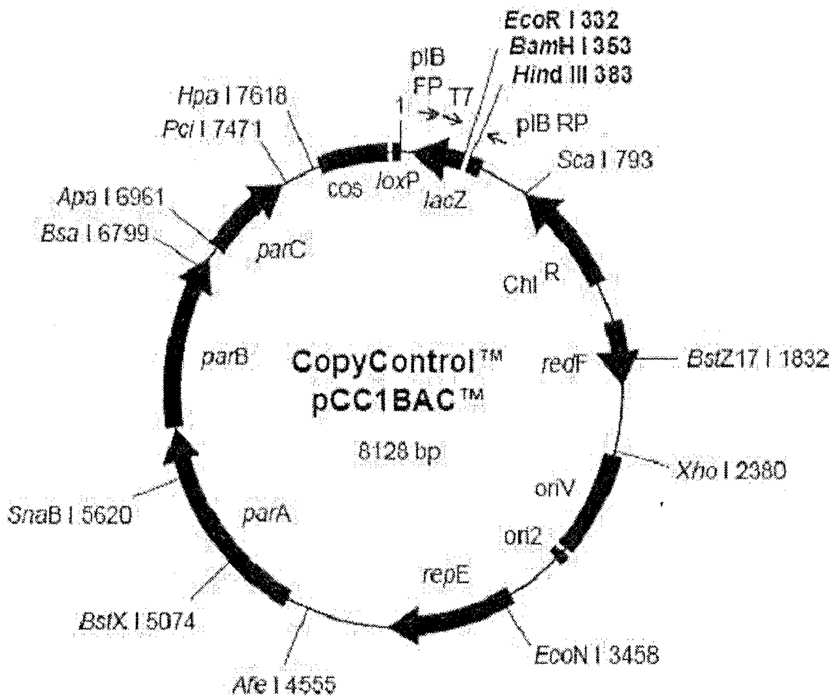
EcoR I 332
BamH I 353
Hind III 383
pIB FP
T7
pIB RP
Hpa I 7618
Pci I 7471
1
Sca I 793
Apa I 6961
Bsa I 6799
cos
loxP
lacZ
parC
Chl R
parB
CopyControl™
pCC1BAC™
8128 bp
redF
BstZ17 I 1832
Xho I 2380
oriV
SnaB I 5620
parA
ori2
repE
BstX I 5074
EcoN I 3458
Afe I 4555

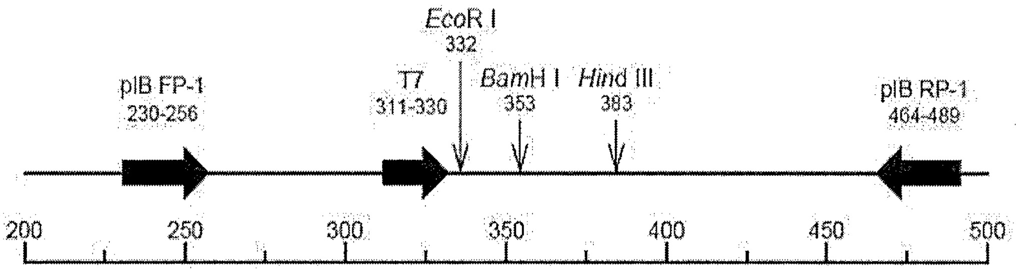
EcoR I
332
pIB FP-1
230-256
T7
311-330
BamH I
353
Hind III
383
pIB RP-1
464-489
200
250
300
350
400
450
500

[Fig. 3]

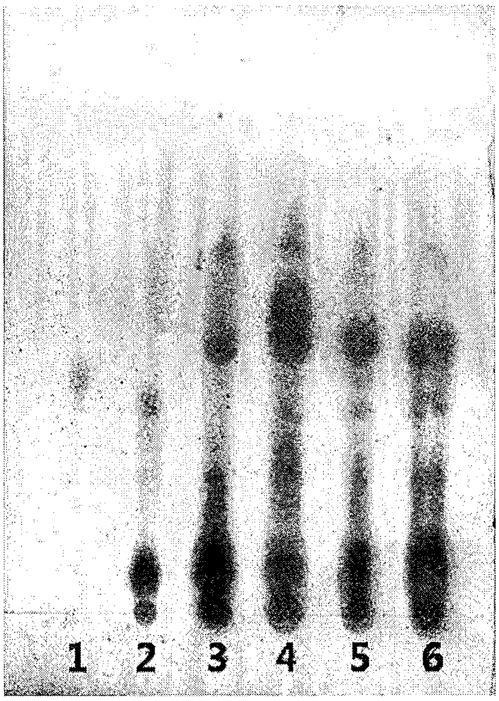
1
2
3
4
5
6

[Fig. 4a]

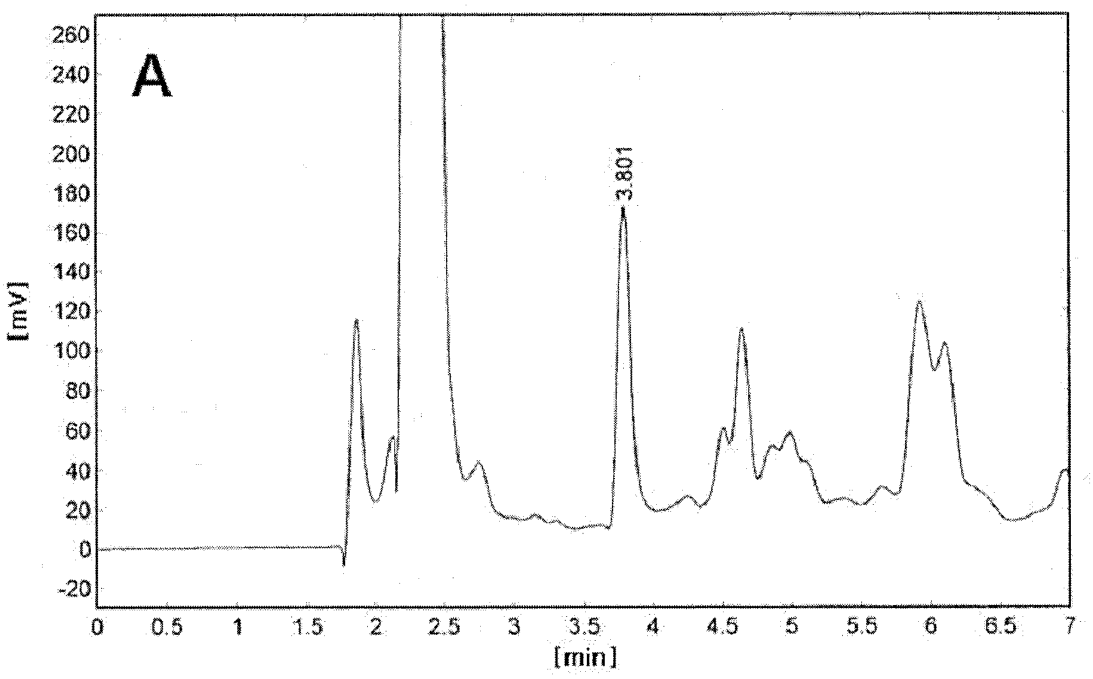
A
3.801
[mV]
[min]
260
240
220
200
180
160
140
120
100
80
60
40
20
0
-20
0
0.5
1
1.5
2
2.5
3
3.5
4
4.5
5
5.5
6
6.5
7

[Fig. 4b]

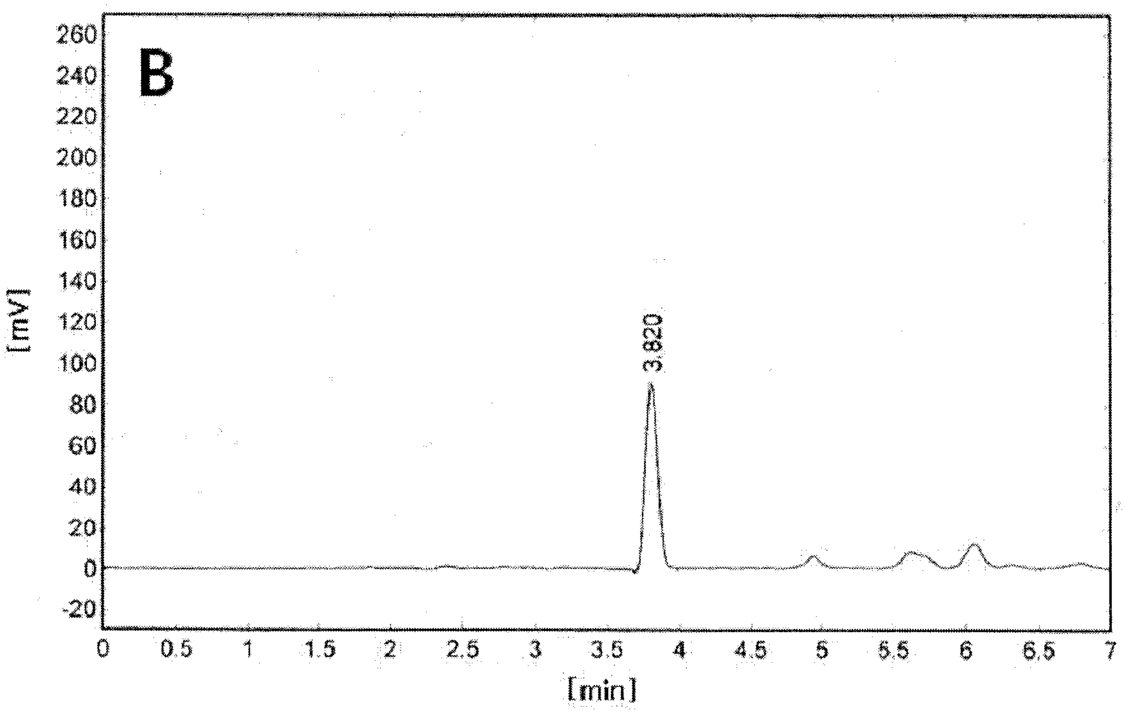
B
[mV]
3.820
[min]

[Fig. 5]

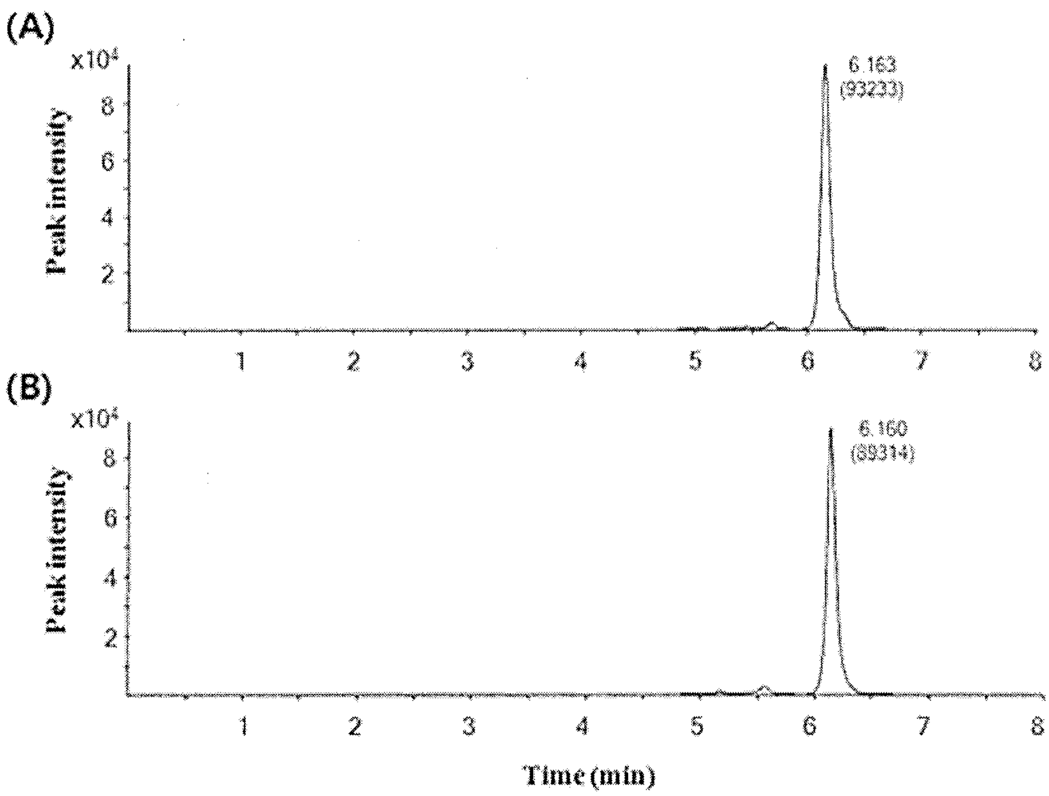
(A)
x10⁴
Peak intensity
6.163
(93233)
(B)
x10⁴
Peak intensity
6.160
(89314)
Time (min)

[Fig. 6]

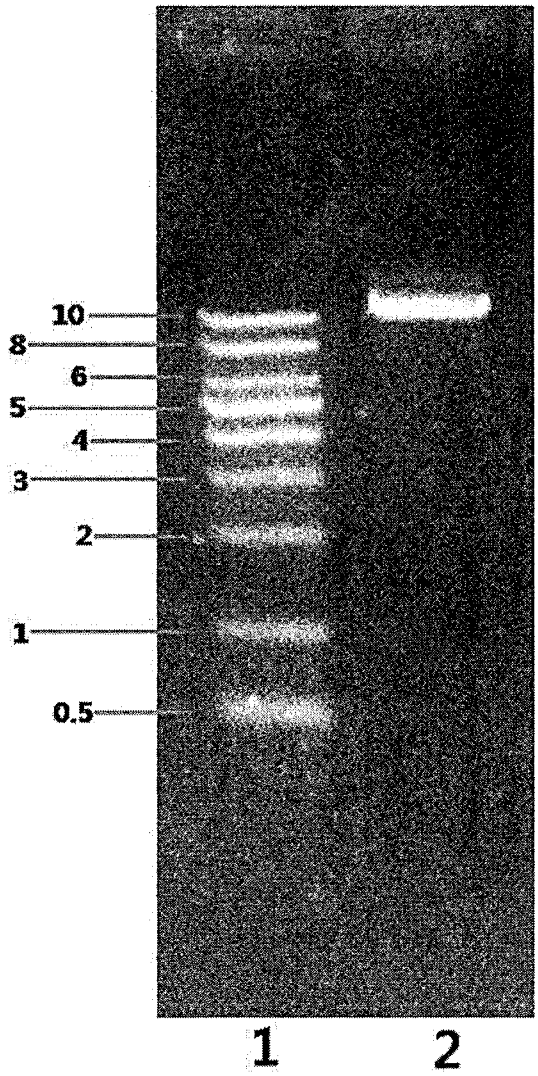

[Fig. 7]

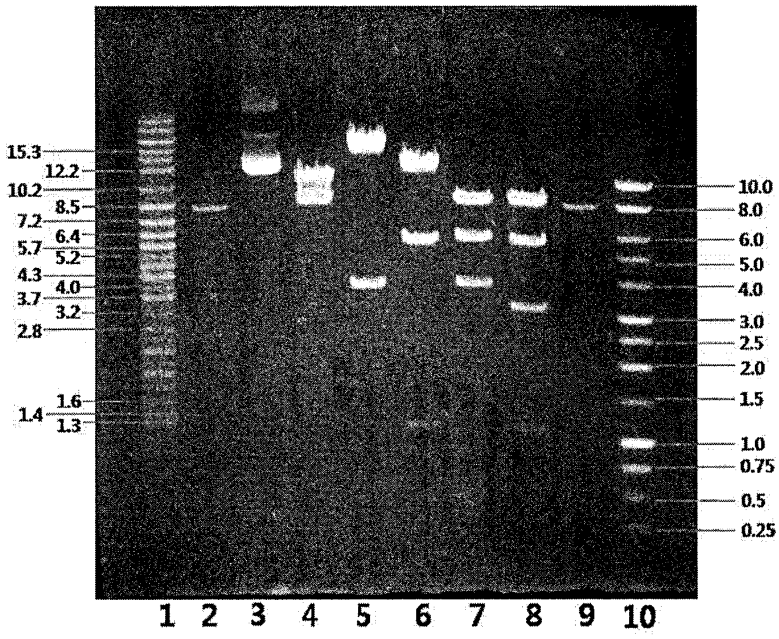


[Fig. 8]

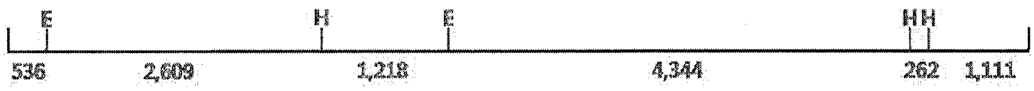


[Fig. 9]

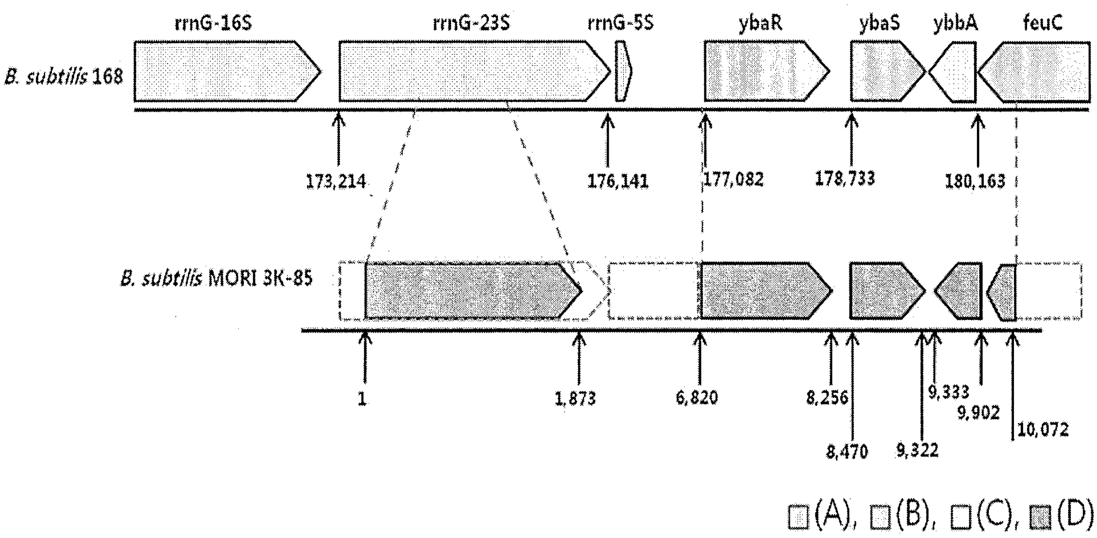


A; RNAs, B; cell envelope and cellular processes, C; similar gene in other organisims or B. subtilis,
D; determined the sequence of the inserts clone

[Fig. 10]

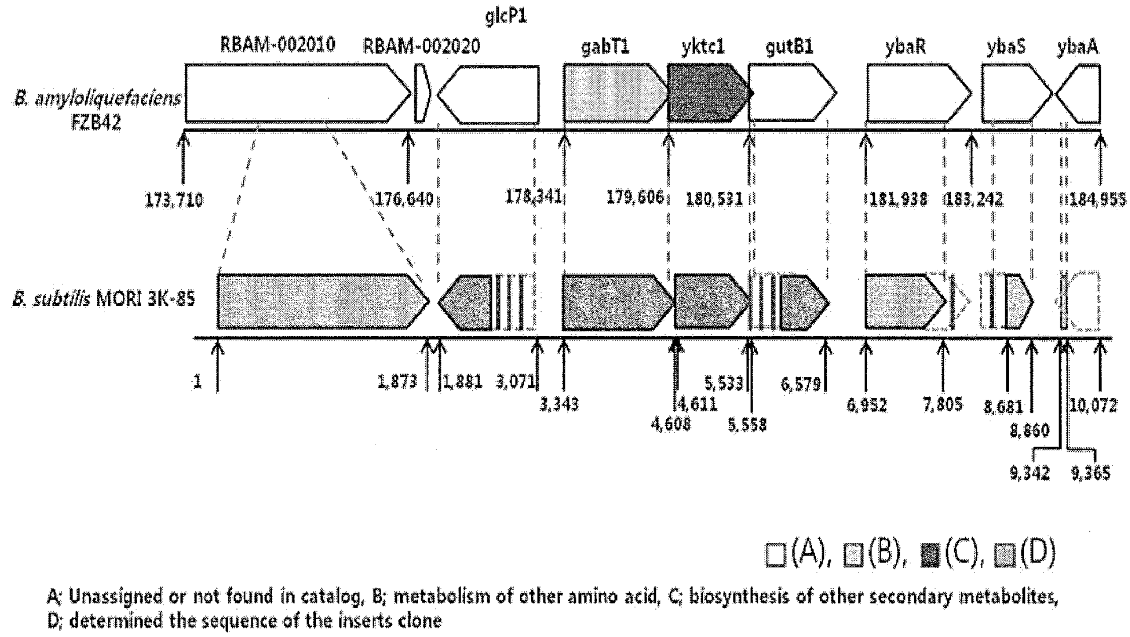
glcP1
RBAM-002010
RBAM-002020
gabT1
yktc1
gutB1
ybaR
ybaS
ybaA
B. amyloliquefaciens FZB42
173,710
176,640
178,341
179,606
180,531
181,938
183,242
184,955
B. subtilis MORI 3K-85
1
1,873
1,881
3,071
3,343
4,608
4,611
5,533
5,558
6,579
6,952
7,805
8,681
8,860
9,342
9,365
10,072
(A), (B), (C), (D)
A; Unassigned or not found in catalog, B; metabolism of other amino acid, C; biosynthesis of other secondary metabolites, D; determined the sequence of the inserts clone

[Fig. 11]

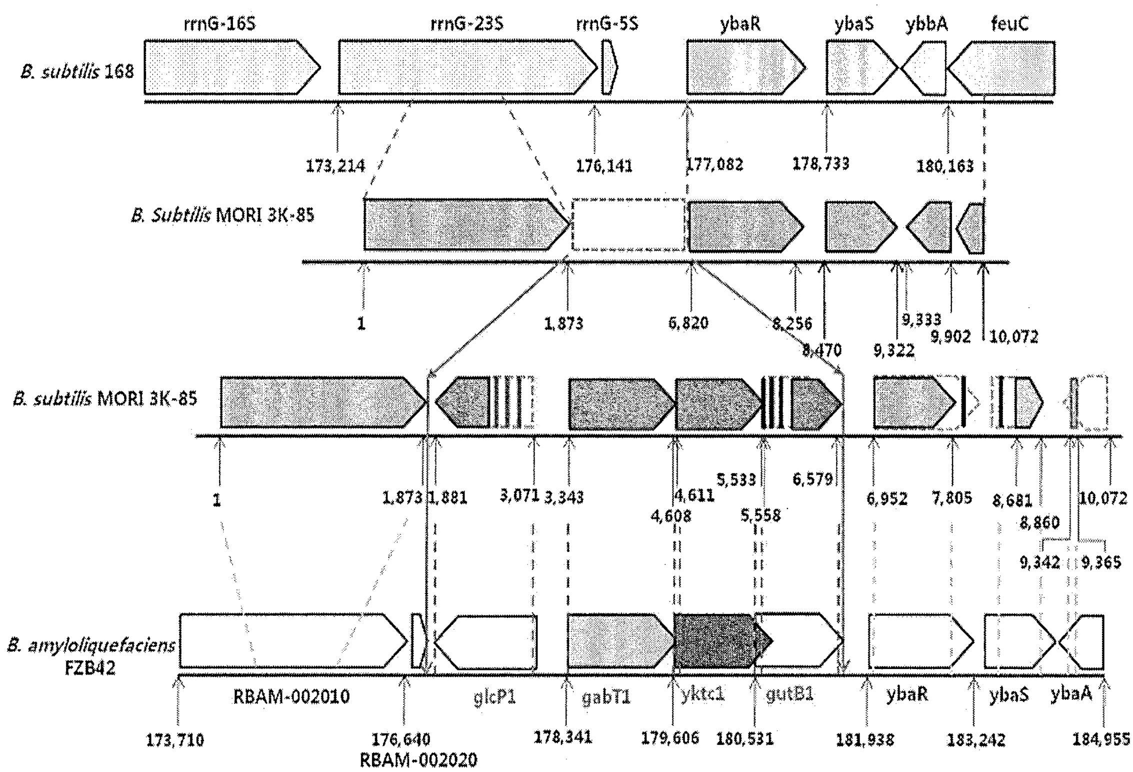
rrnG-16S
rrnG-23S
rrnG-5S
ybaR
ybaS
ybbA
feuC
B. subtilis 168
173,214
176,141
177,082
178,733
180,163
B. Subtilis MORI 3K-85
1
1,873
6,820
8,256
8,470
9,322
9,333
9,902
10,072
B. subtilis MORI 3K-85
1
1,873
1,881
3,071
3,343
4,608
4,611
5,533
5,558
6,579
6,952
7,805
8,681
8,860
9,342
9,365
10,072
B. amyloliquefaciens FZB42
RBAM-002010
glcP1
gabT1
yktc1
gutB1
ybaR
ybaS
ybaA
173,710
176,640
RBAM-002020
178,341
179,606
180,531
181,938
183,242
184,955

[Fig. 12]

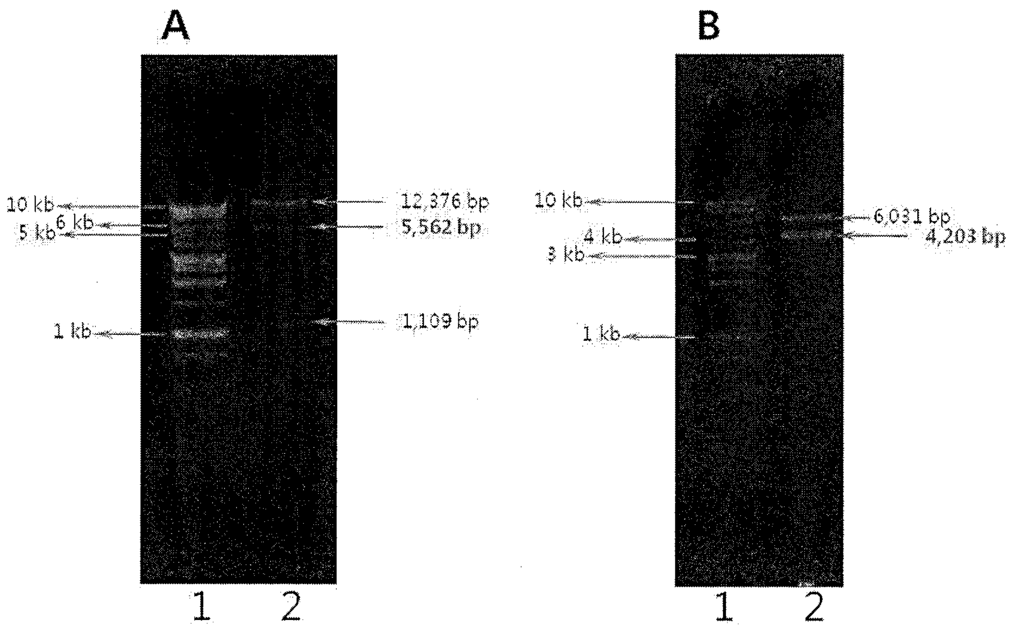


[Fig. 13]

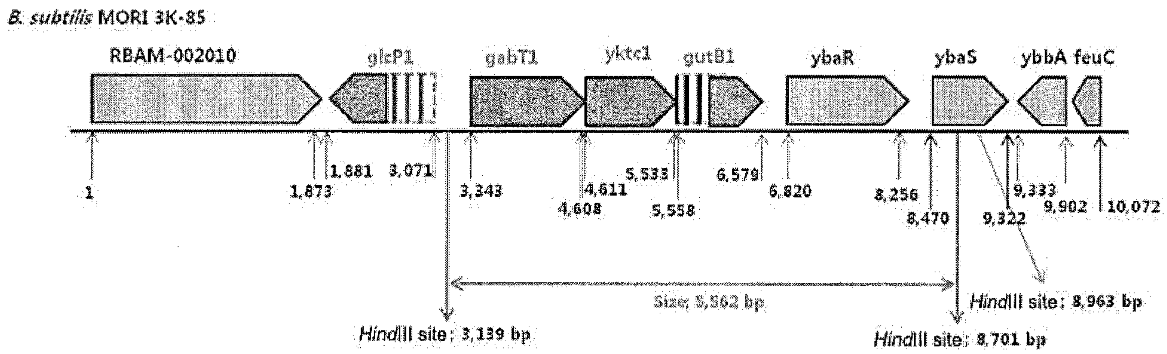


[Fig. 14]

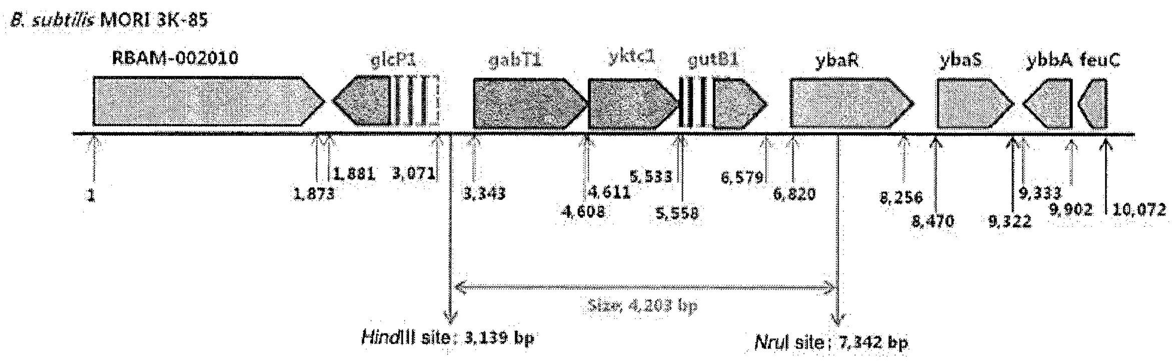

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ASANO, N. et al.** *Carbohydr. Res.,* 1996, vol. 293, 195-382
- **ASANO, N. et al.** *Phytochemistry,* 2000, vol. 53, 397-382
- **ASANO, N. et al.** *J. Agric. Food Chem.,* 2001, vol. 49, 4208-4213
- **EVANS, S. V. et al.** *Phytochemistry,* 1985, vol. 24, 1953-1955
- **MURAO, S. ; MIYATA, S.** *Agric. Biol. Chem.,* 1980, vol. 44, 219-221
- **YAMADA, H. ; OYA, I. ; NAGAI, T.** *Shoyakugaku Zasshi,* 1993, vol. 47, 47-55
- **EZURE, Y. et al.** *Agric. Biol., Chem.,* 1985, vol. 49, 1119-1125
- **HARDICK, D. J. ; HUTCHINSON, D. W.** *Biochemistry,* 1993, vol. 49, 6707-6716
- **SHIBANO, M. et al.** *Phytochemistry,* 2004, vol. 65, 2661-2665
- **HARDICK, D. J. et al.** *Tetrahedron,* 1992, vol. 48, 6285-6296
- **OLDEN, K. et al.** *Pharmacol. Ther.,* 1991, vol. 50, 285-290
- **TAYER, D et al.** *AIDS,* 1991, vol. 5, 693-698
- **SCOFILED, A. M. et al.** *Life Sci.,* 1986, vol. 39, 29-32
- **KIM, J. W. et al.** *Chromatography,* 2003, vol. 1002, 93-99
- **BARBE, V et al.** *Microbiology,* 2009, vol. 155, 1758-1775
- **KUNST, F. et al.** *Nature,* 1997, vol. 390, 249-256
- **LIU, H. et al.** *Microbiology,* 1997, vol. 143, 2763-2767
- **CHEN, X. H. et al.** *Nat. Biotechnol.,* 2007, vol. 25, 1007-1013

SEQUENCE LISTING

<110> BIOTOPIA CO., LTD.

<120> Polypeptide associated with the synthesis of 1-deoxynojirimycin and use therefor

<130> PCB105033BTP

<150> KR 10-2010-0086687
<151> 2010-09-03

<160> 20

<170> KopatentIn 1.71

<210> 1
<211> 10072
<212> DNA
<213> Fragment of glcP1-gabT1-yktc1-gutB1-ybaR-ybaS-ybbA from Bacillus subtilis MORI 3K-85

<400> 1
gagtacggcg gaacacgtga aattccgtcg gaatccggga ggaccatctc ccaaggctaa 60

atactcccta gtgaccgata gtgaaccagt accgtgaggg aaaggtgaaa agcaccccgg 120

aaggggagtg aaagagatcc tgaaaccgtg tgcctacaag tagtcagagc ccgttaacgg 180

gtgatggcgt gccttttgta gaatgaaccg gcgagttacg atcccgtgca aggttaagca 240

gaagatgcgg agccgcagcg aaagcgagtc tgaatagggc gcatgagtac gtggtcgtag 300

acccgaaacc aggtgatcta cccatgtcca gggtgaagtt caggtaacac tgaatggagg 360

cccgaaccca cgcacgttga aaagtgcggg gatgaggtgt gggtaggggt gaaatgccaa 420

tcgaacctgg agatagctgg ttctctccga aatagcttta gggctagcct caaggtaaga 480

gtcttggagg tagagcactg attggactag ggcccctac cgggttaccg aattcagtca 540

aactccgaat gccaatgact tatccttggg agtcagactg cgagtgataa gatccgtagt 600

cgaaagggaa acagcccaga ccgccagcta aggtcccaaa gtatacgtta agtggaaaag 660

gatgtggagt tgcttagaca accaggatgt tggcttagaa gcagccacca tttaaagagt 720

gcgtaatagc tcactggtcg agtgactctg cgccgaaaat gtaccggggc taaacgtatc 780

accgaagctg cggactgttc ttcgaacagt ggtaggagag cgttctaagg gctgtgaagc 840

cagaccggaa ggactggtgg agcgcttaga agtgagaatg ccggtatgag tagcgaaaga 900

ggggtgagaa tcccctccac cgaatgccta aggtttcctg aggaaggctc gtccgctcag 960

ggttagtcgg gacctaagcc gaggccgaaa ggcgtaggcg atggacaaca ggttgatatt 1020

cctgtaccac ctcctcacca tttgagcaat gggggacgc aggaggatag ggtaagcgcg 1080

gtattggata tccgcgtcca agcagttagg ctgggaaata ggcaaatccg tttcccataa 1140

ggctgagctg tgatggcgag cgaaatatag tagcgaagtt cctgattcca cactgccaag 1200

aaaagcctct agcgaggtga gaggtgcccg taccgcaaac cgacacaggt aggcgaggag 1260
agaatcctaa ggtgatcgag agaactctcg ttaaggaact cggcaaaatg accccgtaac 1320
ttcgggagaa ggggtgctct gttagggtgc aagcccgaga gagccgcagt gaataggccc 1380
aggcgactgt ttagcaaaaa cacaggtctc tgcgaagccg taaggcgaag tataggggct 1440
gacgcctgcc cggtgctgga aggttaagag gagcgcttag cgtaagcgaa ggtgcgaatt 1500
gaagccccag taaacggcgg ccgtaactat aacggtccta aggtagcgaa attccttgtc 1560
gggtaagttc cgacccgcac gaaaggcgca acgatctggg cactgtctca acgagagact 1620
cggtgaaatt atagtacctg tgaagatgca ggttacccgc gacaggacgg aaagaccccg 1680
tggagcttta ctgcagcctg atattgaatg ttggtacagc ttgtacagga taggtaggag 1740
ccttggaaac cggagcgcca gcttcggtgg aggcatcggt gggatactac cctggctgta 1800
ttgaccttct aacccgccgc ccttatcggg cggggagaca gtgtcaggtg ggcagtttga 1860
ctggggcggt cgcatgttga tcatgctggg gattgtatta gtattaaaaa tattagagaa 1920
cttgaaaagc agaaagctcg ataaaatgaa tactgaagag tcgtaaataa ccaaaatgct 1980
gtttgtacgg ggaactcatc tagactccat ccgactgcga ttggcaggat agaaccgcct 2040
aatccattgg cagcgagcag catgcttgtc gtttgttctg tttttccagg gtagaattga 2100
ttggtgataa ttaaagcaat ggcaaacata ccagccataa ataaccctaa gaagaaacac 2160
agaacaaacc ctgtaaaagc cgtattacta attgctaaaa tacaaagtga aatgaggccg 2220
ccgatgttaa tcagcctgag ataacgatga taggttagtt tttcggaaac cgggccagtc 2280
agtaaacggc caatgatcat gccgatccaa tatattgtta ccgtcaatgt tgctaaggag 2340
ttagagatct cccacttttc agcgaagatg gaagggataa agtgaacaat cgatacttca 2400
ctgcctccat aaaaaaagaa gtaagcagct gctaaagaaa taaacagtaa gccatgtttt 2460
cgaaaactga taggtgcttg ttgtttacca ttgcccgaag tgtttgtttg acggatgagt 2520
aaatcgtcta aactgccaaa actcattcgg ctccaaacaa gcatcaaaag aaatgaactt 2580
aaccccaata caagaaaggc caatgcccat acattattgc tgattaagaa accagacagc 2640
aacggcatga aaagggctcc taaaccgtac gctacttcca tcttactcat ggcaattgct 2700
tgtttttctt taattgctgt taggataatt gttcctacac aagattctac gagtccggcg 2760
ccaagtccag ccatactgac aagaagaaaa agcagaggcc atggaggaag gaaataaata 2820
aacaattcgc agctgatcat ggcaagtcca ataacaacta cttttcttcg gcccaatctc 2880
tttactgcat aaggcattcc cagtacacca attaaaaacc ccataaattg cgaaaatact 2940
aatactccgc cgttactgta gctttgtgcg taatgggaaa gcaactccgg caggacagca 3000
ccaaggataa cgagagtaaa ggcgattgat ccgtaagata aacatccaat ccacagtaaa 3060

cgagttttca ttcattaatc tcctttaata aaaataatac tcacctagga tttacataat 3120
aggactggta gtaaaaataa gctttttact ctgtttgtat ttagtttttt tacaataaac 3180
ccatttattt ctatcataac atatgaagtt tgaatagaaa ccccccatgc aaacgttaac 3240
aaatagttaa caatttttac tatttatcta gttgacatta atttctagaa tttatacgat 3300
gattaacgag ttttacatat tttatgaata ggggtgttgg tagtggggac taaagaaatt 3360
acgaatccag acagtttgta ttacaaagtg gatgacgttg tcatggaaag aggtgaaggc 3420
atatatttgt acgattcaga gggcaatgag tatattgatt gtgcctctgc tactttcaac 3480
ttaaatttgg gttataccaa taaagaagtt attgatacag tcaaagaaca ggctgaccag 3540
ctgatacacg tcacttcttc ctaccaaacc aacgccgtta ataaattagc tgaaaaactt 3600
gtagaaatct cccccgacaa tctaactaaa gttcacccta aggttagcag cggctctgct 3660
gcgaatgaag gagctattaa aatggctcaa aactattctg gaaaaacaga tgtcatttct 3720
ttattccgaa gccaccttgg ccaaacgtat atgatgtctg cgttatccgg aaattcattt 3780
cgaagagagc cattcccccc tcagttttct tttggcttac aggtgcctga cccctattgc 3840
aaccgttgtt tttacaatca gaagccagat tcatgcggaa tgctttgtgt agaaagaatt 3900
aatgatttta ttgaatatgc gagtaacgga aaaattgccg cgatgattat tgaaccgatt 3960
tccggtaacg gaggaaacat cgttccgcct aaggagtact ttaagcaatt aagaaagctc 4020
tgtgatgagc atgatattgc acttattttt gatgaaattc aaaccggatt tggccggaca 4080
ggcaagatgt ttgcggctga ttactttgat gtgaaaccga atatgatgac tgttgcaaaa 4140
ggattgggag gcacgggatt ccaggttgcc gccaccctca cagaggaaaa atacatggga 4200
ttagccggcc acaatcactc ttttacttat ggctcgaacg tgatggcctc ggcagcagct 4260
tgtaagacaa tcgaaatcat gcagcggccg ggcttcttag aaaatgtaac aactgtcggg 4320
aattacatta tggattcctt agagcacatg aagaaagaat tcacatttat tgctgacgtc 4380
agaggcgtag gtttgatgat cggtgttgaa attgtaaaag agaataatga gcgtgatgta 4440
gagctgacca attacattgc aaaacgggct atggattatg ggttaatttt gcggacttcc 4500
cgttacggat tcggaaatgt atttaaaatc cgtccgcctc tcacgattac acttagtgag 4560
gctgaggtgc tttgtttcaa acttcgtaag ctattggagg atatcaagtg aaagaatata 4620
ttatagagct tggaacattt gtttatgaag aagtgaaggg acagaaagga acactgaaaa 4680
accggttagt taatgggtat tccccaggcg gagatgcaca gtttaacatt gatgccgctg 4740
ccgagaatgc agtgctggaa tatgtgaata gcaaagagga atcagttgcg ttttatacgg 4800
aagatggcgg cctgaaggta tttggggaca atcctcaata tattttgatc gttgatccga 4860
ttgacggcac ccgcccggca gctgcaggac ttgaaatgtc ttgtatctct atcgcattag 4920
ctgcatacaa gcctgatgca aagattaagg atattgaatt tgcttttctg cttgagctga 4980

aaacaggtgc ctatatgtac gcggatgttt attctgaagg gatttattat gaaggctatc 5040
gtggcaccct gccaaatctg agcaaagtga cagacattaa acatatgttc tggagccttg 5100
agtttaatgg gcatcccgcg cacctgatga ttgatgcgta cggtcatttg attgatcagt 5160
cagcgaataa tggcggcgta tttgttttca atagcgcttc ttattcaatc tccaggatta 5220
ttacagggca gatggatgcc tatgtggata tcggcaatcg tctattaaaa gatgatcctg 5280
cgctgctgaa agattttcag gatgtgggga atgggcaggt cctgcatctg tttccatatg 5340
atattgccgc aagtgtgttt ttggcgaaaa aagcaggtgt tgtgattact gatgcgtatg 5400
gaaagtcctt ggatgataca cttctgacgg atcttagcta caataaccag cagtcatgca 5460
tcgctgcatc aacgaaggag ctgcatcaga agctgctgga tcaaattcgc tgggacagaa 5520
aggaagagac atatgaaagc gttggtctgg actcctaatg atcggcttga aatgcaagaa 5580
gtagaagaac ctcaaatcaa aaaaatgaac gatgtgaaag ttaaaatata cgggacaggc 5640
atctgcggaa cggatttaaa tgttctaaaa ggaaagatgc atgcgactca caatatgatc 5700
ctaggccacg aatctgtggg agtggtgaca gaaacagggc ctgatgttaa aaacgtcaag 5760
cctggtgatc gcgtggtaat tgatccgact cagttttgcg ggaagtgtta ttattgccgg 5820
aaaggtttaa cttgttattg tgaaacgttt gaagactggc agctgggatt aggggcgcat 5880
ggcacttttg ccgattatta cgtaggcgag gaccgtttta tgtataaaat cccggacaat 5940
atggattggg aacgagccac tttgattgag ccgctttcct gtgtattaaa tgtgatagag 6000
cgggctgcaa taaaacctga cgattctgta cttgtattag ggtcagggcc gattgggctg 6060
cttgttcaaa tgatggtgaa aaaactatca aggctgaccg ttgcgaccga gatcggagag 6120
tatcggtcag aagcggcacg ccggatatct gactatgttt accacccgca ggatttaacg 6180
gcagatgagg tcaggcggat aaaccaagga agaacatttg atgtgatctt tgacgcgatc 6240
ggcaatcagc ttgattgggc atatccgtta attgacaagg gcggaaggct tgtgccgatg 6300
gggtttgatg atacgtatga aatgaaaatc aggccttttc agctgctttc taacggggtg 6360
acgattgttg gaaccggaga ggctcgacaa atcatggagg atgcggtatc atgtgccgcg 6420
gacatgcctc agctttctga actgattacg gagaaaaccc cgcttgagaa ctatgaggct 6480
gccatccagg aattgatggg catagatccg ttgtcaaatg agagaaaaga tattgccgca 6540
gttaaaacga ttcttgtttc ccatccggat atgatataat gcagcgatat ggctcaaact 6600
tagtttgagc catatttttc ttcagcattc accttgcgta tttgaataaa atttaatatg 6660
atactctttg tataataact ccttgcctca atacaatata ctcaacgttt cccctttttc 6720
tccgatcgtt ttccttttca ttttctcccg taaaataaag aaagctccca ttacaacaga 6780
tacggtgcgg atttattttt taaaaaggag tcgttatatt tgaataatag ttttacatta 6840

aaacagcaat ggttcggaaa tatccgaaaa gacattcttg ctggcatttt agttgcatta 6900
gcgttaatac ctgaagcgat tggcttttct attatagccg gtgttgatcc gatggtcggt 6960
ctttacgctt cattttgtat agccattatt atttctattt ttggcggcag accaggcatg 7020
atttcagctg cgactggctc aatggcggtc gttatggtgt cgctggttgc tgatcatggg 7080
cttcagtatt tatttgcggc tacgattttg acaggaatca ttcaggtgat tttaggcatc 7140
agtaaaatag ctagattaat gaaattcatc ccgcgttctg tgatgatcgg gtttgtgaat 7200
gcgctggcga tccttatctt ctctgcgcag ctccctcagt ttgaaggtgc ttcttggagc 7260
atgtatgcga tgcttgccgg gtcgttggtt attatttatg tgctgccgag gtttacgact 7320
gcggttccgt ctccgcttgt cgcgattatt gtgatgacga ttattgctgt cactttccat 7380
gtggatgtgc ggacggtcgg ggatatgggg aatatttcta gttcattgcc tcatttctta 7440
atcccggatg ttccgtttac ctttgaaaca ctgcaaatca tttttcctta ttccatcgct 7500
ttagcgtttg tcgggttgct agaatcttta ctgacagcac agattattga tgagatgacg 7560
gatacggata gtgacaaaaa taaagagagc cgcggacagg gaattgcgaa tatcgtgacc 7620
gggttctttg gcggtatggc tggctgcgct atgatcggac aatctgtcat taatacgaaa 7680
gcgggcggac gaggacgctt gtcagcattt gttgccggtg catttctgat gtttttaatt 7740
gcggtgttaa gtcaggttgt agtgaaaatt ccgatggctg cgttagtggc tgttatggtg 7800
atggtttctg tcggcacatt tgattggtct tctataaaag gccttaaaaa agcgccgctc 7860
accgactcga ttgtcatggt tgtgactgtg atgacggttg ttgtgacgga tgacttatct 7920
aaaggcgtat tcgtcggcgt tctcttaagt gccgtattct ttgtggcaaa aatctcgaag 7980
ctgaaaattg tttcacatgc agaggatcaa aagctgagaa cgtatcaagt gaagggggcaa 8040
atcttttttg cgtcagtgac tgatcttacg aatgcgttta tttatcaaga ggatattgaa 8100
cgggtcgtga ttgacttaac tgaatctcac gtttgggatg attcaggagc ggcagcgctt 8160
gataagattg ttgcgaaatt taaagaacaa ggcattgaag ctgagttaaa aggtttgaac 8220
aatgcgagta aagcccttat gaaacagatg acataaaata gggaagtcca gatgaacgag 8280
atctgggctt ttttatataa catacggttg aacagcattg atataataga gagaagagaa 8340
attttaaata ggggacttgc atatgatgct tcagaagctg aaccgtgtat tggggagagt 8400
gatgccgctt ttaacgccga ctagtgtggt gctgggcgtg ctgctgtctc agtttttaaa 8460
tgggtatgaa tgggctgtgc cttggatctt tgcttttatt acgtttgcag gcagtttgag 8520
cgctaacttt caatctttga gacacgcgtt gtctcatcct ctacctatga ttcttgcact 8580
atttgttctt catattttta tgccgctttt tgcttggggc agcggccatc ttatttttaa 8640
aggcgatcct ttgacgatca ccggtttaac attggctgtt gtgattccta cggggattac 8700
aagcttgatt tgggcagcga tgtacaaagg gaacgtcggt ttgacgcttt caattatctt 8760

agttgatact gtgctgtcac cgctaatcgt accgctgagc ctttcattgc ttgccggcgc 8820

tcaggttcat atggatgtgt ggggaatgat gaaagggctg attgtgatgg tcgtgattcc 8880

ttcttttctg ggcatgctgt ttaatcagat atcatctccc gaaagaactg catctgtgag 8940

cagtgcgctg tcaccttttt caaagctttg cctgatggct gtgattgcga ttaacagttc 9000

agcgattgct ccttatttca aagcggttga tttgagattc ataggaatag ctttgacggt 9060

tttctttatc gcgttaacag gctatgccgc tgcatggctg atcgggaaaa tgatgaaaag 9120

gcgccaggag gaaattgtct ctcttatttt tacgggaggc atgagaaata tcagtgccgg 9180

cgcagttctt gccgttacgt tcttcccgtc tcaggttgcc gttcccgttg tcatcggcat 9240

gctgtttcag cagatactgg ctgctttgtt tggctatatg ctgaatcgtt ttgaattaaa 9300

acctgtattg cagaaagcat gaaaaaaacc gctcataggg ttgagcggtt ttttccgtat 9360

agccatttga gcgcgatgat gaactgtttg cagaagtact tgcggtggtg caccgcccct 9420

ttttcaattg aaagacacag ttgctcttca gtgaagcctt tttcttttaa actttggtgc 9480

acttgctttg tattctgcag catattccgc tgaatggatt gtttctcgcg gccttcgtta 9540

ctgccgacag acataaaaac acttgaagtg ccgggcttta aagaagataa atgaacggtt 9600

tccggtgcgt tctcgtacca gtatgaacct gagatagaac ctatttttcc aaacaccgac 9660

gggtacttta ataaggcgaa cattgagatg aggccgccta atgaggcacc gatcattcct 9720

cttgattgcg gttccctggt gacgttccag ttttcttcga taaacggaat gaactgtttc 9780

gttatattag acagatagtg gtatcccatc ccgccgaagt ccgggaaccg atcactaagt 9840

gatgcagccg gccaaggcgt gtattcattt agccggtttt cgggtttgat accgatcagc 9900

acgagttcag gcagccttcg ctgtagaaat gcgctttcta ataattcgat ttggtcttga 9960

aacaaagaac tgccatcctg cacgtaaaca gcaggaaatg ccctgctgcc tggactgtag 10020

gaaggaggga ggtacagcgt gaacctcctt ttcccggctt tatgctctga aa 10072

<210> 2
<211> 1191
<212> DNA
<213> glcP1 from Bacillus subtilis MORI 3K-85

<400> 2
tcatgctggg gattgtatta gtattaaaaa tattagagaa cttgaaaagc agaaagctcg 60

ataaaatgaa tactgaagag tcgtaaataa ccaaaatgct gtttgtacgg ggaactcatc 120

tagactccat ccgactgcga ttggcaggat agaaccgcct aatccattgg cagcgagcag 180

catgcttgtc gtttgttctg tttttccagg gtagaattga ttggtgataa ttaaagcaat 240

ggcaaacata ccagccataa ataaccctaa gaagaaacac agaacaaacc ctgtaaaagc 300

cgtattacta attgctaaaa tacaaagtga aatgaggccg ccgatgttaa tcagcctgag 360

```
ataacgatga taggttagtt tttcggaaac cgggccagtc agtaaacggc caatgatcat      420
gccgatccaa tatattgtta ccgtcaatgt tgctaaggag ttagagatct cccacttttc      480
agcgaagatg gaagggataa agtgaacaat cgatacttca ctgcctccat aaaaaaagaa      540
gtaagcagct gctaaagaaa taaacagtaa gccatgtttt cgaaaactga taggtgcttg      600
ttgtttacca ttgcccgaag tgtttgtttg acggatgagt aaatcgtcta aactgccaaa      660
actcattcgg ctccaaacaa gcatcaaaag aaatgaactt aaccccaata caagaaaggc      720
caatgcccat acattattgc tgattaagaa accagacagc aacggcatga aaagggctcc      780
taaaccgtac gctacttcca tcttactcat ggcaattgct tgtttttctt taattgctgt      840
taggataatt gttcctacac aagattctac gagtccggcg ccaagtccag ccatactgac      900
aagaagaaaa agcagaggcc atggaggaag gaaataaata aacaattcgc agctgatcat      960
ggcaagtcca ataacaacta cttttcttcg gcccaatctc tttactgcat aaggcattcc     1020
cagtacacca attaaaaacc ccataaattg cgaaaatact aatactccgc cgttactgta     1080
gctttgtgcg taatgggaaa gcaactccgg caggacagca ccaaggataa cgagagtaaa     1140
ggcgattgat ccgtaagata aacatccaat ccacagtaaa cgagttttca t              1191
```

<210> 3
<211> 1269
<212> DNA
<213> gabT1 from Bacillus subtilis MORI 3K-85

<400> 3

```
gtggggacta aagaaattac gaatccagac agtttgtatt acaaagtgga tgacgttgtc       60
atggaaagag gtgaaggcat atatttgtac gattcagagg gcaatgagta tattgattgt      120
gcctctgcta ctttcaactt aaatttgggt tataccaata aagaagttat tgatacagtc      180
aaagaacagg ctgaccagct gatacacgtc acttcttcct accaaaccaa cgccgttaat      240
aaattagctg aaaaacttgt agaaatctcc cccgacaatc taactaaagt tcaccctaag      300
gttagcagcg gctctgctgc gaatgaagga gctattaaaa tggctcaaaa ctattctgga      360
aaaacagatg tcatttcttt attccgaagc caccttggcc aaacgtatat gatgtctgcg      420
ttatccggaa attcatttcg aagagagcca ttccccccctc agttttcttt tggcttacag      480
gtgcctgacc cctattgcaa ccgttgtttt tacaatcaga agccagattc atgcggaatg      540
ctttgtgtag aaagaattaa tgattttatt gaatatgcga gtaacggaaa aattgccgcg      600
atgattattg aaccgatttc cggtaacgga ggaaacatcg ttccgcctaa ggagtacttt      660
aagcaattaa gaaagctctg tgatgagcat gatattgcac ttatttttga tgaaattcaa      720
accggatttg gccggacagg caagatgttt gcggctgatt actttgatgt gaaaccgaat      780
atgatgactg ttgcaaaagg attgggaggc acgggattcc aggttgccgc caccctcaca      840
```

gaggaaaaat acatgggatt agccggccac aatcactctt ttacttatgg ctcgaacgtg 900

atggcctcgg cagcagcttg taagacaatc gaaatcatgc agcggccggg cttcttagaa 960

aatgtaacaa ctgtcgggaa ttacattatg gattccttag agcacatgaa gaaagaattc 1020

acatttattg ctgacgtcag aggcgtaggt ttgatgatcg gtgttgaaat tgtaaaagag 1080

aataatgagc gtgatgtaga gctgaccaat tacattgcaa aacgggctat ggattatggg 1140

ttaattttgc ggacttcccg ttacggattc ggaaatgtat ttaaaatccg tccgcctctc 1200

acgattacac ttagtgaggc tgaggtgctt tgtttcaaac ttcgtaagct attggaggat 1260

atcaagtga 1269

<210> 4
<211> 951
<212> DNA
<213> yktc1 from Bacillus subtilis MORI 3K-85

<400> 4
gtgaaagaat atattataga gcttggaaca tttgtttatg aagaagtgaa gggacagaaa 60

ggaacactga aaaaccggtt agttaatggg tattccccag gcggagatgc acagtttaac 120

attgatgccg ctgccgagaa tgcagtgctg gaatatgtga atagcaaaga ggaatcagtt 180

gcgttttata cggaagatgg cggcctgaag gtatttgggg acaatcctca atatattttg 240

atcgttgatc cgattgacgg cacccgcccg gcagctgcag gacttgaaat gtcttgtatc 300

tctatcgcat tagctgcata caagcctgat gcaaagatta aggatattga atttgctttt 360

ctgcttgagc tgaaaacagg tgcctatatg tacgcggatg tttattctga agggatttat 420

tatgaaggct atcgtggcac cctgccaaat ctgagcaaag tgacagacat taaacatatg 480

ttctggagcc ttgagtttaa tgggcatccc gcgcacctga tgattgatgc gtacggtcat 540

ttgattgatc agtcagcgaa taatggcggc gtatttgttt tcaatagcgc ttcttattca 600

atctccagga ttattacagg gcagatggat gcctatgtgg atatcggcaa tcgtctatta 660

aaagatgatc ctgcgctgct gaaagatttt caggatgtgg ggaatgggca ggtcctgcat 720

ctgtttccat atgatattgc cgcaagtgtg tttttggcga aaaaagcagg tgttgtgatt 780

actgatgcgt atggaaagtc cttggatgat acacttctga cggatcttag ctacaataac 840

cagcagtcat gcatcgctgc atcaacgaag gagctgcatc agaagctgct ggatcaaatt 900

cgctgggaca gaaaggaaga gacatatgaa agcgttggtc tggactccta a 951

<210> 5
<211> 1047
<212> DNA
<213> gutB1 from Bacillus subtilis MORI 3K-85

<400> 5

atgaaagcgt tggtctggac tcctaatgat cggcttgaaa tgcaagaagt agaagaacct 60

caaatcaaaa aaatgaacga tgtgaaagtt aaaatatacg ggacaggcat ctgcggaacg 120

gatttaaatg ttctaaaagg aaagatgcat gcgactcaca atatgatcct aggccacgaa 180

tctgtgggag tggtgacaga aacagggcct gatgttaaaa acgtcaagcc tggtgatcgc 240

gtggtaattg atccgactca gttttgcggg aagtgttatt attgccggaa aggtttaact 300

tgttattgtg aaacgtttga agactggcag ctgggattag ggcgcatgg cacttttgcc 360

gattattacg taggcgagga ccgttttatg tataaaatcc cggacaatat ggattgggaa 420

cgagccactt tgattgagcc gctttcctgt gtattaaatg tgatagagcg ggctgcaata 480

aaacctgacg attctgtact tgtattaggg tcagggccga ttgggctgct tgttcaaatg 540

atggtgaaaa aactatcaag gctgaccgtt gcgaccgaga tcggagagta tcggtcagaa 600

gcggcacgcc ggatatctga ctatgtttac cacccgcagg atttaacggc agatgaggtc 660

aggcggataa accaaggaag aacatttgat gtgatctttg acgcgatcgg caatcagctt 720

gattgggcat atccgttaat tgacaagggc ggaaggcttg tgccgatggg gtttgatgat 780

acgtatgaaa tgaaaatcag gccttttcag ctgctttcta acggggtgac gattgttgga 840

accggagagg ctcgacaaat catggaggat gcggtatcat gtgccgcgga catgcctcag 900

ctttctgaac tgattacgga gaaaaccccg cttgagaact atgaggctgc catccaggaa 960

ttgatgggca tagatccgtt gtcaaatgag agaaaagata ttgccgcagt taaaacgatt 1020

cttgtttccc atccggatat gatataa 1047

<210> 6
<211> 1437
<212> DNA
<213> ybaR from Bacillus subtilis MORI 3K-85

<400> 6
ttgaataata gttttacatt aaaacagcaa tggttcggaa atatccgaaa agacattctt 60

gctggcattt tagttgcatt agcgttaata cctgaagcga ttggcttttc tattatagcc 120

ggtgttgatc cgatggtcgg tctttacgct tcattttgta tagccattat tatttctatt 180

tttggcggca gaccaggcat gatttcagct gcgactggct caatggcggt cgttatggtg 240

tcgctggttg ctgatcatgg gcttcagtat ttatttgcgg ctacgatttt gacaggaatc 300

attcaggtga ttttaggcat cagtaaaata gctagattaa tgaaattcat cccgcgttct 360

gtgatgatcg ggtttgtgaa tgcgctggcg atccttatct tctctgcgca gctccctcag 420

tttgaaggtg cttcttggag catgtatgcg atgcttgccg ggtcgttggt tattatttat 480

gtgctgccga ggtttacgac tgcggttccg tctccgcttg tcgcgattat tgtgatgacg 540

attattgctg tcactttcca tgtggatgtg cggacggtcg ggatatggg gaatatttct 600

agttcattgc ctcatttctt aatcccggat gttccgttta cctttgaaac actgcaaatc 660

atttttcctt attccatcgc tttagcgttt gtcgggttgc tagaatcttt actgacagca 720

cagattattg atgagatgac ggatacggat agtgacaaaa ataaagagag ccgcggacag 780

ggaattgcga atatcgtgac cgggttcttt ggcggtatgg ctggctgcgc tatgatcgga 840

caatctgtca ttaatacgaa agcgggcgga cgaggacgct tgtcagcatt tgttgccggt 900

gcatttctga tgtttttaat tgcggtgtta agtcaggttg tagtgaaaat tccgatggct 960

gcgttagtgg ctgttatggt gatggtttct gtcggcacat ttgattggtc ttctataaaa 1020

ggccttaaaa aagcgccgct caccgactcg attgtcatgg ttgtgactgt gatgacggtt 1080

gttgtgacgg atgacttatc taaaggcgta ttcgtcggcg ttctcttaag tgccgtattc 1140

tttgtggcaa aaatctcgaa gctgaaaatt gtttcacatg cagaggatca aaagctgaga 1200

acgtatcaag tgaaggggca aatctttttt gcgtcagtga ctgatcttac gaatgcgttt 1260

atttatcaag aggatattga acgggtcgtg attgacttaa ctgaatctca cgtttgggat 1320

gattcaggag cggcagcgct tgataagatt gttgcgaaat ttaaagaaca aggcattgaa 1380

gctgagttaa aaggtttgaa caatgcgagt aaagccctta tgaaacagat gacataa 1437

<210> 7
<211> 853
<212> DNA
<213> ybaS from Bacillus subtilis MORI 3K-85

<400> 7
atgggctgtg ccttggatct ttgcttttat tacgtttgca ggcagtttga gcgctaactt 60

tcaatctttg agacacgcgt tgtctcatcc tctacctatg attcttgcac tatttgttct 120

tcatattttt atgccgcttt ttgcttgggg cagcggccat cttattttta aaggcgatcc 180

tttgacgatc accggtttaa cattggctgt tgtgattcct acggggatta caagcttgat 240

ttgggcagcg atgtacaaag ggaacgtcgg tttgacgctt tcaattatct tagttgatac 300

tgtgctgtca ccgctaatcg taccgctgag cctttcattg cttgccggcg ctcaggttca 360

tatggatgtg tggggaatga tgaaagggct gattgtgatg gtcgtgattc cttcttttct 420

gggcatgctg tttaatcaga tatcatctcc cgaaagaact gcatctgtga gcagtgcgct 480

gtcacctttt tcaaagcttt gcctgatggc tgtgattgcg attaacagtt cagcgattgc 540

tccttatttc aaagcggttg atttgagatt cataggaata gctttgacgg ttttctttat 600

cgcgttaaca ggctatgccg ctgcatggct gatcgggaaa atgatgaaaa ggcgccagga 660

ggaaattgtc tctcttattt ttacgggagg catgagaaat atcagtgccg gcgcagttct 720

tgccgttacg ttcttcccgt ctcaggttgc cgttcccgtt gtcatcggca tgctgtttca 780

gcagatactg gctgctttgt ttggctatat gctgaatcgt tttgaattaa aacctgtatt 840

gcagaaagca tga 853

<210> 8
<211> 570
<212> DNA
<213> ybbA from Bacillus subtilis MORI 3K-85

<400> 8
tcatagggtt gagcggtttt ttccgtatag ccatttgagc gcgatgatga actgtttgca 60

gaagtacttg cggtggtgca ccgccccttt ttcaattgaa agacacagtt gctcttcagt 120

gaagcctttt tcttttaaac tttggtgcac ttgctttgta ttctgcagca tattccgctg 180

aatggattgt ttctcgcggc cttcgttact gccgacagac ataaaaacac ttgaagtgcc 240

gggctttaaa gaagataaat gaacggtttc cggtgcgttc tcgtaccagt atgaacctga 300

gatagaacct atttttccaa acaccgacgg gtactttaat aaggcgaaca ttgagatgag 360

gccgcctaat gaggcaccga tcattcctct tgattgcggt tccctggtga cgttccagtt 420

ttcttcgata aacggaatga actgtttcgt tatattagac agatagtggt atcccatccc 480

gccgaagtcc gggaaccgat cactaagtga tgcagccggc caaggcgtgt attcatttag 540

ccggttttcg ggtttgatac cgatcagcac 570

<210> 9
<211> 396
<212> PRT
<213> glcP1 from Bacillus subtilis MORI 3K-85

<400> 9
Met Lys Thr Arg Leu Leu Trp Ile Gly Cys Leu Ser Tyr Gly Ser Ile
1 5 10 15

Ala Phe Thr Leu Val Ile Leu Gly Ala Val Leu Pro Glu Leu Leu Ser
20 25 30

His Tyr Ala Gln Ser Tyr Ser Asn Gly Gly Val Leu Val Phe Ser Gln
35 40 45

Phe Met Gly Phe Leu Ile Gly Val Leu Gly Met Pro Tyr Ala Val Lys
50 55 60

Arg Leu Gly Arg Arg Lys Val Val Val Ile Gly Leu Ala Met Ile Ser
65 70 75 80

Cys Glu Leu Phe Ile Tyr Phe Leu Pro Pro Trp Pro Leu Leu Phe Leu
85 90 95

Leu Val Ser Met Ala Gly Leu Gly Ala Gly Leu Val Glu Ser Cys Val
100 105 110

Gly Thr Ile Ile Leu Thr Ala Ile Lys Glu Lys Gln Ala Ile Ala Met
115 120 125

Ser Lys Met Glu Val Ala Tyr Gly Leu Gly Ala Leu Phe Met Pro Leu
130 135 140

Leu Ser Gly Phe Leu Ile Ser Asn Asn Val Trp Ala Leu Ala Phe Leu
145 150 155 160

Val Leu Gly Leu Ser Ser Phe Leu Leu Met Leu Val Trp Ser Arg Met
165 170 175

Ser Phe Gly Ser Leu Asp Asp Leu Leu Ile Arg Gln Thr Asn Thr Ser
180 185 190

Gly Asn Gly Lys Gln Gln Ala Pro Ile Ser Phe Arg Lys His Gly Leu
195 200 205

Leu Phe Ile Ser Leu Ala Ala Ala Tyr Phe Phe Phe Tyr Gly Gly Ser
210 215 220

Glu Val Ser Ile Val His Phe Ile Pro Ser Ile Phe Ala Glu Lys Trp
225 230 235 240

Glu Ile Ser Asn Ser Leu Ala Thr Leu Thr Val Thr Ile Tyr Trp Ile
245 250 255

Gly Met Ile Ile Gly Arg Leu Leu Thr Gly Pro Val Ser Glu Lys Leu
260 265 270

Thr Tyr His Arg Tyr Leu Arg Leu Ile Asn Ile Gly Gly Leu Ile Ser
275 280 285

Leu Cys Ile Leu Ala Ile Ser Asn Thr Ala Phe Thr Gly Phe Val Leu
290 295 300

Cys Phe Phe Leu Gly Leu Phe Met Ala Gly Met Phe Ala Ile Ala Leu
305 310 315 320

Ile Ile Thr Asn Gln Phe Tyr Pro Gly Lys Thr Glu Gln Thr Thr Ser
325 330 335

Met Leu Leu Ala Ala Asn Gly Leu Gly Gly Ser Ile Leu Pro Ile Ala
340 345 350

Val Gly Trp Ser Leu Asp Glu Phe Pro Val Gln Thr Ala Phe Trp Leu
355 360 365

Phe Thr Thr Leu Gln Tyr Ser Phe Tyr Arg Ala Phe Cys Phe Ser Ser
370 375 380

Ser Leu Ile Phe Leu Ile Leu Ile Gln Ser Pro Ala
385 390 395

<210> 10
<211> 422
<212> PRT
<213> gabT1 from Bacillus subtilis MORI 3K-85

<400> 10
Met Gly Thr Lys Glu Ile Thr Asn Pro Asp Ser Leu Tyr Tyr Lys Val
1 5 10 15

Asp Asp Val Val Met Glu Arg Gly Glu Gly Ile Tyr Leu Tyr Asp Ser
20 25 30

Glu Gly Asn Glu Tyr Ile Asp Cys Ala Ser Ala Thr Phe Asn Leu Asn
35 40 45

```
Leu Gly Tyr Thr Asn Lys Glu Val Ile Asp Thr Val Lys Glu Gln Ala
    50                  55                  60

Asp Gln Leu Ile His Val Thr Ser Ser Tyr Gln Thr Asn Ala Val Asn
65                  70                  75                  80

Lys Leu Ala Glu Lys Leu Val Glu Ile Ser Pro Asp Asn Leu Thr Lys
                85                  90                  95

Val His Pro Lys Val Ser Ser Gly Ser Ala Ala Asn Glu Gly Ala Ile
            100                 105                 110

Lys Met Ala Gln Asn Tyr Ser Gly Lys Thr Asp Val Ile Ser Leu Phe
        115                 120                 125

Arg Ser His Leu Gly Gln Thr Tyr Met Met Ser Ala Leu Ser Gly Asn
    130                 135                 140

Ser Phe Arg Arg Glu Pro Phe Pro Pro Gln Phe Ser Phe Gly Leu Gln
145                 150                 155                 160

Val Pro Asp Pro Tyr Cys Asn Arg Cys Phe Tyr Asn Gln Lys Pro Asp
                165                 170                 175

Ser Cys Gly Met Leu Cys Val Glu Arg Ile Asn Asp Phe Ile Glu Tyr
            180                 185                 190

Ala Ser Asn Gly Lys Ile Ala Ala Met Ile Ile Glu Pro Ile Ser Gly
        195                 200                 205

Asn Gly Gly Asn Ile Val Pro Pro Lys Glu Tyr Phe Lys Gln Leu Arg
    210                 215                 220

Lys Leu Cys Asp Glu His Asp Ile Ala Leu Ile Phe Asp Glu Ile Gln
225                 230                 235                 240

Thr Gly Phe Gly Arg Thr Gly Lys Met Phe Ala Ala Asp Tyr Phe Asp
                245                 250                 255

Val Lys Pro Asn Met Met Thr Val Ala Lys Gly Leu Gly Gly Thr Gly
            260                 265                 270

Phe Gln Val Ala Ala Thr Leu Thr Glu Glu Lys Tyr Met Gly Leu Ala
        275                 280                 285

Gly His Asn His Ser Phe Thr Tyr Gly Ser Asn Val Met Ala Ser Ala
    290                 295                 300

Ala Ala Cys Lys Thr Ile Glu Ile Met Gln Arg Pro Gly Phe Leu Glu
305                 310                 315                 320

Asn Val Thr Thr Val Gly Asn Tyr Ile Met Asp Ser Leu Glu His Met
                325                 330                 335

Lys Lys Glu Phe Thr Phe Ile Ala Asp Val Arg Gly Val Gly Leu Met
            340                 345                 350

Ile Gly Val Glu Ile Val Lys Glu Asn Asn Glu Arg Asp Val Glu Leu
        355                 360                 365

Thr Asn Tyr Ile Ala Lys Arg Ala Met Asp Tyr Gly Leu Ile Leu Arg
    370                 375                 380
```

```
Thr Ser Arg Tyr Gly Phe Gly Asn Val Phe Lys Ile Arg Pro Pro Leu
385                 390                 395                 400

Thr Ile Thr Leu Ser Glu Ala Glu Val Leu Cys Phe Lys Leu Arg Lys
                405                 410                 415

Leu Leu Glu Asp Ile Lys
            420


<210>   11
<211>   316
<212>   PRT
<213>   yktc1 from Bacillus subtilis MORI 3K-85

<400>   11
Met Lys Glu Tyr Ile Ile Glu Leu Gly Thr Phe Val Tyr Glu Glu Val
 1               5                  10                  15

Lys Gly Gln Lys Gly Thr Leu Lys Asn Arg Leu Val Asn Gly Tyr Ser
            20                  25                  30

Pro Gly Gly Asp Ala Gln Phe Asn Ile Asp Ala Ala Ala Glu Asn Ala
        35                  40                  45

Val Leu Glu Tyr Val Asn Ser Lys Glu Glu Ser Val Ala Phe Tyr Thr
    50                  55                  60

Glu Asp Gly Gly Leu Lys Val Phe Gly Asp Asn Pro Gln Tyr Ile Leu
65                  70                  75                  80

Ile Val Asp Pro Ile Asp Gly Thr Arg Pro Ala Ala Ala Gly Leu Glu
                85                  90                  95

Met Ser Cys Ile Ser Ile Ala Leu Ala Ala Tyr Lys Pro Asp Ala Lys
            100                 105                 110

Ile Lys Asp Ile Glu Phe Ala Phe Leu Leu Glu Leu Lys Thr Gly Ala
        115                 120                 125

Tyr Met Tyr Ala Asp Val Tyr Ser Glu Gly Ile Tyr Tyr Glu Gly Tyr
    130                 135                 140

Arg Gly Thr Leu Pro Asn Leu Ser Lys Val Thr Asp Ile Lys His Met
145                 150                 155                 160

Phe Trp Ser Leu Glu Phe Asn Gly His Pro Ala His Leu Met Ile Asp
                165                 170                 175

Ala Tyr Gly His Leu Ile Asp Gln Ser Ala Asn Asn Gly Gly Val Phe
            180                 185                 190

Val Phe Asn Ser Ala Ser Tyr Ser Ile Ser Arg Ile Ile Thr Gly Gln
        195                 200                 205

Met Asp Ala Tyr Val Asp Ile Gly Asn Arg Leu Leu Lys Asp Asp Pro
    210                 215                 220

Ala Leu Leu Lys Asp Phe Gln Asp Val Gly Asn Gly Gln Val Leu His
225                 230                 235                 240

Leu Phe Pro Tyr Asp Ile Ala Ala Ser Val Phe Leu Ala Lys Lys Ala
```

```
                245                 250                 255

Gly Val Val Ile Thr Asp Ala Tyr Gly Lys Ser Leu Asp Asp Thr Leu
            260                 265                 270

Leu Thr Asp Leu Ser Tyr Asn Asn Gln Gln Ser Cys Ile Ala Ala Ser
        275                 280                 285

Thr Lys Glu Leu His Gln Lys Leu Leu Asp Gln Ile Arg Trp Asp Arg
    290                 295                 300

Lys Glu Glu Thr Tyr Glu Ser Val Gly Leu Asp Ser
305                 310                 315


<210>   12
<211>   348
<212>   PRT
<213>   gutB1 from Bacillus subtilis MORI 3K-85

<400>   12
Met Lys Ala Leu Val Trp Thr Pro Asn Asp Arg Leu Glu Met Gln Glu
1               5                   10                  15

Val Glu Glu Pro Gln Ile Lys Lys Met Asn Asp Val Lys Val Lys Ile
            20                  25                  30

Tyr Gly Thr Gly Ile Cys Gly Thr Asp Leu Asn Val Leu Lys Gly Lys
        35                  40                  45

Met His Ala Thr His Asn Met Ile Leu Gly His Glu Ser Val Gly Val
    50                  55                  60

Val Thr Glu Thr Gly Pro Asp Val Lys Asn Val Lys Pro Gly Asp Arg
65                  70                  75                  80

Val Val Ile Asp Pro Thr Gln Phe Cys Gly Lys Cys Tyr Tyr Cys Arg
                85                  90                  95

Lys Gly Leu Thr Cys Tyr Cys Glu Thr Phe Glu Asp Trp Gln Leu Gly
            100                 105                 110

Leu Gly Ala His Gly Thr Phe Ala Asp Tyr Tyr Val Gly Glu Asp Arg
        115                 120                 125

Phe Met Tyr Lys Ile Pro Asp Asn Met Asp Trp Glu Arg Ala Thr Leu
    130                 135                 140

Ile Glu Pro Leu Ser Cys Val Leu Asn Val Ile Glu Arg Ala Ala Ile
145                 150                 155                 160

Lys Pro Asp Asp Ser Val Leu Val Leu Gly Ser Gly Pro Ile Gly Leu
                165                 170                 175

Leu Val Gln Met Met Val Lys Lys Leu Ser Arg Leu Thr Val Ala Thr
            180                 185                 190

Glu Ile Gly Glu Tyr Arg Ser Glu Ala Ala Arg Arg Ile Ser Asp Tyr
        195                 200                 205

Val Tyr His Pro Gln Asp Leu Thr Ala Asp Glu Val Arg Arg Ile Asn
    210                 215                 220
```

```
Gln Gly Arg Thr Phe Asp Val Ile Phe Asp Ala Ile Gly Asn Gln Leu
225                 230                 235                 240

Asp Trp Ala Tyr Pro Leu Ile Asp Lys Gly Gly Arg Leu Val Pro Met
                245                 250                 255

Gly Phe Asp Asp Thr Tyr Glu Met Lys Ile Arg Pro Phe Gln Leu Leu
            260                 265                 270

Ser Asn Gly Val Thr Ile Val Gly Thr Gly Glu Ala Arg Gln Ile Met
        275                 280                 285

Glu Asp Ala Val Ser Cys Ala Ala Asp Met Pro Gln Leu Ser Glu Leu
    290                 295                 300

Ile Thr Glu Lys Thr Pro Leu Glu Asn Tyr Glu Ala Ala Ile Gln Glu
305                 310                 315                 320

Leu Met Gly Ile Asp Pro Leu Ser Asn Glu Arg Lys Asp Ile Ala Ala
                325                 330                 335

Val Lys Thr Ile Leu Val Ser His Pro Asp Met Ile
            340                 345


<210>   13
<211>   478
<212>   PRT
<213>   ybaR from Bacillus subtilis MORI 3K-85

<400>   13
Met Asn Asn Ser Phe Thr Leu Lys Gln Gln Trp Phe Gly Asn Ile Arg
1               5                   10                  15

Lys Asp Ile Leu Ala Gly Ile Leu Val Ala Leu Ala Leu Ile Pro Glu
            20                  25                  30

Ala Ile Gly Phe Ser Ile Ile Ala Gly Val Asp Pro Met Val Gly Leu
        35                  40                  45

Tyr Ala Ser Phe Cys Ile Ala Ile Ile Ile Ser Ile Phe Gly Gly Arg
    50                  55                  60

Pro Gly Met Ile Ser Ala Ala Thr Gly Ser Met Ala Val Val Met Val
65                  70                  75                  80

Ser Leu Val Ala Asp His Gly Leu Gln Tyr Leu Phe Ala Ala Thr Ile
                85                  90                  95

Leu Thr Gly Ile Ile Gln Val Ile Leu Gly Ile Ser Lys Ile Ala Arg
            100                 105                 110

Leu Met Lys Phe Ile Pro Arg Ser Val Met Ile Gly Phe Val Asn Ala
        115                 120                 125

Leu Ala Ile Leu Ile Phe Ser Ala Gln Leu Pro Gln Phe Glu Gly Ala
    130                 135                 140

Ser Trp Ser Met Tyr Ala Met Leu Ala Gly Ser Leu Val Ile Ile Tyr
145                 150                 155                 160

Val Leu Pro Arg Phe Thr Thr Ala Val Pro Ser Pro Leu Val Ala Ile
                165                 170                 175
```

```
Ile Val Met Thr Ile Ile Ala Val Thr Phe His Val Asp Val Arg Thr
            180                 185                 190

Val Gly Asp Met Gly Asn Ile Ser Ser Ser Leu Pro His Phe Leu Ile
        195                 200                 205

Pro Asp Val Pro Phe Thr Phe Glu Thr Leu Gln Ile Ile Phe Pro Tyr
    210                 215                 220

Ser Ile Ala Leu Ala Phe Val Gly Leu Leu Glu Ser Leu Leu Thr Ala
225                 230                 235                 240

Gln Ile Ile Asp Glu Met Thr Asp Thr Asp Ser Asp Lys Asn Lys Glu
                245                 250                 255

Ser Arg Gly Gln Gly Ile Ala Asn Ile Val Thr Gly Phe Phe Gly Gly
            260                 265                 270

Met Ala Gly Cys Ala Met Ile Gly Gln Ser Val Ile Asn Thr Lys Ala
        275                 280                 285

Gly Gly Arg Gly Arg Leu Ser Ala Phe Val Ala Gly Ala Phe Leu Met
    290                 295                 300

Phe Leu Ile Ala Val Leu Ser Gln Val Val Val Lys Ile Pro Met Ala
305                 310                 315                 320

Ala Leu Val Ala Val Met Val Met Val Ser Val Gly Thr Phe Asp Trp
                325                 330                 335

Ser Ser Ile Lys Gly Leu Lys Lys Ala Pro Leu Thr Asp Ser Ile Val
            340                 345                 350

Met Val Val Thr Val Met Thr Val Val Val Thr Asp Asp Leu Ser Lys
        355                 360                 365

Gly Val Phe Val Gly Val Leu Leu Ser Ala Val Phe Phe Val Ala Lys
    370                 375                 380

Ile Ser Lys Leu Lys Ile Val Ser His Ala Glu Asp Gln Lys Leu Arg
385                 390                 395                 400

Thr Tyr Gln Val Lys Gly Gln Ile Phe Phe Ala Ser Val Thr Asp Leu
                405                 410                 415

Thr Asn Ala Phe Ile Tyr Gln Glu Asp Ile Glu Arg Val Val Ile Asp
            420                 425                 430

Leu Thr Glu Ser His Val Trp Asp Asp Ser Gly Ala Ala Ala Leu Asp
        435                 440                 445

Lys Ile Val Ala Lys Phe Lys Glu Gln Gly Ile Glu Ala Glu Leu Lys
    450                 455                 460

Gly Leu Asn Asn Ala Ser Lys Ala Leu Met Lys Gln Met Thr
465                 470                 475
```

```
<210>   14
<211>   283
<212>   PRT
<213>   ybaS from Bacillus subtilis MORI 3K-85
```

```
<400>    14
Met Gly Cys Ala Leu Asp Leu Cys Phe Tyr Tyr Val Cys Arg Gln Phe
 1               5                  10                  15

Glu Arg Glx Leu Ser Ile Phe Glu Thr Arg Val Val Ser Ser Ser Thr
            20                  25                  30

Tyr Asp Ser Cys Thr Ile Cys Ser Ser Tyr Phe Tyr Ala Ala Phe Cys
        35                  40                  45

Leu Gly Gln Arg Pro Ser Tyr Phe Glx Arg Arg Ser Phe Asp Asp His
    50                  55                  60

Arg Phe Asn Ile Gly Cys Cys Asp Ser Tyr Gly Asp Tyr Lys Leu Asp
65                  70                  75                  80

Leu Gly Ser Asp Val Gln Arg Glu Arg Arg Phe Asp Ala Phe Asn Tyr
                85                  90                  95

Leu Ser Glx Tyr Cys Ala Val Thr Ala Asn Arg Thr Ala Glu Pro Phe
            100                 105                 110

Ile Ala Cys Arg Arg Ser Gly Ser Tyr Gly Cys Val Gly Asn Asp Glu
        115                 120                 125

Arg Ala Asp Cys Asp Gly Arg Asp Ser Phe Phe Ser Gly His Ala Val
    130                 135                 140

Glx Ser Asp Ile Ile Ser Arg Lys Asn Cys Ile Cys Glu Gln Cys Ala
145                 150                 155                 160

Val Thr Phe Phe Lys Ala Leu Pro Asp Gly Cys Asp Cys Asp Glx Gln
                165                 170                 175

Phe Ser Asp Cys Ser Leu Phe Gln Ser Gly Glx Phe Glu Ile His Arg
            180                 185                 190

Asn Ser Phe Asp Gly Phe Leu Tyr Arg Val Asn Arg Leu Cys Arg Cys
        195                 200                 205

Met Ala Asp Arg Glu Asn Asp Glu Lys Ala Pro Gly Gly Asn Cys Leu
    210                 215                 220

Ser Tyr Phe Tyr Gly Arg His Glu Lys Tyr Gln Cys Arg Arg Ser Ser
225                 230                 235                 240

Cys Arg Tyr Val Leu Pro Val Ser Gly Cys Arg Ser Arg Cys His Arg
                245                 250                 255

His Ala Val Ser Ala Asp Thr Gly Cys Phe Val Trp Leu Tyr Ala Glu
            260                 265                 270

Ser Phe Glx Ile Lys Thr Cys Ile Ala Glu Ser
        275                 280


<210>    15
<211>    189
<212>    PRT
<213>    ybbA from Bacillus subtilis MORI 3K-85

<400>    15
```

```
Met Leu Ile Gly Ile Lys Pro Glu Asn Arg Leu Asn Glu Tyr Thr Pro
  1               5                  10                  15

Trp Pro Ala Ala Ser Leu Ser Asp Arg Phe Pro Asp Phe Gly Gly Met
             20                  25                  30

Gly Tyr His Tyr Leu Ser Asn Ile Thr Lys Gln Phe Ile Pro Phe Ile
         35                  40                  45

Glu Glu Asn Trp Asn Val Thr Arg Glu Pro Gln Ser Arg Gly Met Ile
     50                  55                  60

Gly Ala Ser Leu Gly Gly Leu Ile Ser Met Phe Ala Leu Leu Lys Tyr
 65                  70                  75                  80

Pro Ser Val Phe Gly Lys Ile Gly Ser Ile Ser Gly Ser Tyr Trp Tyr
                 85                  90                  95

Glu Asn Ala Pro Glu Thr Val His Leu Ser Ser Leu Lys Pro Gly Thr
             100                 105                 110

Ser Ser Val Phe Met Ser Val Gly Ser Asn Glu Gly Arg Glu Lys Gln
         115                 120                 125

Ser Ile Gln Arg Asn Met Leu Gln Asn Thr Lys Gln Val His Gln Ser
     130                 135                 140

Leu Lys Glu Lys Gly Phe Thr Glu Glu Gln Leu Cys Leu Ser Ile Glu
145                 150                 155                 160

Lys Gly Ala Val His His Arg Lys Tyr Phe Cys Lys Gln Phe Ile Ile
                165                 170                 175

Ala Leu Lys Trp Leu Tyr Gly Lys Asn Arg Ser Thr Leu
            180                 185
```

```
<210>    16
<211>    1079
<212>    PRT
<213>    Frgament of gabT1-yktc1-gutB1 from Bacillus subtilis MORI 3K-85

<400>    16
Val Gly Thr Lys Glu Ile Thr Asn Pro Asp Ser Leu Tyr Tyr Lys Val
  1               5                  10                  15

Asp Asp Val Val Met Glu Arg Gly Glu Gly Ile Tyr Leu Tyr Asp Ser
             20                  25                  30

Glu Gly Asn Glu Tyr Ile Asp Cys Ala Ser Ala Thr Phe Asn Leu Asn
         35                  40                  45

Leu Gly Tyr Thr Asn Lys Glu Val Ile Asp Thr Val Lys Glu Gln Ala
     50                  55                  60

Asp Gln Leu Ile His Val Thr Ser Ser Tyr Gln Thr Asn Ala Val Asn
 65                  70                  75                  80

Lys Leu Ala Glu Lys Leu Val Glu Ile Ser Pro Asp Asn Leu Thr Lys
                 85                  90                  95

Val His Pro Lys Val Ser Ser Gly Ser Ala Ala Asn Glu Gly Ala Ile
            100                 105                 110
```

```
Lys Met Ala Gln Asn Tyr Ser Gly Lys Thr Asp Val Ile Ser Leu Phe
        115                 120                 125

Arg Ser His Leu Gly Gln Thr Tyr Met Met Ser Ala Leu Ser Gly Asn
    130                 135                 140

Ser Phe Arg Arg Glu Pro Phe Pro Pro Gln Phe Ser Phe Gly Leu Gln
145                 150                 155                 160

Val Pro Asp Pro Tyr Cys Asn Arg Cys Phe Tyr Asn Gln Lys Pro Asp
                165                 170                 175

Ser Cys Gly Met Leu Cys Val Glu Arg Ile Asn Asp Phe Ile Glu Tyr
            180                 185                 190

Ala Ser Asn Gly Lys Ile Ala Ala Met Ile Ile Glu Pro Ile Ser Gly
        195                 200                 205

Asn Gly Gly Asn Ile Val Pro Pro Lys Glu Tyr Phe Lys Gln Leu Arg
    210                 215                 220

Lys Leu Cys Asp Glu His Asp Ile Ala Leu Ile Phe Asp Glu Ile Gln
225                 230                 235                 240

Thr Gly Phe Gly Arg Thr Gly Lys Met Phe Ala Ala Asp Tyr Phe Asp
                245                 250                 255

Val Lys Pro Asn Met Met Thr Val Ala Lys Gly Leu Gly Gly Thr Gly
            260                 265                 270

Phe Gln Val Ala Ala Thr Leu Thr Glu Glu Lys Tyr Met Gly Leu Ala
        275                 280                 285

Gly His Asn His Ser Phe Thr Tyr Gly Ser Asn Val Met Ala Ser Ala
    290                 295                 300

Ala Ala Cys Lys Thr Ile Glu Ile Met Gln Arg Pro Gly Phe Leu Glu
305                 310                 315                 320

Asn Val Thr Thr Val Gly Asn Tyr Ile Met Asp Ser Leu Glu His Met
                325                 330                 335

Lys Lys Glu Phe Thr Phe Ile Ala Asp Val Arg Gly Val Gly Leu Met
            340                 345                 350

Ile Gly Val Glu Ile Val Lys Glu Asn Asn Glu Arg Asp Val Glu Leu
        355                 360                 365

Thr Asn Tyr Ile Ala Lys Arg Ala Met Asp Tyr Gly Leu Ile Leu Arg
    370                 375                 380

Thr Ser Arg Tyr Gly Phe Gly Asn Val Phe Lys Ile Arg Pro Pro Leu
385                 390                 395                 400

Thr Ile Thr Leu Ser Glu Ala Glu Val Leu Cys Phe Lys Leu Arg Lys
                405                 410                 415

Leu Leu Glu Asp Ile Lys *** Lys Asn Ile Leu *** Ser Leu Glu His
            420                 425                 430

Leu Phe Met Lys Lys *** Arg Asp Arg Lys Glu His *** Lys Thr Gly
        435                 440                 445
```

```
*** Leu Met Gly Ile Pro Gln Ala Glu Met His Ser Leu Thr Leu Met
    450                 455                 460

Pro Leu Pro Arg Met Gln Cys Trp Asn Met *** Ile Ala Lys Arg Asn
465                 470                 475                 480

Gln Leu Arg Phe Ile Arg Lys Met Ala Ala *** Arg Tyr Leu Gly Thr
                485                 490                 495

Ile Leu Asn Ile Phe *** Ser Leu Ile Arg Leu Thr Ala Pro Ala Arg
            500                 505                 510

Gln Leu Gln Asp Leu Lys Cys Leu Val Ser Leu Ser His *** Leu His
        515                 520                 525

Thr Ser Leu Met Gln Arg Leu Arg Ile Leu Asn Leu Leu Phe Cys Leu
    530                 535                 540

Ser *** Lys Gln Val Pro Ile Cys Thr Arg Met Phe Ile Leu Lys Gly
545                 550                 555                 560

Phe Ile Met Lys Ala Ile Val Ala Pro Cys Gln Ile *** Ala Lys ***
                565                 570                 575

Gln Thr Leu Asn Ile Cys Ser Gly Ala Leu Ser Leu Met Gly Ile Pro
            580                 585                 590

Arg Thr *** *** Leu Met Arg Thr Val Ile *** Leu Ile Ser Gln Arg
        595                 600                 605

Ile Met Ala Ala Tyr Leu Phe Ser Ile Ala Leu Leu Ile Gln Ser Pro
    610                 615                 620

Gly Leu Leu Gln Gly Arg Trp Met Pro Met Trp Ile Ser Ala Ile Val
625                 630                 635                 640

Tyr *** Lys Met Ile Leu Arg Cys *** Lys Ile Phe Arg Met Trp Gly
                645                 650                 655

Met Gly Arg Ser Cys Ile Cys Phe His Met Ile Leu Pro Gln Val Cys
            660                 665                 670

Phe Trp Arg Lys Lys Gln Val Leu *** Leu Leu Met Arg Met Glu Ser
        675                 680                 685

Pro Trp Met Ile His Phe *** Arg Ile Leu Ala Thr Ile Thr Ser Ser
    690                 695                 700

His Ala Ser Leu His Gln Arg Arg Ser Cys Ile Arg Ser Cys Trp Ile
705                 710                 715                 720

Lys Phe Ala Gly Thr Glu Arg Lys Arg His Met Lys Ala Leu Val Trp
                725                 730                 735

Thr Pro Asn Asp Arg Leu Glu Met Gln Glu Val Glu Glu Pro Gln Ile
            740                 745                 750

Lys Lys Met Asn Asp Val Lys Val Lys Ile Tyr Gly Thr Gly Ile Cys
        755                 760                 765

Gly Thr Asp Leu Asn Val Leu Lys Gly Lys Met His Ala Thr His Asn
    770                 775                 780
```

Met Ile Leu Gly His Glu Ser Val Gly Val Val Thr Glu Thr Gly Pro
785 790 795 800

Asp Val Lys Asn Val Lys Pro Gly Asp Arg Val Val Ile Asp Pro Thr
805 810 815

Gln Phe Cys Gly Lys Cys Tyr Tyr Cys Arg Lys Gly Leu Thr Cys Tyr
820 825 830

Cys Glu Thr Phe Glu Asp Trp Gln Leu Gly Leu Gly Ala His Gly Thr
835 840 845

Phe Ala Asp Tyr Tyr Val Gly Glu Asp Arg Phe Met Tyr Lys Ile Pro
850 855 860

Asp Asn Met Asp Trp Glu Arg Ala Thr Leu Ile Glu Pro Leu Ser Cys
865 870 875 880

Val Leu Asn Val Ile Glu Arg Ala Ala Ile Lys Pro Asp Asp Ser Val
885 890 895

Leu Val Leu Gly Ser Gly Pro Ile Gly Leu Leu Val Gln Met Met Val
900 905 910

Lys Lys Leu Ser Arg Leu Thr Val Ala Thr Glu Ile Gly Glu Tyr Arg
915 920 925

Ser Glu Ala Ala Arg Arg Ile Ser Asp Tyr Val Tyr His Pro Gln Asp
930 935 940

Leu Thr Ala Asp Glu Val Arg Arg Ile Asn Gln Gly Arg Thr Phe Asp
945 950 955 960

Val Ile Phe Asp Ala Ile Gly Asn Gln Leu Asp Trp Ala Tyr Pro Leu
965 970 975

Ile Asp Lys Gly Gly Arg Leu Val Pro Met Gly Phe Asp Asp Thr Tyr
980 985 990

Glu Met Lys Ile Arg Pro Phe Gln Leu Leu Ser Asn Gly Val Thr Ile
995 1000 1005

Val Gly Thr Gly Glu Ala Arg Gln Ile Met Glu Asp Ala Val Ser Cys
1010 1015 1020

Ala Ala Asp Met Pro Gln Leu Ser Glu Leu Ile Thr Glu Lys Thr Pro
1025 1030 1035 1040

Leu Glu Asn Tyr Glu Ala Ala Ile Gln Glu Leu Met Gly Ile Asp Pro
1045 1050 1055

Leu Ser Asn Glu Arg Lys Asp Ile Ala Ala Val Lys Thr Ile Leu Val
1060 1065 1070

Ser His Pro Asp Met Ile ***
1075

<210> 17
<211> 1638
<212> PRT
<213> Fragment of gabT1-yktc1-gutB1-ybaR from Bacillus subtilis MORI 3K-85

```
<400>    17
Val Gly Thr Lys Glu Ile Thr Asn Pro Asp Ser Leu Tyr Tyr Lys Val
1               5                   10                  15

Asp Asp Val Val Met Glu Arg Gly Glu Gly Ile Tyr Leu Tyr Asp Ser
            20                  25                  30

Glu Gly Asn Glu Tyr Ile Asp Cys Ala Ser Ala Thr Phe Asn Leu Asn
        35                  40                  45

Leu Gly Tyr Thr Asn Lys Glu Val Ile Asp Thr Val Lys Glu Gln Ala
    50                  55                  60

Asp Gln Leu Ile His Val Thr Ser Ser Tyr Gln Thr Asn Ala Val Asn
65                  70                  75                  80

Lys Leu Ala Glu Lys Leu Val Glu Ile Ser Pro Asp Asn Leu Thr Lys
                85                  90                  95

Val His Pro Lys Val Ser Ser Gly Ser Ala Ala Asn Glu Gly Ala Ile
            100                 105                 110

Lys Met Ala Gln Asn Tyr Ser Gly Lys Thr Asp Val Ile Ser Leu Phe
        115                 120                 125

Arg Ser His Leu Gly Gln Thr Tyr Met Met Ser Ala Leu Ser Gly Asn
    130                 135                 140

Ser Phe Arg Arg Glu Pro Phe Pro Pro Gln Phe Ser Phe Gly Leu Gln
145                 150                 155                 160

Val Pro Asp Pro Tyr Cys Asn Arg Cys Phe Tyr Asn Gln Lys Pro Asp
                165                 170                 175

Ser Cys Gly Met Leu Cys Val Glu Arg Ile Asn Asp Phe Ile Glu Tyr
            180                 185                 190

Ala Ser Asn Gly Lys Ile Ala Ala Met Ile Ile Glu Pro Ile Ser Gly
        195                 200                 205

Asn Gly Gly Asn Ile Val Pro Pro Lys Glu Tyr Phe Lys Gln Leu Arg
    210                 215                 220

Lys Leu Cys Asp Glu His Asp Ile Ala Leu Ile Phe Asp Glu Ile Gln
225                 230                 235                 240

Thr Gly Phe Gly Arg Thr Gly Lys Met Phe Ala Ala Asp Tyr Phe Asp
                245                 250                 255

Val Lys Pro Asn Met Met Thr Val Ala Lys Gly Leu Gly Gly Thr Gly
            260                 265                 270

Phe Gln Val Ala Ala Thr Leu Thr Glu Glu Lys Tyr Met Gly Leu Ala
        275                 280                 285

Gly His Asn His Ser Phe Thr Tyr Gly Ser Asn Val Met Ala Ser Ala
    290                 295                 300

Ala Ala Cys Lys Thr Ile Glu Ile Met Gln Arg Pro Gly Phe Leu Glu
305                 310                 315                 320

Asn Val Thr Thr Val Gly Asn Tyr Ile Met Asp Ser Leu Glu His Met
```

```
                325                 330                 335

Lys Lys Glu Phe Thr Phe Ile Ala Asp Val Arg Gly Val Gly Leu Met
            340                 345                 350

Ile Gly Val Glu Ile Val Lys Glu Asn Asn Glu Arg Asp Val Glu Leu
        355                 360                 365

Thr Asn Tyr Ile Ala Lys Arg Ala Met Asp Tyr Gly Leu Ile Leu Arg
    370                 375                 380

Thr Ser Arg Tyr Gly Phe Gly Asn Val Phe Lys Ile Arg Pro Pro Leu
385                 390                 395                 400

Thr Ile Thr Leu Ser Glu Ala Glu Val Leu Cys Phe Lys Leu Arg Lys
                405                 410                 415

Leu Leu Glu Asp Ile Lys *** Lys Asn Ile Leu *** Ser Leu Glu His
            420                 425                 430

Leu Phe Met Lys Lys *** Arg Asp Arg Lys Glu His *** Lys Thr Gly
        435                 440                 445

*** Leu Met Gly Ile Pro Gln Ala Glu Met His Ser Leu Thr Leu Met
    450                 455                 460

Pro Leu Pro Arg Met Gln Cys Trp Asn Met *** Ile Ala Lys Arg Asn
465                 470                 475                 480

Gln Leu Arg Phe Ile Arg Lys Met Ala Ala *** Arg Tyr Leu Gly Thr
                485                 490                 495

Ile Leu Asn Ile Phe *** Ser Leu Ile Arg Leu Thr Ala Pro Ala Arg
            500                 505                 510

Gln Leu Gln Asp Leu Lys Cys Leu Val Ser Leu Ser His *** Leu His
        515                 520                 525

Thr Ser Leu Met Gln Arg Leu Arg Ile Leu Asn Leu Leu Phe Cys Leu
    530                 535                 540

Ser *** Lys Gln Val Pro Ile Cys Thr Arg Met Phe Ile Leu Lys Gly
545                 550                 555                 560

Phe Ile Met Lys Ala Ile Val Ala Pro Cys Gln Ile *** Ala Lys ***
                565                 570                 575

Gln Thr Leu Asn Ile Cys Ser Gly Ala Leu Ser Leu Met Gly Ile Pro
            580                 585                 590

Arg Thr *** *** Leu Met Arg Thr Val Ile *** Leu Ile Ser Gln Arg
        595                 600                 605

Ile Met Ala Ala Tyr Leu Phe Ser Ile Ala Leu Leu Ile Gln Ser Pro
    610                 615                 620

Gly Leu Leu Gln Gly Arg Trp Met Pro Met Trp Ile Ser Ala Ile Val
625                 630                 635                 640

Tyr *** Lys Met Ile Leu Arg Cys *** Lys Ile Phe Arg Met Trp Gly
                645                 650                 655

Met Gly Arg Ser Cys Ile Cys Phe His Met Ile Leu Pro Gln Val Cys
```

```
                660                 665                 670

Phe Trp Arg Lys Lys Gln Val Leu *** Leu Leu Met Arg Met Glu Ser
            675                 680                 685

Pro Trp Met Ile His Phe *** Arg Ile Leu Ala Thr Ile Thr Ser Ser
    690                 695                 700

His Ala Ser Leu His Gln Arg Arg Ser Cys Ile Arg Ser Cys Trp Ile
705                 710                 715                 720

Lys Phe Ala Gly Thr Glu Arg Lys Arg His Met Lys Ala Leu Val Trp
                725                 730                 735

Thr Pro Asn Asp Arg Leu Glu Met Gln Glu Val Glu Glu Pro Gln Ile
            740                 745                 750

Lys Lys Met Asn Asp Val Lys Val Lys Ile Tyr Gly Thr Gly Ile Cys
        755                 760                 765

Gly Thr Asp Leu Asn Val Leu Lys Gly Lys Met His Ala Thr His Asn
    770                 775                 780

Met Ile Leu Gly His Glu Ser Val Gly Val Val Thr Glu Thr Gly Pro
785                 790                 795                 800

Asp Val Lys Asn Val Lys Pro Gly Asp Arg Val Val Ile Asp Pro Thr
                805                 810                 815

Gln Phe Cys Gly Lys Cys Tyr Tyr Cys Arg Lys Gly Leu Thr Cys Tyr
            820                 825                 830

Cys Glu Thr Phe Glu Asp Trp Gln Leu Gly Leu Gly Ala His Gly Thr
        835                 840                 845

Phe Ala Asp Tyr Tyr Val Gly Glu Asp Arg Phe Met Tyr Lys Ile Pro
    850                 855                 860

Asp Asn Met Asp Trp Glu Arg Ala Thr Leu Ile Glu Pro Leu Ser Cys
865                 870                 875                 880

Val Leu Asn Val Ile Glu Arg Ala Ala Ile Lys Pro Asp Asp Ser Val
                885                 890                 895

Leu Val Leu Gly Ser Gly Pro Ile Gly Leu Leu Val Gln Met Met Val
            900                 905                 910

Lys Lys Leu Ser Arg Leu Thr Val Ala Thr Glu Ile Gly Glu Tyr Arg
        915                 920                 925

Ser Glu Ala Ala Arg Arg Ile Ser Asp Tyr Val Tyr His Pro Gln Asp
    930                 935                 940

Leu Thr Ala Asp Glu Val Arg Arg Ile Asn Gln Gly Arg Thr Phe Asp
945                 950                 955                 960

Val Ile Phe Asp Ala Ile Gly Asn Gln Leu Asp Trp Ala Tyr Pro Leu
                965                 970                 975

Ile Asp Lys Gly Gly Arg Leu Val Pro Met Gly Phe Asp Asp Thr Tyr
            980                 985                 990

Glu Met Lys Ile Arg Pro Phe Gln Leu Leu Ser Asn Gly Val Thr Ile
```

995 1000 1005

Val Gly Thr Gly Glu Ala Arg Gln Ile Met Glu Asp Ala Val Ser Cys
1010 1015 1020

Ala Ala Asp Met Pro Gln Leu Ser Glu Leu Ile Thr Glu Lys Thr Pro
1025 1030 1035 1040

Leu Glu Asn Tyr Glu Ala Ala Ile Gln Glu Leu Met Gly Ile Asp Pro
1045 1050 1055

Leu Ser Asn Glu Arg Lys Asp Ile Ala Ala Val Lys Thr Ile Leu Val
1060 1065 1070

Ser His Pro Asp Met Ile *** Cys Ser Asp Met Ala Gln Thr *** Phe
1075 1080 1085

Glu Pro Tyr Phe Ser Ser Ala Phe Thr Leu Arg Ile *** Ile Lys Phe
1090 1095 1100

Asn Met Ile Leu Phe Val *** *** Leu Leu Ala Ser Ile Gln Tyr Thr
1105 1110 1115 1120

Gln Arg Phe Pro Phe Phe Ser Asp Arg Phe Pro Phe His Phe Leu Pro
1125 1130 1135

*** Asn Lys Glu Ser Ser His Tyr Asn Arg Tyr Gly Ala Asp Leu Phe
1140 1145 1150

Phe Lys Lys Glu Ser Leu Tyr Leu Asn Asn Ser Phe Thr Leu Lys Gln
1155 1160 1165

Gln Trp Phe Gly Asn Ile Arg Lys Asp Ile Leu Ala Gly Ile Leu Val
1170 1175 1180

Ala Leu Ala Leu Ile Pro Glu Ala Ile Gly Phe Ser Ile Ile Ala Gly
1185 1190 1195 1200

Val Asp Pro Met Val Gly Leu Tyr Ala Ser Phe Cys Ile Ala Ile Ile
1205 1210 1215

Ile Ser Ile Phe Gly Gly Arg Pro Gly Met Ile Ser Ala Ala Thr Gly
1220 1225 1230

Ser Met Ala Val Val Met Val Ser Leu Val Ala Asp His Gly Leu Gln
1235 1240 1245

Tyr Leu Phe Ala Ala Thr Ile Leu Thr Gly Ile Ile Gln Val Ile Leu
1250 1255 1260

Gly Ile Ser Lys Ile Ala Arg Leu Met Lys Phe Ile Pro Arg Ser Val
1265 1270 1275 1280

Met Ile Gly Phe Val Asn Ala Leu Ala Ile Leu Ile Phe Ser Ala Gln
1285 1290 1295

Leu Pro Gln Phe Glu Gly Ala Ser Trp Ser Met Tyr Ala Met Leu Ala
1300 1305 1310

Gly Ser Leu Val Ile Ile Tyr Val Leu Pro Arg Phe Thr Thr Ala Val
1315 1320 1325

Pro Ser Pro Leu Val Ala Ile Ile Val Met Thr Ile Ile Ala Val Thr

1330 1335 1340

Phe His Val Asp Val Arg Thr Val Gly Asp Met Gly Asn Ile Ser Ser
1345 1350 1355 1360

Ser Leu Pro His Phe Leu Ile Pro Asp Val Pro Phe Thr Phe Glu Thr
1365 1370 1375

Leu Gln Ile Ile Phe Pro Tyr Ser Ile Ala Leu Ala Phe Val Gly Leu
1380 1385 1390

Leu Glu Ser Leu Leu Thr Ala Gln Ile Ile Asp Glu Met Thr Asp Thr
1395 1400 1405

Asp Ser Asp Lys Asn Lys Glu Ser Arg Gly Gln Gly Ile Ala Asn Ile
1410 1415 1420

Val Thr Gly Phe Phe Gly Gly Met Ala Gly Cys Ala Met Ile Gly Gln
1425 1430 1435 1440

Ser Val Ile Asn Thr Lys Ala Gly Gly Arg Gly Arg Leu Ser Ala Phe
1445 1450 1455

Val Ala Gly Ala Phe Leu Met Phe Leu Ile Ala Val Leu Ser Gln Val
1460 1465 1470

Val Val Lys Ile Pro Met Ala Ala Leu Val Ala Val Met Val Met Val
1475 1480 1485

Ser Val Gly Thr Phe Asp Trp Ser Ser Ile Lys Gly Leu Lys Lys Ala
1490 1495 1500

Pro Leu Thr Asp Ser Ile Val Met Val Val Thr Val Met Thr Val Val
1505 1510 1515 1520

Val Thr Asp Asp Leu Ser Lys Gly Val Phe Val Gly Val Leu Leu Ser
1525 1530 1535

Ala Val Phe Phe Val Ala Lys Ile Ser Lys Leu Lys Ile Val Ser His
1540 1545 1550

Ala Glu Asp Gln Lys Leu Arg Thr Tyr Gln Val Lys Gly Gln Ile Phe
1555 1560 1565

Phe Ala Ser Val Thr Asp Leu Thr Asn Ala Phe Ile Tyr Gln Glu Asp
1570 1575 1580

Ile Glu Arg Val Val Ile Asp Leu Thr Glu Ser His Val Trp Asp Asp
1585 1590 1595 1600

Ser Gly Ala Ala Ala Leu Asp Lys Ile Val Ala Lys Phe Lys Glu Gln
1605 1610 1615

Gly Ile Glu Ala Glu Leu Lys Gly Leu Asn Asn Ala Ser Lys Ala Leu
1620 1625 1630

Met Lys Gln Met Thr ***
1635

<210> 18
<211> 3358
<212> PRT

<213> Fragment of glcP1-gabT1-yktc1-gutB1-ybaR-ybaS-ybbA from Bacillus subtilis MORI 3K-85

<400> 18

```
Glu Tyr Gly Gly Thr Arg Glu Ile Pro Ser Glu Ser Gly Arg Thr Ile
 1               5                  10                  15

Ser Gln Gly *** Ile Leu Pro Ser Asp Arg *** *** Thr Ser Thr Val
            20                  25                  30

Arg Glu Arg *** Lys Ala Pro Arg Lys Gly Ser Glu Arg Asp Pro Glu
        35                  40                  45

Thr Val Cys Leu Gln Val Val Arg Ala Arg *** Arg Val Met Ala Cys
    50                  55                  60

Leu Leu *** Asn Glu Pro Ala Ser Tyr Asp Pro Val Gln Gly *** Ala
65                  70                  75                  80

Glu Asp Ala Glu Pro Gln Arg Lys Arg Val *** Ile Gly Arg Met Ser
                85                  90                  95

Thr Trp Ser *** Thr Arg Asn Gln Val Ile Tyr Pro Cys Pro Gly ***
            100                 105                 110

Ser Ser Gly Asn Thr Glu Trp Arg Pro Glu Pro Thr His Val Glu Lys
        115                 120                 125

Cys Gly Asp Glu Val Trp Val Gly Val Lys Cys Gln Ser Asn Leu Glu
    130                 135                 140

Ile Ala Gly Ser Leu Arg Asn Ser Phe Arg Ala Ser Leu Lys Val Arg
145                 150                 155                 160

Val Leu Glu Val Glu His *** Leu Asp *** Gly Pro Leu Pro Gly Tyr
                165                 170                 175

Arg Ile Gln Ser Asn Ser Glu Cys Gln *** Leu Ile Leu Gly Ser Gln
            180                 185                 190

Thr Ala Ser Asp Lys Ile Arg Ser Arg Lys Gly Asn Ser Pro Asp Arg
        195                 200                 205

Gln Leu Arg Ser Gln Ser Ile Arg *** Val Glu Lys Asp Val Glu Leu
    210                 215                 220

Leu Arg Gln Pro Gly Cys Trp Leu Arg Ser Ser His His Leu Lys Ser
225                 230                 235                 240

Ala *** *** Leu Thr Gly Arg Val Thr Leu Arg Arg Lys Cys Thr Gly
                245                 250                 255

Ala Lys Arg Ile Thr Glu Ala Ala Asp Cys Ser Ser Asn Ser Gly Arg
            260                 265                 270

Arg Ala Phe *** Gly Leu *** Ser Gln Thr Gly Arg Thr Gly Gly Ala
        275                 280                 285

Leu Arg Ser Glu Asn Ala Gly Met Ser Ser Glu Arg Gly Val Arg Ile
    290                 295                 300

Pro Ser Thr Glu Cys Leu Arg Phe Pro Glu Glu Gly Ser Ser Ala Gln
305                 310                 315                 320
```

```
Gly *** Ser Gly Pro Lys Pro Arg Pro Lys Gly Val Gly Asp Gly Gln
                325                 330                 335

Gln Val Asp Ile Pro Val Pro Pro Pro His His Leu Ser Asn Gly Gly
            340                 345                 350

Thr Gln Glu Asp Arg Val Ser Ala Val Leu Asp Ile Arg Val Gln Ala
        355                 360                 365

Val Arg Leu Gly Asn Arg Gln Ile Arg Phe Pro *** Gly *** Ala Val
    370                 375                 380

Met Ala Ser Glu Ile *** *** Arg Ser Ser *** Phe His Thr Ala Lys
385                 390                 395                 400

Lys Ser Leu *** Arg Gly Glu Arg Cys Pro Tyr Arg Lys Pro Thr Gln
                405                 410                 415

Val Gly Glu Glu Arg Ile Leu Arg *** Ser Arg Glu Leu Ser Leu Arg
            420                 425                 430

Asn Ser Ala Lys *** Pro Arg Asn Phe Gly Arg Arg Gly Ala Leu Leu
        435                 440                 445

Gly Cys Lys Pro Glu Arg Ala Ala Val Asn Arg Pro Arg Arg Leu Phe
    450                 455                 460

Ser Lys Asn Thr Gly Leu Cys Glu Ala Val Arg Arg Ser Ile Gly Ala
465                 470                 475                 480

Asp Ala Cys Pro Val Leu Glu Gly *** Glu Glu Arg Leu Ala *** Ala
                485                 490                 495

Lys Val Arg Ile Glu Ala Pro Val Asn Gly Gly Arg Asn Tyr Asn Gly
            500                 505                 510

Pro Lys Val Ala Lys Phe Leu Val Gly *** Val Pro Thr Arg Thr Lys
        515                 520                 525

Gly Ala Thr Ile Trp Ala Leu Ser Gln Arg Glu Thr Arg *** Asn Tyr
    530                 535                 540

Ser Thr Cys Glu Asp Ala Gly Tyr Pro Arg Gln Asp Gly Lys Thr Pro
545                 550                 555                 560

Trp Ser Phe Thr Ala Ala *** Tyr *** Met Leu Val Gln Leu Val Gln
                565                 570                 575

Asp Arg *** Glu Pro Trp Lys Pro Glu Arg Gln Leu Arg Trp Arg His
            580                 585                 590

Arg Trp Asp Thr Thr Leu Ala Val Leu Thr Phe *** Pro Ala Ala Leu
        595                 600                 605

Ile Gly Arg Gly Asp Ser Val Arg Trp Ala Val *** Leu Gly Arg Ser
    610                 615                 620

His Val Asp His Ala Gly Asp Cys Ile Ser Ile Lys Asn Ile Arg Glu
625                 630                 635                 640

Leu Glu Lys Gln Lys Ala Arg *** Asn Glu Tyr *** Arg Val Val Asn
                645                 650                 655
```

```
Asn Gln Asn Ala Val Cys Thr Gly Asn Ser Ser Arg Leu His Pro Thr
            660                 665                 670

Ala Ile Gly Arg Ile Glu Pro Pro Asn Pro Leu Ala Ala Ser Ser Met
        675                 680                 685

Leu Val Val Cys Ser Val Phe Pro Gly *** Asn *** Leu Val Ile Ile
    690                 695                 700

Lys Ala Met Ala Asn Ile Pro Ala Ile Asn Asn Pro Lys Lys Lys His
705                 710                 715                 720

Arg Thr Asn Pro Val Lys Ala Val Leu Leu Ile Ala Lys Ile Gln Ser
                725                 730                 735

Glu Met Arg Pro Pro Met Leu Ile Ser Leu Arg *** Arg *** *** Val
            740                 745                 750

Ser Phe Ser Glu Thr Gly Pro Val Ser Lys Arg Pro Met Ile Met Pro
        755                 760                 765

Ile Gln Tyr Ile Val Thr Val Asn Val Ala Lys Glu Leu Glu Ile Ser
    770                 775                 780

His Phe Ser Ala Lys Met Glu Gly Ile Lys *** Thr Ile Asp Thr Ser
785                 790                 795                 800

Leu Pro Pro *** Lys Lys Lys *** Ala Ala Ala Lys Glu Ile Asn Ser
                805                 810                 815

Lys Pro Cys Phe Arg Lys Leu Ile Gly Ala Cys Cys Leu Pro Leu Pro
            820                 825                 830

Glu Val Phe Val *** Arg Met Ser Lys Ser Ser Lys Leu Pro Lys Leu
        835                 840                 845

Ile Arg Leu Gln Thr Ser Ile Lys Arg Asn Glu Leu Asn Pro Asn Thr
    850                 855                 860

Arg Lys Ala Asn Ala His Thr Leu Leu Leu Ile Lys Lys Pro Asp Ser
865                 870                 875                 880

Asn Gly Met Lys Arg Ala Pro Lys Pro Tyr Ala Thr Ser Ile Leu Leu
                885                 890                 895

Met Ala Ile Ala Cys Phe Ser Leu Ile Ala Val Arg Ile Ile Val Pro
            900                 905                 910

Thr Gln Asp Ser Thr Ser Pro Ala Pro Ser Pro Ala Ile Leu Thr Arg
        915                 920                 925

Arg Lys Ser Arg Gly His Gly Gly Arg Lys *** Ile Asn Asn Ser Gln
    930                 935                 940

Leu Ile Met Ala Ser Pro Ile Thr Thr Thr Phe Leu Arg Pro Asn Leu
945                 950                 955                 960

Phe Thr Ala *** Gly Ile Pro Ser Thr Pro Ile Lys Asn Pro Ile Asn
                965                 970                 975

Cys Glu Asn Thr Asn Thr Pro Pro Leu Leu *** Leu Cys Ala *** Trp
            980                 985                 990
```

```
Glu Ser Asn Ser Gly Arg Thr Ala Pro Arg Ile Thr Arg Val Lys Ala
        995                1000                1005

Ile Asp Pro *** Asp Lys His Pro Ile His Ser Lys Arg Val Phe Ile
   1010                1015                1020

His *** Ser Pro Leu Ile Lys Ile Ile Leu Thr *** Asp Leu His Asn
1025                1030                1035                1040

Arg Thr Gly Ser Lys Asn Lys Leu Phe Thr Leu Phe Val Phe Ser Phe
                1045                1050                1055

Phe Thr Ile Asn Pro Phe Ile Ser Ile Ile Thr Tyr Glu Val *** Ile
            1060                1065                1070

Glu Thr Pro His Ala Asn Val Asn Lys *** Leu Thr Ile Phe Thr Ile
        1075                1080                1085

Tyr Leu Val Asp Ile Asn Phe *** Asn Leu Tyr Asp Asp *** Arg Val
   1090                1095                1100

Leu His Ile Leu *** Ile Gly Val Leu Val Val Gly Thr Lys Glu Ile
1105                1110                1115                1120

Thr Asn Pro Asp Ser Leu Tyr Tyr Lys Val Asp Asp Val Val Met Glu
                1125                1130                1135

Arg Gly Glu Gly Ile Tyr Leu Tyr Asp Ser Glu Gly Asn Glu Tyr Ile
            1140                1145                1150

Asp Cys Ala Ser Ala Thr Phe Asn Leu Asn Leu Gly Tyr Thr Asn Lys
        1155                1160                1165

Glu Val Ile Asp Thr Val Lys Glu Gln Ala Asp Gln Leu Ile His Val
   1170                1175                1180

Thr Ser Ser Tyr Gln Thr Asn Ala Val Asn Lys Leu Ala Glu Lys Leu
1185                1190                1195                1200

Val Glu Ile Ser Pro Asp Asn Leu Thr Lys Val His Pro Lys Val Ser
                1205                1210                1215

Ser Gly Ser Ala Ala Asn Glu Gly Ala Ile Lys Met Ala Gln Asn Tyr
            1220                1225                1230

Ser Gly Lys Thr Asp Val Ile Ser Leu Phe Arg Ser His Leu Gly Gln
        1235                1240                1245

Thr Tyr Met Met Ser Ala Leu Ser Gly Asn Ser Phe Arg Arg Glu Pro
   1250                1255                1260

Phe Pro Pro Gln Phe Ser Phe Gly Leu Gln Val Pro Asp Pro Tyr Cys
1265                1270                1275                1280

Asn Arg Cys Phe Tyr Asn Gln Lys Pro Asp Ser Cys Gly Met Leu Cys
                1285                1290                1295

Val Glu Arg Ile Asn Asp Phe Ile Glu Tyr Ala Ser Asn Gly Lys Ile
            1300                1305                1310

Ala Ala Met Ile Ile Glu Pro Ile Ser Gly Asn Gly Gly Asn Ile Val
        1315                1320                1325
```

```
Pro Pro Lys Glu Tyr Phe Lys Gln Leu Arg Lys Leu Cys Asp Glu His
   1330                1335                1340

Asp Ile Ala Leu Ile Phe Asp Glu Ile Gln Thr Gly Phe Gly Arg Thr
1345                1350                1355                1360

Gly Lys Met Phe Ala Ala Asp Tyr Phe Asp Val Lys Pro Asn Met Met
               1365                1370                1375

Thr Val Ala Lys Gly Leu Gly Gly Thr Gly Phe Gln Val Ala Ala Thr
           1380                1385                1390

Leu Thr Glu Glu Lys Tyr Met Gly Leu Ala Gly His Asn His Ser Phe
       1395                1400                1405

Thr Tyr Gly Ser Asn Val Met Ala Ser Ala Ala Ala Cys Lys Thr Ile
   1410                1415                1420

Glu Ile Met Gln Arg Pro Gly Phe Leu Glu Asn Val Thr Thr Val Gly
1425                1430                1435                1440

Asn Tyr Ile Met Asp Ser Leu Glu His Met Lys Lys Glu Phe Thr Phe
               1445                1450                1455

Ile Ala Asp Val Arg Gly Val Gly Leu Met Ile Gly Val Glu Ile Val
           1460                1465                1470

Lys Glu Asn Asn Glu Arg Asp Val Glu Leu Thr Asn Tyr Ile Ala Lys
       1475                1480                1485

Arg Ala Met Asp Tyr Gly Leu Ile Leu Arg Thr Ser Arg Tyr Gly Phe
   1490                1495                1500

Gly Asn Val Phe Lys Ile Arg Pro Pro Leu Thr Ile Thr Leu Ser Glu
1505                1510                1515                1520

Ala Glu Val Leu Cys Phe Lys Leu Arg Lys Leu Leu Glu Asp Ile Lys
               1525                1530                1535

*** Lys Asn Ile Leu *** Ser Leu Glu His Leu Phe Met Lys Lys ***
           1540                1545                1550

Arg Asp Arg Lys Glu His *** Lys Thr Gly *** Leu Met Gly Ile Pro
       1555                1560                1565

Gln Ala Glu Met His Ser Leu Thr Leu Met Pro Leu Pro Arg Met Gln
   1570                1575                1580

Cys Trp Asn Met *** Ile Ala Lys Arg Asn Gln Leu Arg Phe Ile Arg
1585                1590                1595                1600

Lys Met Ala Ala *** Arg Tyr Leu Gly Thr Ile Leu Asn Ile Phe ***
               1605                1610                1615

Ser Leu Ile Arg Leu Thr Ala Pro Ala Arg Gln Leu Gln Asp Leu Lys
           1620                1625                1630

Cys Leu Val Ser Leu Ser His *** Leu His Thr Ser Leu Met Gln Arg
       1635                1640                1645

Leu Arg Ile Leu Asn Leu Leu Phe Cys Leu Ser *** Lys Gln Val Pro
   1650                1655                1660
```

```
Ile Cys Thr Arg Met Phe Ile Leu Lys Gly Phe Ile Met Lys Ala Ile
1665                1670                1675                1680

Val Ala Pro Cys Gln Ile *** Ala Lys *** Gln Thr Leu Asn Ile Cys
                1685                1690                1695

Ser Gly Ala Leu Ser Leu Met Gly Ile Pro Arg Thr *** *** Leu Met
            1700                1705                1710

Arg Thr Val Ile *** Leu Ile Ser Gln Arg Ile Met Ala Ala Tyr Leu
        1715                1720                1725

Phe Ser Ile Ala Leu Leu Ile Gln Ser Pro Gly Leu Leu Gln Gly Arg
    1730                1735                1740

Trp Met Pro Met Trp Ile Ser Ala Ile Val Tyr *** Lys Met Ile Leu
1745                1750                1755                1760

Arg Cys *** Lys Ile Phe Arg Met Trp Gly Met Gly Arg Ser Cys Ile
                1765                1770                1775

Cys Phe His Met Ile Leu Pro Gln Val Cys Phe Trp Arg Lys Lys Gln
            1780                1785                1790

Val Leu *** Leu Leu Met Arg Met Glu Ser Pro Trp Met Ile His Phe
        1795                1800                1805

*** Arg Ile Leu Ala Thr Ile Thr Ser Ser His Ala Ser Leu His Gln
    1810                1815                1820

Arg Arg Ser Cys Ile Arg Ser Cys Trp Ile Lys Phe Ala Gly Thr Glu
1825                1830                1835                1840

Arg Lys Arg His Met Lys Ala Leu Val Trp Thr Pro Asn Asp Arg Leu
                1845                1850                1855

Glu Met Gln Glu Val Glu Glu Pro Gln Ile Lys Lys Met Asn Asp Val
            1860                1865                1870

Lys Val Lys Ile Tyr Gly Thr Gly Ile Cys Gly Thr Asp Leu Asn Val
        1875                1880                1885

Leu Lys Gly Lys Met His Ala Thr His Asn Met Ile Leu Gly His Glu
    1890                1895                1900

Ser Val Gly Val Val Thr Glu Thr Gly Pro Asp Val Lys Asn Val Lys
1905                1910                1915                1920

Pro Gly Asp Arg Val Val Ile Asp Pro Thr Gln Phe Cys Gly Lys Cys
                1925                1930                1935

Tyr Tyr Cys Arg Lys Gly Leu Thr Cys Tyr Cys Glu Thr Phe Glu Asp
            1940                1945                1950

Trp Gln Leu Gly Leu Gly Ala His Gly Thr Phe Ala Asp Tyr Tyr Val
        1955                1960                1965

Gly Glu Asp Arg Phe Met Tyr Lys Ile Pro Asp Asn Met Asp Trp Glu
    1970                1975                1980

Arg Ala Thr Leu Ile Glu Pro Leu Ser Cys Val Leu Asn Val Ile Glu
1985                1990                1995                2000
```

```
Arg Ala Ala Ile Lys Pro Asp Asp Ser Val Leu Val Leu Gly Ser Gly
                2005                2010                2015

Pro Ile Gly Leu Leu Val Gln Met Met Val Lys Lys Leu Ser Arg Leu
            2020                2025                2030

Thr Val Ala Thr Glu Ile Gly Glu Tyr Arg Ser Glu Ala Ala Arg Arg
        2035                2040                2045

Ile Ser Asp Tyr Val Tyr His Pro Gln Asp Leu Thr Ala Asp Glu Val
    2050                2055                2060

Arg Arg Ile Asn Gln Gly Arg Thr Phe Asp Val Ile Phe Asp Ala Ile
2065                2070                2075                2080

Gly Asn Gln Leu Asp Trp Ala Tyr Pro Leu Ile Asp Lys Gly Gly Arg
                2085                2090                2095

Leu Val Pro Met Gly Phe Asp Asp Thr Tyr Glu Met Lys Ile Arg Pro
            2100                2105                2110

Phe Gln Leu Leu Ser Asn Gly Val Thr Ile Val Gly Thr Gly Glu Ala
        2115                2120                2125

Arg Gln Ile Met Glu Asp Ala Val Ser Cys Ala Ala Asp Met Pro Gln
    2130                2135                2140

Leu Ser Glu Leu Ile Thr Glu Lys Thr Pro Leu Glu Asn Tyr Glu Ala
2145                2150                2155                2160

Ala Ile Gln Glu Leu Met Gly Ile Asp Pro Leu Ser Asn Glu Arg Lys
                2165                2170                2175

Asp Ile Ala Ala Val Lys Thr Ile Leu Val Ser His Pro Asp Met Ile
            2180                2185                2190

*** Cys Ser Asp Met Ala Gln Thr *** Phe Glu Pro Tyr Phe Ser Ser
        2195                2200                2205

Ala Phe Thr Leu Arg Ile *** Ile Lys Phe Asn Met Ile Leu Phe Val
    2210                2215                2220

*** *** Leu Leu Ala Ser Ile Gln Tyr Thr Gln Arg Phe Pro Phe Phe
2225                2230                2235                2240

Ser Asp Arg Phe Pro Phe His Phe Leu Pro *** Asn Lys Glu Ser Ser
                2245                2250                2255

His Tyr Asn Arg Tyr Gly Ala Asp Leu Phe Phe Lys Lys Glu Ser Leu
            2260                2265                2270

Tyr Leu Asn Asn Ser Phe Thr Leu Lys Gln Gln Trp Phe Gly Asn Ile
        2275                2280                2285

Arg Lys Asp Ile Leu Ala Gly Ile Leu Val Ala Leu Ala Leu Ile Pro
    2290                2295                2300

Glu Ala Ile Gly Phe Ser Ile Ile Ala Gly Val Asp Pro Met Val Gly
2305                2310                2315                2320

Leu Tyr Ala Ser Phe Cys Ile Ala Ile Ile Ile Ser Ile Phe Gly Gly
                2325                2330                2335
```

Arg Pro Gly Met Ile Ser Ala Ala Thr Gly Ser Met Ala Val Val Met
2340 2345 2350

Val Ser Leu Val Ala Asp His Gly Leu Gln Tyr Leu Phe Ala Ala Thr
2355 2360 2365

Ile Leu Thr Gly Ile Ile Gln Val Ile Leu Gly Ile Ser Lys Ile Ala
2370 2375 2380

Arg Leu Met Lys Phe Ile Pro Arg Ser Val Met Ile Gly Phe Val Asn
2385 2390 2395 2400

Ala Leu Ala Ile Leu Ile Phe Ser Ala Gln Leu Pro Gln Phe Glu Gly
2405 2410 2415

Ala Ser Trp Ser Met Tyr Ala Met Leu Ala Gly Ser Leu Val Ile Ile
2420 2425 2430

Tyr Val Leu Pro Arg Phe Thr Thr Ala Val Pro Ser Pro Leu Val Ala
2435 2440 2445

Ile Ile Val Met Thr Ile Ile Ala Val Thr Phe His Val Asp Val Arg
2450 2455 2460

Thr Val Gly Asp Met Gly Asn Ile Ser Ser Ser Leu Pro His Phe Leu
2465 2470 2475 2480

Ile Pro Asp Val Pro Phe Thr Phe Glu Thr Leu Gln Ile Ile Phe Pro
2485 2490 2495

Tyr Ser Ile Ala Leu Ala Phe Val Gly Leu Leu Glu Ser Leu Leu Thr
2500 2505 2510

Ala Gln Ile Ile Asp Glu Met Thr Asp Thr Asp Ser Asp Lys Asn Lys
2515 2520 2525

Glu Ser Arg Gly Gln Gly Ile Ala Asn Ile Val Thr Gly Phe Phe Gly
2530 2535 2540

Gly Met Ala Gly Cys Ala Met Ile Gly Gln Ser Val Ile Asn Thr Lys
2545 2550 2555 2560

Ala Gly Gly Arg Gly Arg Leu Ser Ala Phe Val Ala Gly Ala Phe Leu
2565 2570 2575

Met Phe Leu Ile Ala Val Leu Ser Gln Val Val Val Lys Ile Pro Met
2580 2585 2590

Ala Ala Leu Val Ala Val Met Val Met Val Ser Val Gly Thr Phe Asp
2595 2600 2605

Trp Ser Ser Ile Lys Gly Leu Lys Lys Ala Pro Leu Thr Asp Ser Ile
2610 2615 2620

Val Met Val Val Thr Val Met Thr Val Val Val Thr Asp Asp Leu Ser
2625 2630 2635 2640

Lys Gly Val Phe Val Gly Val Leu Leu Ser Ala Val Phe Phe Val Ala
2645 2650 2655

Lys Ile Ser Lys Leu Lys Ile Val Ser His Ala Glu Asp Gln Lys Leu
2660 2665 2670

```
Arg Thr Tyr Gln Val Lys Gly Gln Ile Phe Phe Ala Ser Val Thr Asp
       2675                2680                2685

Leu Thr Asn Ala Phe Ile Tyr Gln Glu Asp Ile Glu Arg Val Val Ile
   2690                2695                2700

Asp Leu Thr Glu Ser His Val Trp Asp Asp Ser Gly Ala Ala Ala Leu
2705                2710                2715                2720

Asp Lys Ile Val Ala Lys Phe Lys Glu Gln Gly Ile Glu Ala Glu Leu
                2725                2730                2735

Lys Gly Leu Asn Asn Ala Ser Lys Ala Leu Met Lys Gln Met Thr ***
            2740                2745                2750

Asn Arg Glu Val Gln Met Asn Glu Ile Trp Ala Phe Leu Tyr Asn Ile
       2755                2760                2765

Arg Leu Asn Ser Ile Asp Ile Ile Glu Arg Arg Glu Ile Leu Asn Arg
   2770                2775                2780

Gly Leu Ala Tyr Asp Ala Ser Glu Ala Glu Pro Cys Ile Gly Glu Ser
2785                2790                2795                2800

Asp Ala Ala Phe Asn Ala Asp *** Cys Gly Ala Gly Arg Ala Ala Val
                2805                2810                2815

Ser Val Phe Lys Trp Val *** Met Gly Cys Ala Leu Asp Leu Cys Phe
            2820                2825                2830

Tyr Tyr Val Cys Arg Gln Phe Glu Arg *** Leu Ser Ile Phe Glu Thr
       2835                2840                2845

Arg Val Val Ser Ser Ser Thr Tyr Asp Ser Cys Thr Ile Cys Ser Ser
   2850                2855                2860

Tyr Phe Tyr Ala Ala Phe Cys Leu Gly Gln Arg Pro Ser Tyr Phe ***
2865                2870                2875                2880

Arg Arg Ser Phe Asp Asp His Arg Phe Asn Ile Gly Cys Cys Asp Ser
                2885                2890                2895

Tyr Gly Asp Tyr Lys Leu Asp Leu Gly Ser Asp Val Gln Arg Glu Arg
            2900                2905                2910

Arg Phe Asp Ala Phe Asn Tyr Leu Ser *** Tyr Cys Ala Val Thr Ala
       2915                2920                2925

Asn Arg Thr Ala Glu Pro Phe Ile Ala Cys Arg Arg Ser Gly Ser Tyr
   2930                2935                2940

Gly Cys Val Gly Asn Asp Glu Arg Ala Asp Cys Asp Gly Arg Asp Ser
2945                2950                2955                2960

Phe Phe Ser Gly His Ala Val *** Ser Asp Ile Ile Ser Arg Lys Asn
                2965                2970                2975

Cys Ile Cys Glu Gln Cys Ala Val Thr Phe Phe Lys Ala Leu Pro Asp
            2980                2985                2990

Gly Cys Asp Cys Asp *** Gln Phe Ser Asp Cys Ser Leu Phe Gln Ser
       2995                3000                3005
```

```
Gly *** Phe Glu Ile His Arg Asn Ser Phe Asp Gly Phe Leu Tyr Arg
   3010                3015                3020

Val Asn Arg Leu Cys Arg Cys Met Ala Asp Arg Glu Asn Asp Glu Lys
3025                3030                3035                3040

Ala Pro Gly Gly Asn Cys Leu Ser Tyr Phe Tyr Gly Arg His Glu Lys
               3045                3050                3055

Tyr Gln Cys Arg Arg Ser Ser Cys Arg Tyr Val Leu Pro Val Ser Gly
           3060                3065                3070

Cys Arg Ser Arg Cys His Arg His Ala Val Ser Ala Asp Thr Gly Cys
       3075                3080                3085

Phe Val Trp Leu Tyr Ala Glu Ser Phe *** Ile Lys Thr Cys Ile Ala
   3090                3095                3100

Glu Ser Met Lys Lys Thr Ala His Arg Val Glu Arg Phe Phe Pro Tyr
3105                3110                3115                3120

Ser His Leu Ser Ala Met Met Asn Cys Leu Gln Lys Tyr Leu Arg Trp
               3125                3130                3135

Cys Thr Ala Pro Phe Ser Ile Glu Arg His Ser Cys Ser Ser Val Lys
           3140                3145                3150

Pro Phe Ser Phe Lys Leu Trp Cys Thr Cys Phe Val Phe Cys Ser Ile
       3155                3160                3165

Phe Arg *** Met Asp Cys Phe Ser Arg Pro Ser Leu Leu Pro Thr Asp
   3170                3175                3180

Ile Lys Thr Leu Glu Val Pro Gly Phe Lys Glu Asp Lys *** Thr Val
3185                3190                3195                3200

Ser Gly Ala Phe Ser Tyr Gln Tyr Glu Pro Glu Ile Glu Pro Ile Phe
               3205                3210                3215

Pro Asn Thr Asp Gly Tyr Phe Asn Lys Ala Asn Ile Glu Met Arg Pro
           3220                3225                3230

Pro Asn Glu Ala Pro Ile Ile Pro Leu Asp Cys Gly Ser Leu Val Thr
       3235                3240                3245

Phe Gln Phe Ser Ser Ile Asn Gly Met Asn Cys Phe Val Ile Leu Asp
   3250                3255                3260

Arg *** Trp Tyr Pro Ile Pro Pro Lys Ser Gly Asn Arg Ser Leu Ser
3265                3270                3275                3280

Asp Ala Ala Gly Gln Gly Val Tyr Ser Phe Ser Arg Phe Ser Gly Leu
               3285                3290                3295

Ile Pro Ile Ser Thr Ser Ser Gly Ser Leu Arg Cys Arg Asn Ala Leu
           3300                3305                3310

Ser Asn Asn Ser Ile Trp Ser *** Asn Lys Glu Leu Pro Ser Cys Thr
       3315                3320                3325

*** Thr Ala Gly Asn Ala Leu Leu Pro Gly Leu *** Glu Gly Gly Arg
   3330                3335                3340
```

```
Tyr Ser Val Asn Leu Leu Phe Pro Ala Leu Cys Ser Glu Xaa
3345                3350                3355


<210>    19
<211>    3237
<212>    DNA
<213>    Fragment of gabT1-yktc1-gutB1 from Bacillus subtilis MORI 3K-85

<400>    19
gtggggacta aagaaattac gaatccagac agtttgtatt acaaagtgga tgacgttgtc        60

atggaaagag gtgaaggcat atatttgtac gattcagagg gcaatgagta tattgattgt       120

gcctctgcta ctttcaactt aaatttgggt tataccaata aagaagttat tgatacagtc       180

aaagaacagg ctgaccagct gatacacgtc acttcttcct accaaaccaa cgccgttaat       240

aaattagctg aaaaacttgt agaaatctcc cccgacaatc taactaaagt tcaccctaag       300

gttagcagcg gctctgctgc gaatgaagga gctattaaaa tggctcaaaa ctattctgga       360

aaaacagatg tcatttcttt attccgaagc caccttggcc aaacgtatat gatgtctgcg       420

ttatccggaa attcatttcg aagagagcca ttccccccctc agttttcttt tggcttacag       480

gtgcctgacc cctattgcaa ccgttgtttt tacaatcaga agccagattc atgcggaatg       540

ctttgtgtag aaagaattaa tgattttatt gaatatgcga gtaacggaaa aattgccgcg       600

atgattattg aaccgatttc cggtaacgga ggaaacatcg ttccgcctaa ggagtacttt       660

aagcaattaa gaaagctctg tgatgagcat gatattgcac ttatttttga tgaaattcaa       720

accggatttg gccggacagg caagatgttt gcggctgatt actttgatgt gaaaccgaat       780

atgatgactg ttgcaaaagg attgggaggc acgggattcc aggttgccgc caccctcaca       840

gaggaaaaat acatgggatt agccggccac aatcactctt ttacttatgg ctcgaacgtg       900

atggcctcgg cagcagcttg taagacaatc gaaatcatgc agcggccggg cttcttagaa       960

aatgtaacaa ctgtcgggaa ttacattatg gattccttag agcacatgaa gaaagaattc      1020

acatttattg ctgacgtcag aggcgtaggt ttgatgatcg gtgttgaaat tgtaaaagag      1080

aataatgagc gtgatgtaga gctgaccaat tacattgcaa aacgggctat ggattatggg      1140

ttaattttgc ggacttcccg ttacggattc ggaaatgtat ttaaaatccg tccgcctctc      1200

acgattacac ttagtgaggc tgaggtgctt tgtttcaaac ttcgtaagct attggaggat      1260

atcaagtgaa agaatatatt atagagcttg gaacatttgt ttatgaagaa gtgaagggac      1320

agaaaggaac actgaaaaac cggttagtta atgggtattc cccaggcgga gatgcacagt      1380

ttaacattga tgccgctgcc gagaatgcag tgctggaata tgtgaatagc aaagaggaat      1440

cagttgcgtt ttatacggaa gatggcggcc tgaaggtatt tggggacaat cctcaatata      1500

ttttgatcgt tgatccgatt gacggcaccc gcccggcagc tgcaggactt gaaatgtctt      1560
```

gtatctctat cgcattagct gcatacaagc ctgatgcaaa gattaaggat attgaatttg 1620

cttttctgct tgagctgaaa acaggtgcct atatgtacgc ggatgtttat tctgaaggga 1680

tttattatga aggctatcgt ggcaccctgc caaatctgag caaagtgaca gacattaaac 1740

atatgttctg gagccttgag tttaatgggc atcccgcgca cctgatgatt gatgcgtacg 1800

gtcatttgat tgatcagtca gcgaataatg gcggcgtatt tgttttcaat agcgcttctt 1860

attcaatctc caggattatt acagggcaga tggatgccta tgtggatatc ggcaatcgtc 1920

tattaaaaga tgatcctgcg ctgctgaaag attttcagga tgtggggaat gggcaggtcc 1980

tgcatctgtt tccatatgat attgccgcaa gtgtgttttt ggcgaaaaaa gcaggtgttg 2040

tgattactga tgcgtatgga aagtccttgg atgatacact tctgacggat cttagctaca 2100

ataaccagca gtcatgcatc gctgcatcaa cgaaggagct gcatcagaag ctgctggatc 2160

aaattcgctg ggacagaaag gaagagacat atgaaagcgt tggtctggac tcctaatgat 2220

cggcttgaaa tgcaagaagt agaagaacct caaatcaaaa aaatgaacga tgtgaaagtt 2280

aaaatatacg ggacaggcat ctgcggaacg gatttaaatg ttctaaaagg aaagatgcat 2340

gcgactcaca atatgatcct aggccacgaa tctgtgggag tggtgacaga aacagggcct 2400

gatgttaaaa acgtcaagcc tggtgatcgc gtggtaattg atccgactca gttttgcggg 2460

aagtgttatt attgccggaa aggtttaact tgttattgtg aaacgtttga agactggcag 2520

ctgggattag ggcgcatgg cacttttgcc gattattacg taggcgagga ccgttttatg 2580

tataaaatcc cggacaatat ggattgggaa cgagccactt tgattgagcc gctttcctgt 2640

gtattaaatg tgatagagcg ggctgcaata aaacctgacg attctgtact tgtattaggg 2700

tcagggccga ttgggctgct tgttcaaatg atggtgaaaa aactatcaag gctgaccgtt 2760

gcgaccgaga tcggagagta tcggtcagaa gcggcacgcc ggatatctga ctatgtttac 2820

cacccgcagg atttaacggc agatgaggtc aggcggataa accaaggaag aacatttgat 2880

gtgatctttg acgcgatcgg caatcagctt gattgggcat atccgttaat tgacaagggc 2940

ggaaggcttg tgccgatggg gtttgatgat acgtatgaaa tgaaaatcag gccttttcag 3000

ctgctttcta acggggtgac gattgttgga accggagagg ctcgacaaat catggaggat 3060

gcggtatcat gtgccgcgga catgcctcag ctttctgaac tgattacgga gaaaaccccg 3120

cttgagaact atgaggctgc catccaggaa ttgatgggca tagatccgtt gtcaaatgag 3180

agaaaagata ttgccgcagt taaaacgatt cttgtttccc atccggatat gatataa 3237

<210> 20
<211> 4914
<212> DNA
<213> Fragment of gabT1-yktc1-gutB1-ybaR from Bacillus subtilis MORI 3K-85

<400> 20

```
gtggggacta aagaaattac gaatccagac agtttgtatt acaaagtgga tgacgttgtc        60
atggaaagag gtgaaggcat atatttgtac gattcagagg gcaatgagta tattgattgt       120
gcctctgcta ctttcaactt aaatttgggt tataccaata aagaagttat tgatacagtc       180
aaagaacagg ctgaccagct gatacacgtc acttcttcct accaaaccaa cgccgttaat       240
aaattagctg aaaaacttgt agaaatctcc cccgacaatc taactaaagt tcaccctaag       300
gttagcagcg gctctgctgc gaatgaagga gctattaaaa tggctcaaaa ctattctgga       360
aaaacagatg tcatttcttt attccgaagc caccttggcc aaacgtatat gatgtctgcg       420
ttatccggaa attcatttcg aagagagcca ttccccccct agttttcttt tggcttacag       480
gtgcctgacc cctattgcaa ccgttgtttt tacaatcaga agccagattc atgcggaatg       540
ctttgtgtag aaagaattaa tgattttatt gaatatgcga gtaacggaaa aattgccgcg       600
atgattattg aaccgatttc cggtaacgga ggaaacatcg ttccgcctaa ggagtacttt       660
aagcaattaa gaaagctctg tgatgagcat gatattgcac ttatttttga tgaaattcaa       720
accggatttg gccggacagg caagatgttt gcggctgatt actttgatgt gaaaccgaat       780
atgatgactg ttgcaaaagg attgggaggc acgggattcc aggttgccgc caccctcaca       840
gaggaaaaat acatgggatt agccggccac aatcactctt ttacttatgg ctcgaacgtg       900
atggcctcgg cagcagcttg taagacaatc gaaatcatgc agcggccggg cttcttagaa       960
aatgtaacaa ctgtcgggaa ttacattatg gattccttag agcacatgaa gaaagaattc      1020
acatttattg ctgacgtcag aggcgtaggt ttgatgatcg gtgttgaaat tgtaaaagag      1080
aataatgagc gtgatgtaga gctgaccaat tacattgcaa aacgggctat ggattatggg      1140
ttaattttgc ggacttcccg ttacggattc ggaaatgtat ttaaaatccg tccgcctctc      1200
acgattacac ttagtgaggc tgaggtgctt tgtttcaaac ttcgtaagct attggaggat      1260
atcaagtgaa agaatatatt atagagcttg gaacatttgt ttatgaagaa gtgaagggac      1320
agaaaggaac actgaaaaac cggttagtta atgggtattc cccaggcgga gatgcacagt      1380
ttaacattga tgccgctgcc gagaatgcag tgctggaata tgtgaatagc aaagaggaat      1440
cagttgcgtt ttatacggaa gatggcggcc tgaaggtatt tggggacaat cctcaatata      1500
ttttgatcgt tgatccgatt gacggcaccc gcccggcagc tgcaggactt gaaatgtctt      1560
gtatctctat cgcattagct gcatacaagc ctgatgcaaa gattaaggat attgaatttg      1620
cttttctgct tgagctgaaa acaggtgcct atatgtacgc ggatgtttat tctgaaggga      1680
tttattatga aggctatcgt ggcaccctgc caaatctgag caaagtgaca gacattaaac      1740
atatgttctg gagccttgag tttaatgggc atcccgcgca cctgatgatt gatgcgtacg      1800
gtcatttgat tgatcagtca gcgaataatg gcggcgtatt tgttttcaat agcgcttctt      1860
attcaatctc caggattatt acagggcaga tggatgccta tgtggatatc ggcaatcgtc      1920
```

tattaaaaga tgatcctgcg ctgctgaaag attttcagga tgtggggaat gggcaggtcc 1980
tgcatctgtt tccatatgat attgccgcaa gtgtgttttt ggcgaaaaaa gcaggtgttg 2040
tgattactga tgcgtatgga aagtccttgg atgatacact tctgacggat cttagctaca 2100
ataaccagca gtcatgcatc gctgcatcaa cgaaggagct gcatcagaag ctgctggatc 2160
aaattcgctg ggacagaaag gaagagacat atgaaagcgt tggtctggac tcctaatgat 2220
cggcttgaaa tgcaagaagt agaagaacct caaatcaaaa aaatgaacga tgtgaaagtt 2280
aaaatatacg ggacaggcat ctgcggaacg gatttaaatg ttctaaaagg aaagatgcat 2340
gcgactcaca atatgatcct aggccacgaa tctgtgggag tggtgacaga aacagggcct 2400
gatgttaaaa acgtcaagcc tggtgatcgc gtggtaattg atccgactca gttttgcggg 2460
aagtgttatt attgccggaa aggtttaact tgttattgtg aaacgtttga agactggcag 2520
ctgggattag ggcgcatgg cacttttgcc gattattacg taggcgagga ccgttttatg 2580
tataaaatcc cggacaatat ggattgggaa cgagccactt tgattgagcc gctttcctgt 2640
gtattaaatg tgatagagcg ggctgcaata aaacctgacg attctgtact tgtattaggg 2700
tcagggccga ttgggctgct tgttcaaatg atggtgaaaa aactatcaag gctgaccgtt 2760
gcgaccgaga tcggagagta tcggtcagaa gcggcacgcc ggatatctga ctatgtttac 2820
cacccgcagg atttaacggc agatgaggtc aggcggataa accaaggaag aacatttgat 2880
gtgatctttg acgcgatcgg caatcagctt gattgggcat atccgttaat tgacaagggc 2940
ggaaggcttg tgccgatggg gtttgatgat acgtatgaaa tgaaaatcag gccttttcag 3000
ctgctttcta acggggtgac gattgttgga accggagagg ctcgacaaat catggaggat 3060
gcggtatcat gtgccgcgga catgcctcag ctttctgaac tgattacgga gaaaaccccg 3120
cttgagaact atgaggctgc catccaggaa ttgatgggca tagatccgtt gtcaaatgag 3180
agaaaagata ttgccgcagt taaaacgatt cttgtttccc atccggatat gatataatgc 3240
agcgatatgg ctcaaactta gtttgagcca tatttttctt cagcattcac cttgcgtatt 3300
tgaataaaat ttaatatgat actctttgta taataactcc ttgcctcaat acaatatact 3360
caacgtttcc ccttttttctc cgatcgtttt ccttttcatt ttctcccgta aaataaagaa 3420
agctcccatt acaacagata cggtgcggat ttattttta aaaaggagtc gttatatttg 3480
aataatagtt ttacattaaa acagcaatgg ttcggaaata tccgaaaaga cattcttgct 3540
ggcattttag ttgcattagc gttaatacct gaagcgattg gcttttctat tatagccggt 3600
gttgatccga tggtcggtct ttacgcttca ttttgtatag ccattattat ttctattttt 3660
ggcggcagac caggcatgat ttcagctgcg actggctcaa tggcggtcgt tatggtgtcg 3720
ctggttgctg atcatgggct tcagtattta tttgcggcta cgattttgac aggaatcatt 3780

caggtgattt taggcatcag taaaatagct agattaatga aattcatccc gcgttctgtg 3840
atgatcgggt ttgtgaatgc gctggcgatc cttatcttct ctgcgcagct ccctcagttt 3900
gaaggtgctt cttggagcat gtatgcgatg cttgccgggt cgttggttat tatttatgtg 3960
ctgccgaggt ttacgactgc ggttccgtct ccgcttgtcg cgattattgt gatgacgatt 4020
attgctgtca ctttccatgt ggatgtgcgg acggtcgggg atatggggaa tatttctagt 4080
tcattgcctc atttcttaat cccggatgtt ccgtttacct ttgaaacact gcaaatcatt 4140
tttccttatt ccatcgcttt agcgtttgtc gggttgctag aatctttact gacagcacag 4200
attattgatg agatgacgga tacggatagt gacaaaaata aagagagccg cggacaggga 4260
attgcgaata tcgtgaccgg gttctttggc ggtatggctg gctgcgctat gatcggacaa 4320
tctgtcatta atacgaaagc gggcggacga ggacgcttgt cagcatttgt tgccggtgca 4380
tttctgatgt ttttaattgc ggtgttaagt caggttgtag tgaaaattcc gatggctgcg 4440
ttagtggctg ttatggtgat ggtttctgtc ggcacatttg attggtcttc tataaaaggc 4500
cttaaaaaag cgccgctcac cgactcgatt gtcatggttg tgactgtgat gacggttgtt 4560
gtgacggatg acttatctaa aggcgtattc gtcggcgttc tcttaagtgc cgtattcttt 4620
gtggcaaaaa tctcgaagct gaaaattgtt tcacatgcag aggatcaaaa gctgagaacg 4680
tatcaagtga aggggcaaat ctttttttgcg tcagtgactg atcttacgaa tgcgtttatt 4740
tatcaagagg atattgaacg ggtcgtgatt gacttaactg aatctcacgt ttgggatgat 4800
tcaggagcgg cagcgcttga taagattgtt gcgaaattta aagaacaagg cattgaagct 4860
gagttaaaag gtttgaacaa tgcgagtaaa gcccttatga aacagatgac ataa 4914